# EUROPEAN PATENT APPLICATION

(11) **EP 2 351 838 A1**
(43) Date of publication of application: **03.08.2011**
(21) Application number: 10173971.2
(22) Date of filing: 22.10.2001
(51) Int. Cl.: C12N 15/09, C12N 15/62, C07K 16/28, A61K 39/395

(54) **Crosslinking agonistic antibodies**

(30) Priority: 20.10.2000 JP 2000321821; 20.10.2000 JP 2000321822; 12.09.2001 JP 2001277314; 12.03.2001 WO PCT/JP01/01912; 17.04.2001 WO PCT/JP01/03288
(62) Divisional of application: 01978852.0
(71) Applicant: Chugai Seiyaku Kabushiki Kaisha, Kita-ku Tokyo 115-8543 (JP)
(72) Inventor: Fukushima, Naoshi, Shizuoka 412-8513 (JP); Tsuchiya, Masayuki, Shizuoka 412-8513 (JP); Uno, Shinsuke, Shizuoka 412-8513 (JP); Ohtomo, Toshihiko, Shizuoka 412-8513 (JP); Yabuta, Naohiro, Kanagawa 247-8530 (JP); Tsunoda, Hiroyuki, Shizuoka 412-8513 (JP)
(74) Representative: HOFFMANN EITLE

(57) **Abstract**

The invention relates to a modified antibody which contains two or more H chain V regions and two or more L chain V regions of monoclonal antibody and can transduce a signal into cells by crosslinking a cell surface molecule(s) to thereby serve as an agonist. The modified antibody can be used as a signal transduction agonist and, therefore, useful as a preventive and/or remedy for various diseases such as cancer, inflammation, hormone disorders and blood diseases.

SEQUENCE LISTING

<110> CHUGAI SEIYAKU KABUSHIKI KAISHA

<120> Small remodeling agonist antibody

<130> FP1032

<141> 2001-10-22

<150> JP2000-321821

<151> 2000-10-20

<150> JP2000-321822

<151> 2000-10-20

<150> PCT/JP01/01912

<151> 2001-03-12

<150> PCT/JP01/03288

<151> 2001-04-17

<150> JP2001-277314

<151> 2001-09-12

<160> 113

<210> 1

<211> 27

<212> DNA

<213> Artificial Sequence

<220>

<223> PCR primer

<400> 1

ccatcctaat acgactcact atagggc 27

<210> 2

<211> 27

<212> DNA

<213> Artificial Sequence

<220>

<223> PCR primer

<400> 2

ggatcccggg tggatggtgg gaagatg  27

<210> 3

<211> 28

<212> DNA

<213> Artificial Sequence

<220>

<223> PCR primer

<400> 3

ggatcccggg ccagtggata gacagatg  28

<210> 4

<211> 26

<212> DNA

<213> Artificial Sequence

<220>

<223> PCR primer

<400> 4

ggatcccggg agtggataga ccgatg  26

<210> 5

<211> 394

<212> DNA

<213> Mus

<220>

<221> CDS

<222> (1)...(393)

<223> pGEM-M1L. 1-57;signal peptide, 58-394;mature peptide

<400> 5

atg aag ttg cct gtt agg ctg ttg gtg ctg atg ttc tgg att cct gcg 48

Met Lys Leu Pro Val Arg Leu Leu Val Leu Met Phe Trp Ile Pro Ala

1               5                   10                  15

tcc agc agt gat gtt gtg atg acc caa act cca ctc tcc ctg cct gtc 96

Ser Ser Ser Asp Val Val Met Thr Gln Thr Pro Leu Ser Leu Pro Val

            20                  25                  30

agt ctt gga gat caa gcc tcc atc tct tgc aga tct agt cag agc ctt 144

Ser Leu Gly Asp Gln Ala Ser Ile Ser Cys Arg Ser Ser Gln Ser Leu

        35                  40                  45

cta cac agt aaa gga aac acc tat tta caa tgg tac cta cag aag cca 192

Leu His Ser Lys Gly Asn Thr Tyr Leu Gln Trp Tyr Leu Gln Lys Pro

    50                  55                  60

ggc cag tct cca aag ctc ctg atc tac aaa gtt tcc aac cga ttt tct 240

Gly Gln Ser Pro Lys Leu Leu Ile Tyr Lys Val Ser Asn Arg Phe Ser

65                  70                  75                  80

ggg gtc cca gac agg ttc agt ggc agt gga tca ggg aca gat ttc aca 288

Gly Val Pro Asp Arg Phe Ser Gly Ser Gly Ser Gly Thr Asp Phe Thr

                85                  90                  95

ctc aag atc agc aga gtg gag gct gag gat ctg gga gtt tat ttc tgc 336

Leu Lys Ile Ser Arg Val Glu Ala Glu Asp Leu Gly Val Tyr Phe Cys

            100                 105                 110
tct caa agt aca cat gtt ccg tac acg tcc gga ggg ggg acc aag ctg 384
Ser Gln Ser Thr His Val Pro Tyr Thr Ser Gly Gly Gly Thr Lys Leu
        115                 120                 125
gaa ata aaa c 394
Glu Ile Lys
    130

<210> 6
<211> 409
<212> DNA
<213> Mus
<220>
<221> CDS
<222> (1)...(408)
<223> pGEM-M1H. 1-57;signal peptide, 58-409;mature peptide
<400> 6
atg gaa tgg agc tgg ata ttt ctc ttc ctc ctg tca gga act gca ggt 48
Met Glu Trp Ser Trp Ile Phe Leu Phe Leu Leu Ser Gly Thr Ala Gly
1               5                   10                  15
gtc cac tcc cag gtc cag ctg cag cag tct gga cct gac ctg gta aag 96
Val His Ser Gln Val Gln Leu Gln Gln Ser Gly Pro Asp Leu Val Lys
            20                  25                  30
cct ggg gct tca gtg aag atg tcc tgc aag gct tct gga tac acc ttc 144
Pro Gly Ala Ser Val Lys Met Ser Cys Lys Ala Ser Gly Tyr Thr Phe
        35                  40                  45
gtt aac cat gtt atg cac tgg gtg aag cag aag cca ggg cag ggc ctt 192
Val Asn His Val Met His Trp Val Lys Gln Lys Pro Gly Gln Gly Leu

    50                  55                  60
gag tgg att gga tat att tat cct tac aat gat ggt act aag tac aat 240
Glu Trp Ile Gly Tyr Ile Tyr Pro Tyr Asn Asp Gly Thr Lys Tyr Asn
65                  70                  75                  80
gag aag ttc aag ggc aag gcc aca ctg act tca gag aaa tcc tcc agc 288
Glu Lys Phe Lys Gly Lys Ala Thr Leu Thr Ser Glu Lys Ser Ser Ser
                85                  90                  95
gca gcc tac atg gag ctc agc agc ctg gcc tct gag gac tct gcg gtc 336
Ala Ala Tyr Met Glu Leu Ser Ser Leu Ala Ser Glu Asp Ser Ala Val
            100                 105                 110
tac tac tgt gca aga ggg ggt tac tat agt tac gac gac tgg ggc caa 384
Tyr Tyr Cys Ala Arg Gly Gly Tyr Tyr Ser Tyr Asp Asp Trp Gly Gln
        115                 120                 125
ggc acc act ctc aca gtc tcc tca g 409
Gly Thr Thr Leu Thr Val Ser Ser
    130                 135

<210> 7
<211> 394
<212> DNA
<213> Mus
<220>
<221> CDS
<222> (1)...(393)
<223> pGEM-M2L. 1-57;signal peptide, 58-394;mature peptide
<400> 7
atg aag ttg cct gtt agg ctg ttg gtg ctg atg ttc tgg att cct ggt 48
Met Lys Leu Pro Val Arg Leu Leu Val Leu Met Phe Trp Ile Pro Gly

1                   5                   10                  15
tcc agc agt gat gtt gtg atg acc caa agt cca ctc tcc ctg cct gtc    96
Ser Ser Ser Asp Val Val Met Thr Gln Ser Pro Leu Ser Leu Pro Val
                20                  25                  30
agt ctt gga gat caa gcc tcc atc tct tgc aga tca agt cag agc ctt    144
Ser Leu Gly Asp Gln Ala Ser Ile Ser Cys Arg Ser Ser Gln Ser Leu
            35                  40                  45
gtg cac agt aat gga aag acc tat tta cat tgg tac ctg cag aag cca    192
Val His Ser Asn Gly Lys Thr Tyr Leu His Trp Tyr Leu Gln Lys Pro
        50                  55                  60
ggc cag tct cca aaa ctc ctg atc tac aaa gtt tcc aac cga ttt tct    240
Gly Gln Ser Pro Lys Leu Leu Ile Tyr Lys Val Ser Asn Arg Phe Ser
65                  70                  75                  80
ggg gtc cca gac agg ttc agt ggc agt gga tca gtg aca gat ttc aca    288
Gly Val Pro Asp Arg Phe Ser Gly Ser Gly Ser Val Thr Asp Phe Thr
                85                  90                  95
ctc atg atc agc aga gtg gag gct gag gat ctg gga gtt tat ttc tgc    336
Leu Met Ile Ser Arg Val Glu Ala Glu Asp Leu Gly Val Tyr Phe Cys
            100                 105                 110
tct caa agt aca cat gtt ccg tac acg ttc gga ggg ggg acc aag ctg    384
Ser Gln Ser Thr His Val Pro Tyr Thr Phe Gly Gly Gly Thr Lys Leu
        115                 120                 125
gaa ata aaa c                                                      394
Glu Ile Lys
    130

<210> 8
<211> 409

<212> DNA
<213> Mus
<220>
<221> CDS
<222> (1)...(408)
<223> pGEM-M2H. 1-57;signal peptide, 58-409;mature peptide
<400> 8
atg gaa tgg agc tgg ata ttt ctc ttc ctc ctg tca gga act gca ggt 48
Met Glu Trp Ser Trp Ile Phe Leu Phe Leu Leu Ser Gly Thr Ala Gly
1               5                   10                  15
gtc cac tcc cag gtc cag ctg cag cag tct gga cct gaa ctg gta aag 96
Val His Ser Gln Val Gln Leu Gln Gln Ser Gly Pro Glu Leu Val Lys
            20                  25                  30
cct ggg gct tca gtg aag atg tcc tgc aag gct tct gga tac acc ttc 144
Pro Gly Ala Ser Val Lys Met Ser Cys Lys Ala Ser Gly Tyr Thr Phe
        35                  40                  45
gct aac cat gtt att cac tgg gtg aag cag aag cca ggg cag ggc ctt 192
Ala Asn His Val Ile His Trp Val Lys Gln Lys Pro Gly Gln Gly Leu
    50                  55                  60
gag tgg att gga tat att tat cct tac aat gat ggt act aag tat aat 240
Glu Trp Ile Gly Tyr Ile Tyr Pro Tyr Asn Asp Gly Thr Lys Tyr Asn
65                  70                  75                  80
gag aag ttc aag gac aag gcc act ctg act tca gac aaa tcc tcc acc 288
Glu Lys Phe Lys Asp Lys Ala Thr Leu Thr Ser Asp Lys Ser Ser Thr
                85                  90                  95
aca gcc tac atg gac ctc agc agc ctg gcc tct gag gac tct gcg gtc 336
Thr Ala Tyr Met Asp Leu Ser Ser Leu Ala Ser Glu Asp Ser Ala Val
            100                 105                 110

tat tac tgt gca aga ggg ggt tac tat act tac gac gac tgg ggc caa 384
Tyr Tyr Cys Ala Arg Gly Gly Tyr Tyr Thr Tyr Asp Asp Trp Gly Gln
        115                 120                 125
ggc acc act ctc aca gtc tcc tca g 409
Gly Thr Thr Leu Thr Val Ser Ser
    130                 135

<210> 9
<211> 32
<212> DNA
<213> Artificial Sequence
<220>
<223> PCR primer
<400> 9
cccaagcttc caccatgaag ttgcctgtta gg 32

<210> 10
<211> 32
<212> DNA
<213> Artificial Sequence
<220>
<223> PCR primer
<400> 10
cccaagcttc caccatggaa tggagctgga ta 32

<210> 11
<211> 34
<212> DNA

<213> Artificial Sequence

<220>

<223> PCR primer

<400> 11

cgcggatcca ctcacgtttt atttccagct tggt 34

<210> 12

<211> 34

<212> DNA

<213> Artificial Sequence

<220>

<223> PCR primer

<400> 12

cgcggatcca ctcacctgag gagactgtga gagt 34

<210> 13

<211> 30

<212> DNA

<213> Artificial Sequence

<220>

<223> PCR primer

<400> 13

catgccatgg cgcaggtcca gctgcagcag 30

<210> 14

<211> 27

<212> DNA

<213> Artificial Sequence

<220>

<223> PCR primer

<400> 14

accaccacct gaggagactg tgagagt   27

<210> 15

<211> 27

<212> DNA

<213> Artificial Sequence

<220>

<223> PCR primer

<400> 15

gtctcctcag gtggtggtgg ttcgggt   27

<210> 16

<211> 27

<212> DNA

<213> Artificial Sequence

<220>

<223> PCR primer

<400> 16
   cacaacatcc gatccgccac cacccga   27

<210> 17

<211> 27

<212> DNA

<213> Artificial Sequence

<220>

<223> PCR primer

<400> 17

ggcggatcgg atgttgtgat gacccaa   27

<210> 18

<211> 57

<212> DNA

<213> Artificial Sequence

<220>

<223> PCR primer

<400> 18

ccggaattct cattatttat cgtcatcgtc tttgtagtct tttatttcca gcttggt   57

<210> 19

<211> 45

<212> DNA

<213> Artificial Sequence

<220>

<223> Linker amino acid sequence and nucleotide sequence

<400> 19

ggt ggt ggt ggt tcg ggt ggt ggt ggt tcg ggt ggt ggc gga tcg   45

Gly Gly Gly Gly Ser Gly Gly Gly Gly Ser Gly Gly Gly Gly Ser

                    5                   10                  15

<210> 20

<211> 828

<212> DNA

<213> Mus

<220>

<221> CDS

<222> (1)...(822)

<223> pscM1. MABL1-scFv

<400> 20
   atg aaa tac cta ttg cct acg gca gcc gct gga ttg tta tta ctc gct 48
   Met Lys Tyr Leu Leu Pro Thr Ala Ala Ala Gly Leu Leu Leu Leu Ala
   1 5 10 15
   gcc caa cca gcc atg gcg cag gtc cag ctg cag cag tct gga cct gac 96
   Ala Gln Pro Ala Met Ala Gln Val Gln Leu Gln Gln Ser Gly Pro Asp
   20 25 30
   ctg gta aag cct ggg gct tca gtg aag atg tcc tgc aag gct tct gga 144
   Leu Val Lys Pro Gly Ala Ser Val Lys Met Ser Cys Lys Ala Ser Gly
   35 40 45
   tac acc ttc gtt aac cat gtt atg cac tgg gtg aag cag aag cca ggg 192
   Tyr Thr Phe Val Asn His Val Met His Trp Val Lys Gln Lys Pro Gly
   50 55 60
   cag ggc ctt gag tgg att gga tat att tat cct tac aat gat ggt act 240
   Gln Gly Leu Glu Trp Ile Gly Tyr Ile Tyr Pro Tyr Asn Asp Gly Thr
   65 70 75 80
   aag tac aat gag aag ttc aag ggc aag gcc aca ctg act tca gag aaa 288
   Lys Tyr Asn Glu Lys Phe Lys Gly Lys Ala Thr Leu Thr Ser Glu Lys
   85 90 95
   tcc tcc agc gca gcc tac atg gag ctc agc agc ctg gcc tct gag gac 336
   Ser Ser Ser Ala Ala Tyr Met Glu Leu Ser Ser Leu Ala Ser Glu Asp
   100 105 110
   tct gcg gtc tac tac tgt gca aga ggg ggt tac tat agt tac gac gac 384
   Ser Ala Val Tyr Tyr Cys Ala Arg Gly Gly Tyr Tyr Ser Tyr Asp Asp
   115 120 125
   tgg ggc caa ggc acc act ctc aca gtc tcc tca ggt ggt ggt ggt tcg 432
   Trp Gly Gln Gly Thr Thr Leu Thr Val Ser Ser Gly Gly Gly Gly Ser
   130 135 140
   ggt ggt ggt ggt tcg ggt ggt ggc gga tcg gat gtt gtg atg acc caa 480
   Gly Gly Gly Gly Ser Gly Gly Gly Gly Ser Asp Val Val Met Thr Gln
   145 150 155 160
   act cca ctc tcc ctg cct gtc agt ctt gga gat caa gcc tcc atc tct 528
   Thr Pro Leu Ser Leu Pro Val Ser Leu Gly Asp Gln Ala Ser Ile Ser
   165 170 175
   tgc aga tct agt cag agc ctt cta cac agt aaa gga aac acc tat tta 576
   Cys Arg Ser Ser Gln Ser Leu Leu His Ser Lys Gly Asn Thr Tyr Leu
   180 185 190
   caa tgg tac cta cag aag cca ggc cag tct cca aag ctc ctg atc tac 624
   Gln Trp Tyr Leu Gln Lys Pro Gly Gln Ser Pro Lys Leu Leu Ile Tyr
   195 200 205
   aaa gtt tcc aac cga ttt tct ggg gtc cca gac agg ttc agt ggc agt 672
   Lys Val Ser Asn Arg Phe Ser Gly Val Pro Asp Arg Phe Ser Gly Ser
   210 215 220
   gga tca ggg aca gat ttc aca ctc aag atc agc aga gtg gag gct gag 720
   Gly Ser Gly Thr Asp Phe Thr Leu Lys Ile Ser Arg Val Glu Ala Glu
   225 230 235 240
   gat ctg gga gtt tat ttc tgc tct caa agt aca cat gtt ccg tac acg 768
   Asp Leu Gly Val Tyr Phe Cys Ser Gln Ser Thr His Val Pro Tyr Thr
   245 250 255
   tcc gga ggg ggg acc aag ctg gaa ata aaa gac tac aaa gac gat gac 816
   Ser Gly Gly Gly Thr Lys Leu Glu Ile Lys Asp Tyr Lys Asp Asp Asp
   260 265 270
   gat aaa taatga                                                    828
   Asp Lys

<210> 21
<211> 31
<212> DNA
<213> Artificial Sequence
<220>
<223> PCR primer
<400> 21
acgcgtcgac tcccaggtcc agctgcagca g   31

<210> 22
<211> 18
<212> DNA
<213> Artificial Sequence
<220>
<223> PCR primer
<400> 22
gaaggtgtat ccagaagc   18

<210> 23
<211> 819
<212> DNA
<213> Mus
<220>
<221> CDS
<222> (1)...(813)

<223> pCHOM1. MABL1-scFv

<400> 23

atg gga tgg agc tgt atc atc ctc ttc ttg gta gca aca gct aca ggt    48

Met Gly Trp Ser Cys Ile Ile Leu Phe Leu Val Ala Thr Ala Thr Gly

1               5                   10                  15

gtc gac tcc cag gtc cag ctg cag cag tct gga cct gac ctg gta aag    96

Val Asp Ser Gln Val Gln Leu Gln Gln Ser Gly Pro Asp Leu Val Lys

            20                  25                  30

cct ggg gct tca gtg aag atg tcc tgc aag gct tct gga tac acc ttc    144

Pro Gly Ala Ser Val Lys Met Ser Cys Lys Ala Ser Gly Tyr Thr Phe

        35                  40                  45

gtt aac cat gtt atg cac tgg gtg aag cag aag cca ggg cag ggc ctt    192

Val Asn His Val Met His Trp Val Lys Gln Lys Pro Gly Gln Gly Leu

    50                  55                  60

gag tgg att gga tat att tat cct tac aat gat ggt act aag tac aat    240

Glu Trp Ile Gly Tyr Ile Tyr Pro Tyr Asn Asp Gly Thr Lys Tyr Asn

65                  70                  75                  80

gag aag ttc aag ggc aag gcc aca ctg act tca gag aaa tcc tcc agc    288

Glu Lys Phe Lys Gly Lys Ala Thr Leu Thr Ser Glu Lys Ser Ser Ser

                85                  90                  95

gca gcc tac atg gag ctc agc agc ctg gcc tct gag gac tct gcg gtc    336

Ala Ala Tyr Met Glu Leu Ser Ser Leu Ala Ser Glu Asp Ser Ala Val

            100                 105                 110

tac tac tgt gca aga ggg ggt tac tat agt tac gac gac tgg ggc caa    384

Tyr Tyr Cys Ala Arg Gly Gly Tyr Tyr Ser Tyr Asp Asp Trp Gly Gln

        115                 120                 125

ggc acc act ctc aca gtc tcc tca ggt ggt ggt ggt tcg ggt ggt ggt    432

Gly Thr Thr Leu Thr Val Ser Ser Gly Gly Gly Gly Ser Gly Gly Gly

    130                 135                 140

ggt tcg ggt ggt ggc gga tcg gat gtt gtg atg acc caa act cca ctc 480

Gly Ser Gly Gly Gly Gly Ser Asp Val Val Met Thr Gln Thr Pro Leu

145                 150                 155                 160

tcc ctg cct gtc agt ctt gga gat caa gcc tcc atc tct tgc aga tct 528

Ser Leu Pro Val Ser Leu Gly Asp Gln Ala Ser Ile Ser Cys Arg Ser

                165                 170                 175

agt cag agc ctt cta cac agt aaa gga aac acc tat tta caa tgg tac 576

Ser Gln Ser Leu Leu His Ser Lys Gly Asn Thr Tyr Leu Gln Trp Tyr

            180                 185                 190

cta cag aag cca ggc cag tct cca aag ctc ctg atc tac aaa gtt tcc 624

Leu Gln Lys Pro Gly Gln Ser Pro Lys Leu Leu Ile Tyr Lys Val Ser

        195                 200                 205

aac cga ttt tct ggg gtc cca gac agg ttc agt ggc agt gga tca ggg 672

Asn Arg Phe Ser Gly Val Pro Asp Arg Phe Ser Gly Ser Gly Ser Gly

    210                 215                 220

aca gat ttc aca ctc aag atc agc aga gtg gag gct gag gat ctg gga 720

Thr Asp Phe Thr Leu Lys Ile Ser Arg Val Glu Ala Glu Asp Leu Gly

225                 230                 235                 240

gtt tat ttc tgc tct caa agt aca cat gtt ccg tac acg tcc gga ggg 768

Val Tyr Phe Cys Ser Gln Ser Thr His Val Pro Tyr Thr Ser Gly Gly

                245                 250                 255

ggg acc aag ctg gaa ata aaa gac tac aaa gac gat gac gat aaa taa 816

Gly Thr Lys Leu Glu Ile Lys Asp Tyr Lys Asp Asp Asp Asp Lys

            260                 265                 270

tga 819

<210> 24

<211> 828

<212> DNA

<213> Mus

<220>

<221> CDS

<222> (1)...(822)

<223> pscM2. MABL2-scFv

<400> 24

atg aaa tac cta ttg cct acg gca gcc gct gga ttg tta tta ctc gct    48

Met Lys Tyr Leu Leu Pro Thr Ala Ala Ala Gly Leu Leu Leu Leu Ala

  1               5                  10                  15

gcc caa cca gcc atg gcg cag gtc cag ctg cag cag tct gga cct gaa    96

Ala Gln Pro Ala Met Ala Gln Val Gln Leu Gln Gln Ser Gly Pro Glu

             20                  25                  30

ctg gta aag cct ggg gct tca gtg aag atg tcc tgc aag gct tct gga   144

Leu Val Lys Pro Gly Ala Ser Val Lys Met Ser Cys Lys Ala Ser Gly

         35                  40                  45

tac acc ttc gct aac cat gtt att cac tgg gtg aag cag aag cca ggg   192

Tyr Thr Phe Ala Asn His Val Ile His Trp Val Lys Gln Lys Pro Gly

     50                  55                  60

cag ggc ctt gag tgg att gga tat att tat cct tac aat gat ggt act   240

Gln Gly Leu Glu Trp Ile Gly Tyr Ile Tyr Pro Tyr Asn Asp Gly Thr

 65                  70                  75                  80

aag tat aat gag aag ttc aag gac aag gcc act ctg act tca gac aaa   288

Lys Tyr Asn Glu Lys Phe Lys Asp Lys Ala Thr Leu Thr Ser Asp Lys

                 85                  90                  95

tcc tcc acc aca gcc tac atg gac ctc agc agc ctg gcc tct gag gac   336

Ser Ser Thr Thr Ala Tyr Met Asp Leu Ser Ser Leu Ala Ser Glu Asp

            100                 105                 110

tct gcg gtc tat tac tgt gca aga ggg ggt tac tat act tac gac gac 384

Ser Ala Val Tyr Tyr Cys Ala Arg Gly Gly Tyr Tyr Thr Tyr Asp Asp

        115                 120                 125

tgg ggc caa ggc acc act ctc aca gtc tcc tca ggt ggt ggt ggt tcg 432

Trp Gly Gln Gly Thr Thr Leu Thr Val Ser Ser Gly Gly Gly Gly Ser

    130                 135                 140

ggt ggt ggt ggt tcg ggt ggt ggc gga tcg gat gtt gtg atg acc caa 480

Gly Gly Gly Gly Ser Gly Gly Gly Gly Ser Asp Val Val Met Thr Gln

145                 150                 155                 160

agt cca ctc tcc ctg cct gtc agt ctt gga gat caa gcc tcc atc tct 528

Ser Pro Leu Ser Leu Pro Val Ser Leu Gly Asp Gln Ala Ser Ile Ser

                165                 170                 175

tgc aga tca agt cag agc ctt gtg cac agt aat gga aag acc tat tta 576

Cys Arg Ser Ser Gln Ser Leu Val His Ser Asn Gly Lys Thr Tyr

            180                 185                 190

cat tgg tac ctg cag aag cca ggc cag tct cca aaa ctc ctg atc tac 624

His Trp Tyr Leu Gln Lys Pro Gly Gln Ser Pro Lys Leu Leu Ile Tyr

            195                 200                 205

aaa gtt tcc aac cga ttt tct ggg gtc cca gac agg ttc agt ggc agt 672

Lys Val Ser Asn Arg Phe Ser Gly Val Pro Asp Arg Phe Ser Gly Ser

        210                 215                 220

gga tca gtg aca gat ttc aca ctc atg atc agc aga gtg gag gct gag 720

Gly Ser Val Thr Asp Phe Thr Leu Met Ile Ser Arg Val Glu Ala Glu

225 230 235 240

gat ctg gga gtt tat ttc tgc tct caa agt aca cat gtt ccg tac acg 768

Asp Leu Gly Val Tyr Phe Cys Ser Gln Ser Thr His Val Pro Tyr Thr

245 250 255

ttc gga ggg ggg acc aag ctg gaa ata aaa gac tac aaa gac gat gac 816
Phe Gly Gly Gly Thr Lys Leu Glu Ile Lys Asp Tyr Lys Asp Asp Asp
                260                 265                 270
gat aaa taatga 828
Asp Lys

<210> 25
<211> 819
<212> DNA
<213> Mus
<220>
<221> CDS
<222> (1)...(813)
<223> pCHOM2. MABL2-scFv
<400> 25
atg gga tgg agc tgt atc atc ctc ttc ttg gta gca aca gct aca ggt 48
Met Gly Trp Ser Cys Ile Ile Leu Phe Leu Val Ala Thr Ala Thr Gly
1               5                   10                  15
gtc gac tcc cag gtc cag ctg cag cag tct gga cct gaa ctg gta aag 96
Val Asp Ser Gln Val Gln Leu Gln Gln Ser Gly Pro Glu Leu Val Lys
            20                  25                  30
cct ggg gct tca gtg aag atg tcc tgc aag gct tct gga tac acc ttc 144
Pro Gly Ala Ser Val Lys Met Ser Cys Lys Ala Ser Gly Tyr Thr Phe
        35                  40                  45
gct aac cat gtt att cac tgg gtg aag cag aag cca ggg cag ggc ctt 192
Ala Asn His Val Ile His Trp Val Lys Gln Lys Pro Gly Gln Gly Leu
    50                  55                  60
gag tgg att gga tat att tat cct tac aat gat ggt act aag tat aat 240

Glu Trp Ile Gly Tyr Ile Tyr Pro Tyr Asn Asp Gly Thr Lys Tyr Asn
65                  70                  75                  80
gag aag ttc aag gac aag gcc act ctg act tca gac aaa tcc tcc acc    288
Glu Lys Phe Lys Asp Lys Ala Thr Leu Thr Ser Asp Lys Ser Ser Thr
                85                  90                  95
aca gcc tac atg gac ctc agc agc ctg gcc tct gag gac tct gcg gtc    336
Thr Ala Tyr Met Asp Leu Ser Ser Leu Ala Ser Glu Asp Ser Ala Val
            100                 105                 110
tat tac tgt gca aga ggg ggt tac tat act tac gac gac tgg ggc caa    384
Tyr Tyr Cys Ala Arg Gly Gly Tyr Tyr Thr Tyr Asp Asp Trp Gly Gln
        115                 120                 125
ggc acc act ctc aca gtc tcc tca ggt ggt ggt ggt tcg ggt ggt ggt    432
Gly Thr Thr Leu Thr Val Ser Ser Gly Gly Gly Gly Ser Gly Gly Gly
    130                 135                 140
ggt tcg ggt ggt ggc gga tcg gat gtt gtg atg acc caa agt cca ctc    480
Gly Ser Gly Gly Gly Gly Ser Asp Val Val Met Thr Gln Ser Pro Leu
145                 150                 155                 160
tcc ctg cct gtc agt ctt gga gat caa gcc tcc atc tct tgc aga tca    528
Ser Leu Pro Val Ser Leu Gly Asp Gln Ala Ser Ile Ser Cys Arg Ser
                165                 170                 175
agt cag agc ctt gtg cac agt aat gga aag acc tat tta cat tgg tac    576
Ser Gln Ser Leu Val His Ser Asn Gly Lys Thr Tyr Leu His Trp Tyr
            180                 185                 190
ctg cag aag cca ggc cag tct cca aaa ctc ctg atc tac aaa gtt tcc    624
Leu Gln Lys Pro Gly Gln Ser Pro Lys Leu Leu Ile Tyr Lys Val Ser
        195                 200                 205
aac cga ttt tct ggg gtc cca gac agg ttc agt ggc agt gga tca gtg    672
Asn Arg Phe Ser Gly Val Pro Asp Arg Phe Ser Gly Ser Gly Ser Val

210                    215                    220

aca gat ttc aca ctc atg atc agc aga gtg gag gct gag gat ctg gga   720

Thr Asp Phe Thr Leu Met Ile Ser Arg Val Glu Ala Glu Asp Leu Gly

225                    230                    235                    240

gtt tat ttc tgc tct caa agt aca cat gtt ccg tac acg ttc gga ggg   768

Val Tyr Phe Cys Ser Gln Ser Thr His Val Pro Tyr Thr Phe Gly Gly

                245                    250                    255

ggg acc aag ctg gaa ata aaa gac tac aaa gac gat gac gat aaa taa   816

Gly Thr Lys Leu Glu Ile Lys Asp Tyr Lys Asp Asp Asp Asp Lys

            260                    265                    270

tga                                                               819

<210> 26

<211> 456

<212> DNA

<213> Mus

<220>

<221> CDS

<222> (1)...(450)

<223> pCHO-shIAP. Soluble human IAP

<400> 26

atg tgg ccc ctg gta gcg gcg ctg ttg ctg ggc tcg gcg tgc tgc gga    48

Met Trp Pro Leu Val Ala Ala Leu Leu Leu Gly Ser Ala Cys Cys Gly

  1                5                   10                   15

tca gct cag cta cta ttt aat aaa aca aaa tct gta gaa ttc acg ttt    96

Ser Ala Gln Leu Leu Phe Asn Lys Thr Lys Ser Val Glu Phe Thr Phe

                20                   25                   30

tgt aat gac act gtc gtc att cca tgc ttt gtt act aat atg gag gca   144

Cys Asn Asp Thr Val Val Ile Pro Cys Phe Val Thr Asn Met Glu Ala
        35                  40                  45
caa aac act act gaa gta tac gta aag tgg aaa ttt aaa gga aga gat 192
Gln Asn Thr Thr Glu Val Tyr Val Lys Trp Lys Phe Lys Gly Arg Asp
    50                  55                  60
att tac acc ttt gat gga gct cta aac aag tcc act gtc ccc act gac 240
Ile Tyr Thr Phe Asp Gly Ala Leu Asn Lys Ser Thr Val Pro Thr Asp
65                  70                  75                  80
ttt agt agt gca aaa att gaa gtc tca caa tta cta aaa gga gat gcc 288
Phe Ser Ser Ala Lys Ile Glu Val Ser Gln Leu Leu Lys Gly Asp Ala
                85                  90                  95
tct ttg aag atg gat aag agt gat gct gtc tca cac aca gga aac tac 336
Ser Leu Lys Met Asp Lys Ser Asp Ala Val Ser His Thr Gly Asn Tyr
            100                 105                 110
act tgt gaa gta aca gaa tta acc aga gaa ggt gaa acg atc atc gag 384
Thr Cys Glu Val Thr Glu Leu Thr Arg Glu Gly Glu Thr Ile Ile Glu
        115                 120                 125
cta aaa tat cgt gtt gtt tca tgg ttt tct cca aat gaa aat gac tac 432
Leu Lys Tyr Arg Val Val Ser Trp Phe Ser Pro Asn Glu Asn Asp Tyr
    130                 135                 140
aag gac gac gat gac aag tgatag 456
Lys Asp Asp Asp Asp Lys
145                 150

<210> 27
<211> 46
<212> DNA
<213> Artificial Sequence

<220>

<223> PCR primer

<400> 27

ggaattccat atgcaagtgc aacttcaaca gtctggacct gaactg   46

<210> 28

<211> 31

<212> DNA

<213> Artificial Sequence

<220>

<223> PCR primer

<400> 28

ggaattctca ttattttatt tccagcttgg t   31

<210> 29

<211> 741

<212> DNA

<213> Mus

<220>

<221> CDS

<222> (1)...(735)

<223> pscM2DEm02. MABL2-scFv

<400> 29

atg caa gtg caa ctt caa cag tct gga cct gaa ctg gta aag cct ggg   48

Met Gln Val Gln Leu Gln Gln Ser Gly Pro Glu Leu Val Lys Pro Gly

 1               5                  10                  15

gct tca gtg aag atg tcc tgc aag gct tct gga tac acc ttc gct aac   96

Ala Ser Val Lys Met Ser Cys Lys Ala Ser Gly Tyr Thr Phe Ala Asn

20                  25                  30
cat gtt att cac tgg gtg aag cag aag cca ggg cag ggc ctt gag tgg   144
His Val Ile His Trp Val Lys Gln Lys Pro Gly Gln Gly Leu Glu Trp
        35                  40                  45
att gga tat att tat cct tac aat gat ggt act aag tat aat gag aag   192
Ile Gly Tyr Ile Tyr Pro Tyr Asn Asp Gly Thr Lys Tyr Asn Glu Lys
    50                  55                  60
ttc aag gac aag gcc act ctg act tca gac aaa tcc tcc acc aca gcc   240
Phe Lys Asp Lys Ala Thr Leu Thr Ser Asp Lys Ser Ser Thr Thr Ala
65                  70                  75                  80
tac atg gac ctc agc agc ctg gcc tct gag gac tct gcg gtc tat tac   288
Tyr Met Asp Leu Ser Ser Leu Ala Ser Glu Asp Ser Ala Val Tyr Tyr
                85                  90                  95
tgt gca aga ggg ggt tac tat act tac gac gac tgg ggc caa ggc acc   336
Cys Ala Arg Gly Gly Tyr Tyr Thr Tyr Asp Asp Trp Gly Gln Gly Thr
            100                 105                 110
act ctc aca gtc tcc tca ggt ggt ggt ggt tcg ggt ggt ggt ggt tcg   384
Thr Leu Thr Val Ser Ser Gly Gly Gly Gly Ser Gly Gly Gly Gly Ser
        115                 120                 125
ggt ggt ggc gga tcg gat gtt gtg atg acc caa agt cca ctc tcc ctg   432
Gly Gly Gly Gly Ser Asp Val Val Met Thr Gln Ser Pro Leu Ser Leu
    130                 135                 140
cct gtc agt ctt gga gat caa gcc tcc atc tct tgc aga tca agt cag   480
Pro Val Ser Leu Gly Asp Gln Ala Ser Ile Ser Cys Arg Ser Ser Gln
145                 150                 155                 160
agc ctt gtg cac agt aat gga aag acc tat tta cat tgg tac ctg cag   528
Ser Leu Val His Ser Asn Gly Lys Thr Tyr Leu His Trp Tyr Leu Gln
                165                 170                 175

aag cca ggc cag tct cca aaa ctc ctg atc tac aaa gtt tcc aac cga 576
Lys Pro Gly Gln Ser Pro Lys Leu Leu Ile Tyr Lys Val Ser Asn Arg
            180                 185                 190
ttt tct ggg gtc cca gac agg ttc agt ggc agt gga tca gtg aca gat 624
Phe Ser Gly Val Pro Asp Arg Phe Ser Gly Ser Gly Ser Val Thr Asp
        195                 200                 205
ttc aca ctc atg atc agc aga gtg gag gct gag gat ctg gga gtt tat 672
Phe Thr Leu Met Ile Ser Arg Val Glu Ala Glu Asp Leu Gly Val Tyr
    210                 215                 220
ttc tgc tct caa agt aca cat gtt ccg tac acg ttc gga ggg ggg acc 720
Phe Cys Ser Gln Ser Thr His Val Pro Tyr Thr Phe Gly Gly Gly Thr
225                 230                 235                 240
aag ctg gaa ata aaa taatga 741
Lys Leu Glu Ile Lys
                245

<210> 30
<211> 18
<212> DNA
<213> Artificial Sequence
<220>
<223> PCR primer
<400> 30
cagacagtgg ttcaaagt 18

<210> 31
<211> 72
<212> DNA

<213> Artificial Sequence

<220>

<223> PCR primer

<400> 31

cgcgtcgacc gatccgccac cacccgaacc accaccaccc gaaccaccac caccttttat 60

ttccagcttg gt 72

<210> 32

<211> 1605

<212> DNA

<213> Mus

<220>

<221> CDS

<222> (1)...(1599)

<223> pCHOM2(Fv)2. MABL2-sc(Fv)2

<400> 32
   atg gga tgg agc tgt atc atc ctc ttc ttg gta gca aca gct aca ggt 48
   Met Gly Trp Ser Cys Ile Ile Leu Phe Leu Val Ala Thr Ala Thr Gly
   1 5 10 15
   gtc gac tcc cag gtc cag ctg cag cag tct gga cct gaa ctg gta aag 96
   Val Asp Ser Gln Val Gln Leu Gln Gln Ser Gly Pro Glu Leu Val Lys
   20 25 30
   cct ggg gct tca gtg aag atg tcc tgc aag gct tct gga tac acc ttc 144
   Pro Gly Ala Ser Val Lys Met Ser Cys Lys Ala Ser Gly Tyr Thr Phe
   35 40 45
   gct aac cat gtt att cac tgg gtg aag cag aag cca ggg cag ggc ctt 192
   Ala Asn His Val Ile His Trp Val Lys Gln Lys Pro Gly Gln Gly Leu
   50 55 60
   gag tgg att gga tat att tat cct tac aat gat ggt act aag tat aat 240
   Glu Trp Ile Gly Tyr Ile Tyr Pro Tyr Asn Asp Gly Thr Lys Tyr Asn
   65 70 75 80
   gag aag ttc aag gac aag gcc act ctg act tca gac aaa tcc tcc acc 288
   Glu Lys Phe Lys Asp Lys Ala Thr Leu Thr Ser Asp Lys Ser Ser Thr
   85 90 95
   aca gcc tac atg gac ctc agc agc ctg gcc tct gag gac tct gcg gtc 336
   Thr Ala Tyr Met Asp Leu Ser Ser Leu Ala Ser Glu Asp Ser Ala Val
   100 105 110
   tat tac tgt gca aga ggg ggt tac tat act tac gac gac tgg ggc caa 384
   Tyr Tyr Cys Ala Arg Gly Gly Tyr Tyr Thr Tyr Asp Asp Trp Gly Gln
   115 120 125
   ggc acc act ctc aca gtc tcc tca ggt ggt ggt ggt tcg ggt ggt ggt 432
   Gly Thr Thr Leu Thr Val Ser Ser Gly Gly Gly Gly Ser Gly Gly Gly
   130 135 140
   ggt tcg ggt ggt ggc gga tcg gat gtt gtg atg acc caa agt cca ctc 480
   Gly Ser Gly Gly Gly Gly Ser Asp Val Val Met Thr Gln Ser Pro Leu
   145 150 155 160
   tcc ctg cct gtc agt ctt gga gat caa gcc tcc atc tct tgc aga tca 528
   Ser Leu Pro Val Ser Leu Gly Asp Gln Ala Ser Ile Ser Cys Arg Ser
   165 170 175
   agt cag agc ctt gtg cac agt aat gga aag acc tat tta cat tgg tac 576
   Ser Gln Ser Leu Val His Ser Asn Gly Lys Thr Tyr Leu His Trp Tyr
   180 185 190
   ctg cag aag cca ggc cag tct cca aaa ctc ctg atc tac aaa gtt tcc 624
   Leu Gln Lys Pro Gly Gln Ser Pro Lys Leu Leu Ile Tyr Lys Val Ser
   195 200 205
   aac cga ttt tct ggg gtc cca gac agg ttc agt ggc agt gga tca gtg 672
   Asn Arg Phe Ser Gly Val Pro Asp Arg Phe Ser Gly Ser Gly Ser Val
   210 215 220
   aca gat ttc aca ctc atg atc agc aga gtg gag gct gag gat ctg gga 720
   Thr Asp Phe Thr Leu Met Ile Ser Arg Val Glu Ala Glu Asp Leu Gly
   225 230 235 240
   gtt tat ttc tgc tct caa agt aca cat gtt ccg tac acg ttc gga ggg 768
   Val Tyr Phe Cys Ser Gln Ser Thr His Val Pro Tyr Thr Phe Gly Gly
   245 250 255
   ggg acc aag ctg gaa ata aaa ggt ggt ggt ggt tcg ggt ggt ggt ggt 816
   Gly Thr Lys Leu Glu Ile Lys Gly Gly Gly Gly Ser Gly Gly Gly Gly
   260 265 270
   tcg ggt ggt ggc gga tcg gtc gac tcc cag gtc cag ctg cag cag tct 864
   Ser Gly Gly Gly Gly Ser Val Asp Ser Gln Val Gln Leu Gln Gln Ser
   275 280 285
   gga cct gaa ctg gta aag cct ggg gct tca gtg aag atg tcc tgc aag 912
   Gly Pro Glu Leu Val Lys Pro Gly Ala Ser Val Lys Met Ser Cys Lys
   290 295 300
   gct tct gga tac acc ttc gct aac cat gtt att cac tgg gtg aag cag 960
   Ala Ser Gly Tyr Thr Phe Ala Asn His Val Ile His Trp Val Lys Gln
   305 310 315 320
   aag cca ggg cag ggc ctt gag tgg att gga tat att tat cct tac aat 1008
   Lys Pro Gly Gln Gly Leu Glu Trp Ile Gly Tyr Ile Tyr Pro Tyr Asn
   325 330 335
   gat ggt act aag tat aat gag aag ttc aag gac aag gcc act ctg act 1056
   Asp Gly Thr Lys Tyr Asn Glu Lys Phe Lys Asp Lys Ala Thr Leu Thr
   340 345 350
   tca gac aaa tcc tcc acc aca gcc tac atg gac ctc agc agc ctg gcc 1104
   Ser Asp Lys Ser Ser Thr Thr Ala Tyr Met Asp Leu Ser Ser Leu Ala
   355 360 365
   tct gag gac tct gcg gtc tat tac tgt gca aga ggg ggt tac tat act 1152
   Ser Glu Asp Ser Ala Val Tyr Tyr Cys Ala Arg Gly Gly Tyr Tyr Thr
   370 375 380
   tac gac gac tgg ggc caa ggc acc act ctc aca gtc tcc tca ggt ggt 1200
   Tyr Asp Asp Trp Gly Gln Gly Thr Thr Leu Thr Val Ser Ser Gly Gly
   385 390 395 400
   ggt ggt tcg ggt ggt ggt ggt tcg ggt ggt ggc gga tcg gat gtt gtg 1248
   Gly Gly Ser Gly Gly Gly Gly Ser Gly Gly Gly Gly Ser Asp Val Val
   405 410 415
   atg acc caa agt cca ctc tcc ctg cct gtc agt ctt gga gat caa gcc 1296
   Met Thr Gln Ser Pro Leu Ser Leu Pro Val Ser Leu Gly Asp Gln Ala
   420 425 430
   tcc atc tct tgc aga tca agt cag agc ctt gtg cac agt aat gga aag 1344
   Ser Ile Ser Cys Arg Ser Ser Gln Ser Leu Val His Ser Asn Gly Lys
   435 440 445
   acc tat tta cat tgg tac ctg cag aag cca ggc cag tct cca aaa ctc 1392
   Thr Tyr Leu His Trp Tyr Leu Gln Lys Pro Gly Gln Ser Pro Lys Leu
   450 455 460
   ctg atc tac aaa gtt tcc aac cga ttt tct ggg gtc cca gac agg ttc 1440
   Leu Ile Tyr Lys Val Ser Asn Arg Phe Ser Gly Val Pro Asp Arg Phe
   465 470 475 480
   agt ggc agt gga tca gtg aca gat ttc aca ctc atg atc agc aga gtg 1488
   Ser Gly Ser Gly Ser Val Thr Asp Phe Thr Leu Met Ile Ser Arg Val
   485 490 495
   gag gct gag gat ctg gga gtt tat ttc tgc tct caa agt aca cat gtt 1536
   Glu Ala Glu Asp Leu Gly Val Tyr Phe Cys Ser Gln Ser Thr His Val
   500 505 510
   ccg tac acg ttc gga ggg ggg acc aag ctg gaa ata aaa gac tac aaa 1584
   Pro Tyr Thr Phe Gly Gly Gly Thr Lys Leu Glu Ile Lys Asp Tyr Lys
   515 520 525
   gac gat gac gat aaa taatga 1605
   Asp Asp Asp Asp Lys
   530

<210> 33
<211> 23
<212> DNA
<213> Artificial Sequence
<220>
<223> PCR primer
<400> 33
tgaggaattc ccaccatggg atg 33

<210> 34
<211> 40
<212> DNA
<213> Artificial Sequence
<220>
<223> PCR primer
<400> 34
cacgacgtca ctcgagactg tgagagtggt gccttggccc 40

<210> 35
<211> 40
<212> DNA

<213> Artificial Sequence

<220>

<223> PCR primer

<400> 35

agtctcgagt gacgtcgtga tgacccaaag tccactctcc   40

<210> 36

<211> 31

<212> DNA

<213> Artificial Sequence

<220>

<223> PCR primer

<400> 36

gactggatcc tcattattta tcgtcatcgt c   31

<210> 37

<211> 22

<212> DNA

<213> Artificial Sequence

<220>

<223> PCR primer

<400> 37

cgcgtaatac gactcactat ag   22

<210> 38

<211> 46

<212> DNA

<213> Artificial Sequence

<220>

<223> PCR primer

<400> 38

gcaattggac ctgttttatc tcgagcttgg tcccccctcc gaacgt 46

<210> 39

<211> 45

<212> DNA

<213> Artificial Sequence

<220>

<223> PCR primer

<400> 39

gctcgagata aaacaggtcc aattgcagca gtctggacct gaact 45

<210> 40

<211> 60

<212> DNA

<213> Artificial Sequence

<220>

<223> PCR primer

<400> 40

gactggatcc tcattattta tcgtcatcgt ctttgtagtc tgaggagact gtgagagtgg 60

<210> 41

<211> 32

<212> DNA

<213> Artificial Sequence

<220>

<223> PCR primer

<400> 41

gactgaattc ccaccatgaa gttgcctgtt ag   32

<210> 42

<211> 40

<212> DNA

<213> Artificial Sequence

<220>

<223> PCR primer

<400> 42

cagtctcgag tggtggttcc gacgtcgtga tgacccaaag   40

<210> 43

<211> 43

<212> DNA

<213> Artificial Sequence

<220>

<223> PCR primer

<400> 43

cagtctcgag tggtggtggt tccgacgtcg tgatgaccca aag   43

<210> 44

<211> 46

<212> DNA

<213> Artificial Sequence

<220>

<223> PCR primer

<400> 44

cagtctcgag tggtggtggt ggttccgacg tcgtgatgac ccaaag   46

<210> 45

<211> 49

<212> DNA

<213> Artificial Sequence

<220>

<223> PCR primer

<400> 45

cagtctcgag tggtggtggt ggtggttccg acgtcgtgat gacccaaag   49

<210> 46

<211> 52

<212> DNA

<213> Artificial Sequence

<220>

<223> PCR primer

<400> 46

cagtctcgag tggtggtggt ggtggtggtt ccgacgtcgt gatgacccaa ag   52

<210> 47

<211> 20

<212> DNA

<213> Artificial Sequence

<220>

<223> PCR primer

<400> 47

ggccgcatgt tgtcacgaat 20

<210> 48
<211> 780
<212> DNA
<213> Mus
<220>
<221> CDS
<222> (1)...(768)
<223> CF2HL-0/pCOS1. MABL2-scFv<HL-0>
<400> 48
atg gga tgg agc tgt atc atc ctc ttc ttg gta gca aca gct aca ggt gtc 51
MET Gly Trp Ser Cys Ile Ile Leu Phe Leu Val Ala Thr Ala Thr Gly Val
                5                   10                  15
gac tcc cag gtc cag ctg cag cag tct gga cct gaa ctg gta aag cct ggg 102
Asp Ser Gln Val Gln Leu Gln Gln Ser Gly Pro Glu Leu Val Lys Pro Gly
        20                  25                  30
gct tca gtg aag atg tcc tgc aag gct tct gga tac acc ttc gct aac cat 153
Ala Ser Val Lys MET Ser Cys Lys Ala Ser Gly Tyr Thr Phe Ala Asn His
35                  40                  45                  50
gtt att cac tgg gtg aag cag aag cca ggg cag ggc ctt gag tgg att gga 204
Val Ile His Trp Val Lys Gln Lys Pro Gly Gln Gly Leu Glu Trp Ile Gly
            55                  60                  65
tat att tat cct tac aat gat ggt act aag tat aat gag aag ttc aag gac 255
Tyr Ile Tyr Pro Tyr Asn Asp Gly Thr Lys Tyr Asn Glu Lys Phe Lys Asp
    70                  75                  80                  85
aag gcc act ctg act tca gac aaa tcc tcc acc aca gcc tac atg gac ctc 306
Lys Ala Thr Leu Thr Ser Asp Lys Ser Ser Thr Thr Ala Tyr MET Asp Leu

                90                  95                  100

agc agc ctg gcc tct gag gac tct gcg gtc tat tac tgt gca aga ggg ggt 357

Ser Ser Leu Ala Ser Glu Asp Ser Ala Val Tyr Tyr Cys Ala Arg Gly Gly

        105                 110                 115

tac tat act tac gac gac tgg ggc caa ggc acc act ctc aca gtc tcg agt 408

Tyr Tyr Thr Tyr Asp Asp Trp Gly Gln Gly Thr Thr Leu Thr Val Ser Ser

120                 125                 130                 135

gac gtc gtg atg acc caa agt cca ctc tcc ctg cct gtc agt ctt gga gat 459

Asp Val Val MET Thr Gln Ser Pro Leu Ser Leu Pro Val Ser Leu Gly Asp

            140                 145                 150

caa gcc tcc atc tct tgc aga tca agt cag agc ctt gtg cac agt aat gga 510

Gln Ala Ser Ile Ser Cys Arg Ser Ser Gln Ser Leu Val His Ser Asn Gly

    155                 160                 165                 170

aag acc tat tta cat tgg tac ctg cag aag cca ggc cag tct cca aaa ctc 561

Lys Thr Tyr Leu His Trp Tyr Leu Gln Lys Pro Gly Gln Ser Pro Lys Leu

                175                 180                 185

ctg atc tac aaa gtt tcc aac cga ttt tct ggg gtc cca gac agg ttc agt 612

Leu Ile Tyr Lys Val Ser Asn Arg Phe Ser Gly Val Pro Asp Arg Phe Ser

        190                 195                 200

ggc agt gga tca gtg aca gat ttc aca ctc atg atc agc aga gtg gag gct 663

Gly Ser Gly Ser Val Thr Asp Phe Thr Leu MET Ile Ser Arg Val Glu Ala

205                 210                 215                 220

gag gat ctg gga gtt tat ttc tgc tct caa agt aca cat gtt ccg tac acg 714

Glu Asp Leu Gly Val Tyr Phe Cys Ser Gln Ser Thr His Val Pro Tyr Thr

            225                 230                 235

ttc gga ggg ggg acc aag ctg gaa ata aaa gac tac aaa gac gat gac gat 765

Phe Gly Gly Gly Thr Lys Leu Glu Ile Lys Asp Tyr Lys Asp Asp Asp Asp

    240                 245                 250                 255

aaa taa tga gga tcc 780
Lys

<210> 49
<211> 45
<212> DNA
<213> Artificial Sequence
<220>
<223> PCR primer
<400> 49
caagctcgag ataaaatccg gaggccaggt ccaattgcag cagtc 45

<210> 50
<211> 48
<212> DNA
<213> Artificial Sequence
<220>
<223> PCR primer
<400> 50
caagctcgag ataaaatccg gaggtggcca ggtccaattg cagcagtc 48

<210> 51
<211> 51
<212> DNA
<213> Artificial Sequence
<220>
<223> PCR primer
<400> 51

caagctcgag ataaaatccg gaggtggtgg ccaggtccaa ttgcagcagt c 51

<210> 52
<211> 54
<212> DNA
<213> Artificial Sequence
<220>
<223> PCR primer
<400> 52
caagctcgag ataaaatccg gaggtggtgg tggccaggtc caattgcagc agtc 54

<210> 53
<211> 57
<212> DNA
<213> Artificial Sequence
<220>
<223> PCR primer
<400> 53
caagctcgag ataaaatccg gaggtggtgg tggtggccag gtccaattgc agcagtc 57

<210> 54
<211> 780
<212> DNA
<213> Mus
<220>
<221> CDS
<222> (1)...(768)
<223> CF2LH-0/pCOS1. MABL2-scFv<LH-0>

<400> 54

atg aag ttg cct gtt agg ctg ttg gtg ctg atg ttc tgg att cct ggt tcc 51

MET Lys Leu Pro Val Arg Leu Leu Val Leu MET Phe Trp Ile Pro Gly Ser

                5                   10                  15

agc agt gat gtt gtg atg acc caa agt cca ctc tcc ctg cct gtc agt ctt 102

Ser Ser Asp Val Val MET Thr Gln Ser Pro Leu Ser Leu Pro Val Ser Leu

        20                  25                  30

gga gat caa gcc tcc atc tct tgc aga tca agt cag agc ctt gtg cac agt 153

Gly Asp Gln Ala Ser Ile Ser Cys Arg Ser Ser Gln Ser Leu Val His Ser

35                  40                  45                  50

aat gga aag acc tat tta cat tgg tac ctg cag aag cca ggc cag tct cca 204

Asn Gly Lys Thr Tyr Leu His Trp Tyr Leu Gln Lys Pro Gly Gln Ser Pro

            55                  60                  65

aaa ctc ctg atc tac aaa gtt tcc aac cga ttt tct ggg gtc cca gac agg 255

Lys Leu Leu Ile Tyr Lys Val Ser Asn Arg Phe Ser Gly Val Pro Asp Arg

    70                  75                  80                  85

ttc agt ggc agt gga tca gtg aca gat ttc aca ctc atg atc agc aga gtg 306

Phe Ser Gly Ser Gly Ser Val Thr Asp Phe Thr Leu MET Ile Ser Arg Val

                90                  95                  100

gag gct gag gat ctg gga gtt tat ttc tgc tct caa agt aca cat gtt ccg 357

Glu Ala Glu Asp Leu Gly Val Tyr Phe Cys Ser Gln Ser Thr His Val Pro

        105                 110                 115

tac acg ttc gga ggg ggg acc aag ctc gag ata aaa cag gtc caa ttg cag 408

Tyr Thr Phe Gly Gly Gly Thr Lys Leu Glu Ile Lys Gln Val Gln Leu Gln

120                 125                 130                 135

cag tct gga cct gaa ctg gta aag cct ggg gct tca gtg aag atg tcc tgc 459

Gln Ser Gly Pro Glu Leu Val Lys Pro Gly Ala Ser Val Lys MET Ser Cys

            140                 145                 150

aag gct tct gga tac acc ttc gct aac cat gtt att cac tgg gtg aag cag 510
Lys Ala Ser Gly Tyr Thr Phe Ala Asn His Val Ile His Trp Val Lys Gln
    155                 160                 165                 170
aag cca ggg cag ggc ctt gag tgg att gga tat att tat cct tac aat gat 561
Lys Pro Gly Gln Gly Leu Glu Trp Ile Gly Tyr Ile Tyr Pro Tyr Asn Asp
                175                 180                 185
ggt act aag tat aat gag aag ttc aag gac aag gcc act ctg act tca gac 612
Gly Thr Lys Tyr Asn Glu Lys Phe Lys Asp Lys Ala Thr Leu Thr Ser Asp
        190                 195                 200
aaa tcc tcc acc aca gcc tac atg gac ctc agc agc ctg gcc tct gag gac 663
Lys Ser Ser Thr Thr Ala Tyr MET Asp Leu Ser Ser Leu Ala Ser Glu Asp
205                 210                 215                 220
tct gcg gtc tat tac tgt gca aga ggg ggt tac tat act tac gac gac tgg 714
Ser Ala Val Tyr Tyr Cys Ala Arg Gly Gly Tyr Tyr Thr Tyr Asp Asp Trp
            225                 230                 235
ggc caa ggc acc act ctc aca gtc tcc tca gac tac aaa gac gat gac gat 765
Gly Gln Gly Thr Thr Leu Thr Val Ser Ser Asp Tyr Lys Asp Asp Asp Asp
    240                 245                 250                 255
aaa taa tga gga tcc 780
Lys

<210> 55
<211> 351
<212> DNA
<213> Human
<220>
<221> CDS
<222> (1)...(351)

<223> 12B5HV. 1-351 peptide

<400> 55

cag gtg cag ctg gtg cag tct ggg gga ggc ttg gtc cgg ccc ggg ggg    48

Gln Val Gln Leu Val Gln Ser Gly Gly Gly Leu Val Arg Pro Gly Gly

1               5                   10                  15

tcc ctg agt ctc tcc tgt gca gtc tct gga atc acc ctc agg acc tac    96

Ser Leu Ser Leu Ser Cys Ala Val Ser Gly Ile Thr Leu Arg Thr Tyr

            20                  25                  30

ggc atg cac tgg gtc cgc cag gct cca ggc aag ggg ctg gag tgg gtg    144

Gly Met His Trp Val Arg Gln Ala Pro Gly Lys Gly Leu Glu Trp Val

        35                  40                  45

gca ggt ata tcc ttt gac gga aga agt gaa tac tat gca gac tcc gtg    192

Ala Gly Ile Ser Phe Asp Gly Arg Ser Glu Tyr Tyr Ala Asp Ser Val

    50                  55                  60

cag ggc cga ttc acc atc tcc aga gac agt tcc aag aac acc ctg tat    240

Gln Gly Arg Phe Thr Ile Ser Arg Asp Ser Ser Lys Asn Thr Leu Tyr

65                  70                  75                  80

ctg caa atg aac agc ctg aga gcc gag gac acg gct gtg tat tac tgt    288

Leu Gln Met Asn Ser Leu Arg Ala Glu Asp Thr Ala Val Tyr Tyr Cys

                85                  90                  95

gcg aga gga gca cat tat ggt ttc gat atc tgg ggc caa ggg aca atg    336

Ala Arg Gly Ala His Tyr Gly Phe Asp Ile Trp Gly Gln Gly Thr Met

            100                 105                 110

gtc acc gtc tcg agt                                                351

Val Thr Val Ser Ser

        115

<210> 56

<211> 57

<212> DNA

<213> Human

<220>

<221> CDS

<222> (1)...(57)

<223> reader sequence

<400> 56

atg gag ttt ggg ctg agc tgg gtt ttc ctc gtt gct ctt tta aga ggt   48

Met Glu Phe Gly Leu Ser Trp Val Phe Leu Val Ala Leu Leu Arg Gly

                    5                   10                  15

gtc cag tgt                                                       57

Val Gln Cys

<210> 57

<211> 115

<212> DNA

<213> Artificial Sequence

<220>

<223> 12B5VH-1

<400> 57

atggagtttg ggctgagctg ggttttcctc gttgctcttt taagaggtgt ccagtgtcag   60

gtgcagctgg tgcagtctgg gggaggcttg gtccggcccg gggggtccct gagtc       115

<210> 58

<211> 115

<212> DNA

<213> Artificial Sequence

<220>

<223> 12B5VH-2

<400> 58

aaggatatac ctgccaccca ctccagcccc ttgcctggag cctggcggac ccagtgcatg 60

ccgtaggtcc tgagggtgat tccagagact gcacaggaga gactcaggga ccccc 115

<210> 59

<211> 115

<212> DNA

<213> Artificial Sequence

<220>

<223> 12B5VH-3

<400> 59

ggcaggtata tcctttgacg gaagaagtga atactatgca gactccgtgc agggccgatt 60

caccatctcc agagacagtt ccaagaacac cctgtatctg caaatgaaca gcctg 115

<210> 60

<211> 108

<212> DNA

<213> Artificial Sequence

<220>

<223> 12B5VH-4

<400> 60

actcgagacg gtgaccattg tcccttggcc ccagatatcg aaaccataat gtgctcctct 60

cgcacagtaa tacacagccg tgtcctcggc tctcaggctg ttcatttg 108

<210> 61

<211> 32

<212> DNA

<213> Artificial Sequence

<220>

<223> 12B5VH-S, PCR primer

<400> 61

ttcaagcttc caccatggag tttgggctga gc   32

<210> 62

<211> 34

<212> DNA

<213> Artificial Sequence

<220>

<223> 12B5VH-A, PCR primer

<400> 62

ttgggatcca ctcaccactc gagacggtga ccat   34

<210> 63

<211> 588

<212> DNA

<213> Human

<220>

<221> CDS

<222> (236)...(558)

<223> 1-235;intron, 236-558;Human IgG constant region (partial)

<400> 63

gaattcgtga gtggatccca agctagcttt ctggggcagg ccaggcctga ccttggcttt   60

ggggcaggga gggggctaag gtgaggcagg tggcgccagc caggtgcaca cccaatgccc   120

atgagcccag acactggacg ctgaacctcg cggacagtta agaacccagg ggcctctgcg 180
ccctgggccc agctctgtcc cacaccgcgg tcacatggca caacctctct tgca gcc 237
Ala
1
tcc acc aag ggc cca tcg gtc ttc ccc ctg gca ccc tcc tcc aag agc 285
Ser Thr Lys Gly Pro Ser Val Phe Pro Leu Ala Pro Ser Ser Lys Ser
            5                   10                  15
acc tct ggg ggc aca gcg gcc ctg ggc tgc ctg gtc aag gac tac ttc 333
Thr Ser Gly Gly Thr Ala Ala Leu Gly Cys Leu Val Lys Asp Tyr Phe
        20                  25                  30
ccc gaa ccg gtg acg gtg tcg tgg aac tca ggc gcc ctg acc agc ggc 381
Pro Glu Pro Val Thr Val Ser Trp Asn Ser Gly Ala Leu Thr Ser Gly
    35                  40                  45
gtg cac acc ttc ccg gct gtc cta cag tcc tca gga ctc tac tcc ctc 429
Val His Thr Phe Pro Ala Val Leu Gln Ser Ser Gly Leu Tyr Ser Leu
50                  55                  60                  65
agc agc gtg gtg acc gtg ccc tcc agc agc ttg ggc acc cag acc tac 477
Ser Ser Val Val Thr Val Pro Ser Ser Ser Leu Gly Thr Gln Thr Tyr
                70                  75                  80
atc tgc aac gtg aat cac aag ccc agc aac acc aag gtg gac aag aaa 525
Ile Cys Asn Val Asn His Lys Pro Ser Asn Thr Lys Val Asp Lys Lys
            85                  90                  95
gtt gag ccc aaa tct tgt gac aaa act cac aca 558
Val Glu Pro Lys Ser Cys Asp Lys Thr His Thr
        100                 105

<210> 64
<211> 27

<212> DNA

<213> Artificial Sequence

<220>

<223> G1CH1-S, PCR primer

<400> 64

tgagaattcg tgagtggatc ccaagct   27

<210> 65

<211> 60

<212> DNA

<213> Artificial Sequence

<220>

<223> G1CH1-A, PCR primer

<400> 65

aaaagatctt tatcatgtgt gagttttgtc acaagatttg ggctcaactt tcttgtccac   60

<210> 66

<211> 432

<212> DNA

<213> Human

<220>

<221> CDS

<222> (12)...(419)

<223> HEF-12B5H-g gamma. 12-419 peptide

<400> 66

aagcttccac c atg gag ttt ggg ctg agc tgg gtt ttc ctc gtt gct ctt   50

Met Glu Phe Gly Leu Ser Trp Val Phe Leu Val Ala Leu

1               5                   10

tta aga ggt gtc cag tgt cag gtg cag ctg gtg cag tct ggg gga ggc    98
Leu Arg Gly Val Gln Cys Gln Val Gln Leu Val Gln Ser Gly Gly Gly
    15                  20                  25
ttg gtc cgg ccc ggg ggg tcc ctg agt ctc tcc tgt gca gtc tct gga    146
Leu Val Arg Pro Gly Gly Ser Leu Ser Leu Ser Cys Ala Val Ser Gly
30                  35                  40                  45
atc acc ctc agg acc tac ggc atg cac tgg gtc cgc cag gct cca ggc    194
Ile Thr Leu Arg Thr Tyr Gly Met His Trp Val Arg Gln Ala Pro Gly
                    50                  55                  60
aag ggg ctg gag tgg gtg gca ggt ata tcc ttt gac gga aga agt gaa    242
Lys Gly Leu Glu Trp Val Ala Gly Ile Ser Phe Asp Gly Arg Ser Glu
                65                  70                  75
tac tat gca gac tcc gtg cag ggc cga ttc acc atc tcc aga gac agt    290
Tyr Tyr Ala Asp Ser Val Gln Gly Arg Phe Thr Ile Ser Arg Asp Ser
            80                  85                  90
tcc aag aac acc ctg tat ctg caa atg aac agc ctg aga gcc gag gac    338
Ser Lys Asn Thr Leu Tyr Leu Gln Met Asn Ser Leu Arg Ala Glu Asp
    95                  100                 105
acg gct gtg tat tac tgt gcg aga gga gca cat tat ggt ttc gat atc    386
Thr Ala Val Tyr Tyr Cys Ala Arg Gly Ala His Tyr Gly Phe Asp Ile
110                 115                 120                 125
tgg ggc caa ggg aca atg gtc acc gtc tcg agt ggtgagtgga tcc         432
Trp Gly Gln Gly Thr Met Val Thr Val Ser Ser
                130                 135

<210> 67
<211> 321
<212> DNA

<213> Human

<220>

<221> CDS

<222> (1)...(321)

<223> 12B5LV. 1-321 peptide

<400> 67

gac atc cag atg acc cag tct cct tcc acc ctg tct gca tct att gga 48

Asp Ile Gln Met Thr Gln Ser Pro Ser Thr Leu Ser Ala Ser Ile Gly

1               5                   10                  15

gac aga gtc acc atc acc tgc cgg gcc agc gag ggt att tat cac tgg 96

Asp Arg Val Thr Ile Thr Cys Arg Ala Ser Glu Gly Ile Tyr His Trp

            20                  25                  30

ttg gcc tgg tat cag cag aag cca ggg aaa gcc cct aaa ctc ctg atc 144

Leu Ala Trp Tyr Gln Gln Lys Pro Gly Lys Ala Pro Lys Leu Leu Ile

        35                  40                  45

tat aag gcc tct agt tta gcc agt ggg gcc cca tca agg ttc agc ggc 192

Tyr Lys Ala Ser Ser Leu Ala Ser Gly Ala Pro Ser Arg Phe Ser Gly

    50                  55                  60

agt gga tct ggg aca gat ttc act ctc acc atc agc agc ctg cag cct 240

Ser Gly Ser Gly Thr Asp Phe Thr Leu Thr Ile Ser Ser Leu Gln Pro

65                  70                  75                  80

gat gat ttt gca act tat tac tgc caa caa tat agt aat tat ccg ctc 288

Asp Asp Phe Ala Thr Tyr Tyr Cys Gln Gln Tyr Ser Asn Tyr Pro Leu

                85                  90                  95

act ttc ggc gga ggg acc aag ctg gag atc aaa 321

Thr Phe Gly Gly Gly Thr Lys Leu Glu Ile Lys

            100                 105

<210> 68

<211> 66

<212> DNA

<213> Human

<220>

<221> CDS

<222> (1)...(66)

<223> reader sequence

<400> 68

atg gac atg agg gtc ccc gct cag ctc ctg ggg ctc ctg ctg ctc tgg   48

MET Asp MET Arg Val Pro Ala Gln Leu Leu Gly Leu Leu Leu Leu Trp

                  5                   10                  15

ctc cca ggt gcc aaa tgt                                           66

Leu Pro Gly Ala Lys Cys

         20

<210> 69

<211> 110

<212> DNA

<213> Artificial Sequence

<220>

<223> 12B5VL-1

<400> 69

atggacatga gggtccccgc tcagctcctg gggctcctgc tgctctggct cccaggtgcc   60

aaatgtgaca tccagatgac ccagtctcct tccaccctgt ctgcatctat              110

<210> 70

<211> 110

<212> DNA

<213> Artificial Sequence

<220>

<223> 12B5VL-2

<400> 70

ggagtttagg ggctttccct ggcttctgct gataccaggc caaccagtga taaataccct 60

cgctggcccg gcaggtgatg gtgactctgt ctccaataga tgcagacagg 110

<210> 71

<211> 110

<212> DNA

<213> Artificial Sequence

<220>

<223> 12B5VL-3

<400> 71

aagcccctaa actcctgatc tataaggcct ctagtttagc cagtggggcc ccatcaaggt 60

tcagcggcag tggatctggg acagatttca ctctcaccat cagcagcctg 110

<210> 72

<211> 103

<212> DNA

<213> Artificial Sequence

<220>

<223> 12B5VL-4

<400> 72

tttgatctcc agcttggtcc ctccgccgaa agtgagcgga taattactat attgttggca 60

gtaataagtt gcaaaatcat caggctgcag gctgctgatg gtg 103

<210> 73

<211> 32

<212> DNA

<213> Artificial Sequence

<220>

<223> 12B5VL-S, PCR primer

<400> 73

ttcaagcttc caccatggac atgagggtcc cc   32

<210> 74

<211> 35

<212> DNA

<213> Artificial Sequence

<220>

<223> 12B5VL-A, PCR primer

<400> 74

tctaggatcc actcacgttt gatctccagc ttggt   35

<210> 75

<211> 415

<212> DNA

<213> Human

<220>

<221> CDS

<222> (12)...(398)

<223> HEF-12B5H-g kappa. 12-398 peptide

<400> 75

aagcttccac c atg gac atg agg gtc ccc gct cag ctc ctg ggg ctc ctg   50

Met Asp Met Arg Val Pro Ala Gln Leu Leu Gly Leu Leu
1               5                   10
ctg ctc tgg ctc cca ggt gcc aaa tgt gac atc cag atg acc cag tct    98
Leu Leu Trp Leu Pro Gly Ala Lys Cys Asp Ile Gln Met Thr Gln Ser
    15                  20                  25
cct tcc acc ctg tct gca tct att gga gac aga gtc acc atc acc tgc    146
Pro Ser Thr Leu Ser Ala Ser Ile Gly Asp Arg Val Thr Ile Thr Cys
30                  35                  40                  45
cgg gcc agc gag ggt att tat cac tgg ttg gcc tgg tat cag cag aag    194
Arg Ala Ser Glu Gly Ile Tyr His Trp Leu Ala Trp Tyr Gln Gln Lys
                    50                  55                  60
cca ggg aaa gcc cct aaa ctc ctg atc tat aag gcc tct agt tta gcc    242
Pro Gly Lys Ala Pro Lys Leu Leu Ile Tyr Lys Ala Ser Ser Leu Ala
                65                  70                  75
agt ggg gcc cca tca agg ttc agc ggc agt gga tct ggg aca gat ttc    290
Ser Gly Ala Pro Ser Arg Phe Ser Gly Ser Gly Ser Gly Thr Asp Phe
            80                  85                  90
act ctc acc atc agc agc ctg cag cct gat gat ttt gca act tat tac    338
Thr Leu Thr Ile Ser Ser Leu Gln Pro Asp Asp Phe Ala Thr Tyr Tyr
    95                  100                 105
tgc caa caa tat agt aat tat ccg ctc act ttc ggc gga ggg acc aag    386
Cys Gln Gln Tyr Ser Asn Tyr Pro Leu Thr Phe Gly Gly Gly Thr Lys
110                 115                 120                 125
ctg gag atc aaa cgtgagtgga tcctaga                                 415
Leu Glu Ile Lys

<210> 76
<211> 24

<212> DNA

<213> Artificial Sequence

<220>

<223> FLAG tag sequence

<400> 76

gac tac aag gat gac gac gat aag  24

Asp Tyr Lys Asp Asp Asp Asp Lys

                5

<210> 77

<211> 31

<212> DNA

<213> Artificial Sequence

<220>

<223> 12B5-S, PCR primer

<400> 77

atagaattcc accatggagt ttgggctgag c  31

<210> 78

<211> 24

<212> DNA

<213> Artificial Sequence

<220>

<223> HuVHJ3, PCR primer

<400> 78

tgaagagacg gtgaccattg tccc  24

<210> 79

<211> 28

<212> DNA

<213> Artificial Sequence

<220>

<223> RhuJH3, PCR primer

<400> 79

ggacaatggt caccgtctct tcaggtgg 28

<210> 80

<211> 32

<212> DNA

<213> Artificial Sequence

<220>

<223> RhuVK1, PCR primer

<400> 80

ggagactggg tcatctggat gtccgatccg cc 32

<210> 81

<211> 23

<212> DNA

<213> Artificial Sequence

<220>

<223> HuVK1.2, PCR primer

<400> 81

gacatccaga tgacccagtc tcc 23

<210> 82

<211> 59

<212> DNA
<213> Artificial Sequence
<220>
<223> 12B5F-A, PCR primer
<400> 82
attgcggccg cttatcactt atcgtcgtca tccttgtagt ctttgatctc cagcttggt 59

<210> 83
<211> 45
<212> DNA
<213> Artificial Sequence
<220>
<223> Linker amino acid sequence and nucleotide sequence
<400> 83
ggt ggt ggt ggt tcg ggt ggt ggt ggt tcg ggt ggt ggc gga tcg 45
Gly Gly Gly Gly Ser Gly Gly Gly Gly Ser Gly Gly Gly Gly Ser
                5                   10                  15

<210> 84
<211> 823
<212> DNA
<213> Human
<220>
<221> CDS
<222> (12)...(809)
<223> sc12B5, Single chain Fv
<400> 84
aagcttccac c atg gag ttt ggg ctg agc tgg gtt ttc ctc gtt gct ctt 50

Met Glu Phe Gly Leu Ser Trp Val Phe Leu Val Ala Leu
1               5                   10
tta aga ggt gtc cag tgt cag gtg cag ctg gtg cag tct ggg gga ggc 98
Leu Arg Gly Val Gln Cys Gln Val Gln Leu Val Gln Ser Gly Gly Gly
    15                  20                  25
ttg gtc cgg ccc ggg ggg tcc ctg agt ctc tcc tgt gca gtc tct gga 146
Leu Val Arg Pro Gly Gly Ser Leu Ser Leu Ser Cys Ala Val Ser Gly
30                  35                  40                  45
atc acc ctc agg acc tac ggc atg cac tgg gtc cgc cag gct cca ggc 194
Ile Thr Leu Arg Thr Tyr Gly Met His Trp Val Arg Gln Ala Pro Gly
                50                  55                  60
aag ggg ctg gag tgg gtg gca ggt ata tcc ttt gac gga aga agt gaa 242
Lys Gly Leu Glu Trp Val Ala Gly Ile Ser Phe Asp Gly Arg Ser Glu
            65                  70                  75
tac tat gca gac tcc gtg cag ggc cga ttc acc atc tcc aga gac agt 290
Tyr Tyr Ala Asp Ser Val Gln Gly Arg Phe Thr Ile Ser Arg Asp Ser
        80                  85                  90
tcc aag aac acc ctg tat ctg caa atg aac agc ctg aga gcc gag gac 338
Ser Lys Asn Thr Leu Tyr Leu Gln Met Asn Ser Leu Arg Ala Glu Asp
    95                  100                 105
acg gct gtg tat tac tgt gcg aga gga gca cat tat ggt ttc gat atc 386
Thr Ala Val Tyr Tyr Cys Ala Arg Gly Ala His Tyr Gly Phe Asp Ile
110                 115                 120                 125
tgg ggc caa ggg aca atg gtc acc gtc tcg agt ggt ggt ggt ggt tcg 434
Trp Gly Gln Gly Thr Met Val Thr Val Ser Ser Gly Gly Gly Gly Ser
                130                 135                 140
ggt ggt ggt ggt tcg ggt ggt ggc gga tcg gac atc cag atg acc cag 482
Gly Gly Gly Gly Ser Gly Gly Gly Gly Ser Asp Ile Gln Met Thr Gln

            145                 150                 155

tct cct tcc acc ctg tct gca tct att gga gac aga gtc acc atc acc 530

Ser Pro Ser Thr Leu Ser Ala Ser Ile Gly Asp Arg Val Thr Ile Thr

        160                 165                 170

tgc cgg gcc agc gag ggt att tat cac tgg ttg gcc tgg tat cag cag 578

Cys Arg Ala Ser Glu Gly Ile Tyr His Trp Leu Ala Trp Tyr Gln Gln

    175                 180                 185

aag cca ggg aaa gcc cct aaa ctc ctg atc tat aag gcc tct agt tta 626

Lys Pro Gly Lys Ala Pro Lys Leu Leu Ile Tyr Lys Ala Ser Ser Leu

190                 195                 200                 205

gcc agt ggg gcc cca tca agg ttc agc ggc agt gga tct ggg aca gat 674

Ala Ser Gly Ala Pro Ser Arg Phe Ser Gly Ser Gly Ser Gly Thr Asp

                210                 215                 220

ttc act ctc acc atc agc agc ctg cag cct gat gat ttt gca act tat 722

Phe Thr Leu Thr Ile Ser Ser Leu Gln Pro Asp Asp Phe Ala Thr Tyr

            225                 230                 235

TAC TGC CAA CAA TAT AGT AAT TAT CCG CTC ACT TTC GGC GGA GGG ACC 770

Tyr Cys Gln Gln Tyr Ser Asn Tyr Pro Leu Thr Phe Gly Gly Gly Thr

        240                 245                 250

aag ctg gag atc aaa gac tac aag gat gac gac gat aag tgataagcgg c 820

Lys Leu Glu Ile Lys Asp Tyr Lys Asp Asp Asp Asp Lys

    255                 260                 265

cgc 823

<210> 85

<211> 114

<212> PRT

<213> Human

<400> 85

Gln Val Gln Leu Gln Gln Ser Gly Pro Gly Leu Val Lys Pro Ser Glu

1               5                   10                  15

Thr Leu Ser Leu Thr Cys Thr Val Ser Gly Asp Ser Ile Ser Ser Tyr

            20                  25                  30

Tyr Trp Ser Trp Ile Arg Gln Pro Pro Gly Lys Gly Leu Glu Trp Ile

        35                  40                  45

Gly Tyr Ile Tyr Tyr Ser Gly Ser Thr Asn Tyr Asn Pro Ser Leu Lys

    50                  55                  60

Ser Arg Val Thr Ile Ser Val Asp Thr Ser Lys Ser Gln Phe Ser Leu

65                  70                  75                  80

Lys Leu Ser Ser Val Thr Ala Ala Asp Thr Ala Val Tyr Tyr Cys Ala

                85                  90                  95

Arg Gly Arg Tyr Phe Asp Val Trp Gly Arg Gly Thr Met Val Thr Val

            100                 105                 110

Ser Ser

<210> 86

<211> 342

<212> DNA

<213> Human

<400> 86

caggtgcagc tgcagcagtc gggcccagga ctggtgaagc cttcggagac cctgtccctc    60

acctgcactg tctctggtga ctccatcagt agttactact ggagctggat tcggcagccc   120

ccagggaagg gactggagtg gattgggtat atctattaca gtgggagcac caactacaac   180

ccctccctca agagtcgagt caccatatca gtagacacgt ccaagagcca gttctccctg   240

aagctgagct ctgtgaccgc cgcagacacg gccgtgtatt actgtgcgag agggcggtac 300

ttcgatgtct ggggccgtgg caccatggtc actgtctcct ca 342

<210> 87

<211> 57

<212> DNA

<213> Human

<220>

<221> CDS

<222> (1)...(57)

<223> reader sequence

<308> GenBank No. AF062252

<400> 87

atg aaa cat ctg tgg ttc ttc ctt ctc ctg gtg gca gct ccc aga tgg 48

Met Lys His Leu Trp Phe Phe Leu Leu Leu Val Ala Ala Pro Arg Trp

1               5                   10                  15

gtc ctg tcc 57

Val Leu Ser

<210> 88

<211> 110

<212> DNA

<213> Artificial Sequence

<220>

<223> 12E10VH1

<400> 88

atgaaacatc tgtggttctt ccttctcctg gtggcagctc ccagatgggt cctgtcccag 60

gtgcagctgc agcagtcggg cccaggactg gtgaagcctt cggagaccct 110

<210> 89

<211> 110

<212> DNA

<213> Artificial Sequence

<220>

<223> 12E10VH2

<400> 89

acccaatcca ctccagtccc ttccctgggg gctgccgaat ccagctccag tagtaactac    60

tgatggagtc accagagaca gtgcaggtga gggacagggt ctccgaaggc              110

<210> 90

<211> 110

<212> DNA

<213> Artificial Sequence

<220>

<223> 12E10VH3

<400> 90

tggagtggat tgggtatatc tattacagtg ggagcaccaa ctacaacccc tccctcaaga    60

gtcgagtcac catatcagta gacacgtcca agagccagtt ctccctgaag              110

<210> 91

<211> 114

<212> DNA

<213> Artificial Sequence

<220>

<223> 12E10VH4

<400> 91

tgaggagaca gtgaccatgg tgccacggcc ccagacatcg aagtaccgcc ctctcgcaca 60
gtaatacacg gccgtgtctg cggcggtcac agagctcagc ttcagggaga actg 114

<210> 92
<211> 32
<212> DNA
<213> Artificial Sequence
<220>
<223> 12E10VHS, PCR primer
<400> 92
ttcaagcttc caccatgaaa catctgtggt tc 32

<210> 93
<211> 34
<212> DNA
<213> Artificial Sequence
<220>
<223> 12E10VHA, PCR primer
<400> 93
ttgggatcca ctcacctgag gagacagtga ccat 34

<210> 94
<211> 426
<212> DNA
<213> Mus
<220>
<221> CDS
<222> (12)...(417)

<223> 12E10H, H chain V region

<400> 94

aagcttccac c atg aaa cat ctg tgg ttc ttc ctt ctc ctg gtg gca gct   50

Met Lys His Leu Trp Phe Phe Leu Leu Leu Val Ala Ala

1               5                   10

ccc aga tgg gtc ctg tcc cag gtg cag ctg cag cag tcg ggc cca gga   98

Pro Arg Trp Val Leu Ser Gln Val Gln Leu Gln Gln Ser Gly Pro Gly

15                  20                  25

ctg gtg aag cct tcg gag acc ctg tcc ctc acc tgc act gtc tct ggt   146

Leu Val Lys Pro Ser Glu Thr Leu Ser Leu Thr Cys Thr Val Ser Gly

30                  35                  40                  45

gac tcc atc agt agt tac tac tgg agc tgg att cgg cag ccc cca ggg   194

Asp Ser Ile Ser Ser Tyr Tyr Trp Ser Trp Ile Arg Gln Pro Pro Gly

                50                  55                  60

aag gga ctg gag tgg att ggg tat atc tat tac agt ggg agc acc aac   242

Lys Gly Leu Glu Trp Ile Gly Tyr Ile Tyr Tyr Ser Gly Ser Thr Asn

            65                  70                  75

tac aac ccc tcc ctc aag agt cga gtc acc ata tca gta gac acg tcc   290

Tyr Asn Pro Ser Leu Lys Ser Arg Val Thr Ile Ser Val Asp Thr Ser

        80                  85                  90

aag agc cag ttc tcc ctg aag ctg agc tct gtg acc gcc gca gac acg   338

Lys Ser Gln Phe Ser Leu Lys Leu Ser Ser Val Thr Ala Ala Asp Thr

    95                  100                 105

gcc gtg tat tac tgt gcg aga ggg cgg tac ttc gat gtc tgg ggc cgt   386

Ala Val Tyr Tyr Cys Ala Arg Gly Arg Tyr Phe Asp Val Trp Gly Arg

110                 115                 120                 125

ggc acc atg gtc act gtc tcc tca ggtgagtgga tcccaa   426

Gly Thr Met Val Thr Val Ser Ser

                130

<210> 95

<211> 110

<212> PRT

<213> Mus

<400> 95

Ser Tyr Val Leu Thr Gln Pro Pro Ser Val Ser Gly Ser Pro Gly Gln

1               5                   10                  15

Ser Ile Thr Ile Ser Cys Thr Gly Thr Ser Ser Asp Val Gly Gly Tyr

            20                  25                  30

Asn Tyr Val Ser Trp Tyr Gln Gln His Pro Gly Lys Ala Pro Lys Leu

        35                  40                  45

Met Ile Tyr Glu Gly Ser Lys Arg Pro Ser Gly Val Ser Asn Arg Phe

    50                  55                  60

Ser Gly Ser Lys Ser Gly Asn Thr Ala Ser Leu Thr Ile Ser Gly Leu

65                  70                  75                  80

Gln Ala Glu Asp Glu Ala Asp Tyr Tyr Cys Ser Ser Tyr Thr Thr Arg

                85                  90                  95

Ser Thr Arg Val Phe Gly Gly Gly Thr Lys Leu Thr Val Leu

            100                 105                 110

<210> 96

<211> 330

<212> DNA

<213> Mus

<400> 96

tcctatgtgc tgactcagcc accctcggtg tcagggtctc ctggacagtc gatcaccatc    60

tcctgcactg gaaccagcag tgacgttggt ggttataact atgtctcctg gtaccaacag   120

cacccaggca aagcccccaa actcatgatt tatgagggca gtaaacggcc ctcaggggtt   180

tctaatcgct tctctggctc caagtctggc aacacggcct ccctgaccat ctctgggctc   240

caggctgagg acgaggctga ttattactgc agctcatata caaccagaag cactcgggtg   300

ttcggcggag ggaccaagct gaccgtccta                                    330

<210> 97

<211> 57

<212> DNA

<213> Human

<220>

<221> CDS

<222> (1)...(57)

<223> reader sequence

<310>

<400> 97

atg gcc tgg acc gtt ctc ctc ctc ggc ctc ctc tct cac tgc aca ggc   48

Met Ala Trp Thr Val Leu Leu Leu Gly Leu Leu Ser His Cys Thr Gly

  1               5                  10                  15

tct gtg acc                                                       57

Ser Val Thr

<210> 98

<211> 110

<212> DNA

<213> Artificial Sequence

<220>

<223> 12E10VL1, PCR primer

<400> 98

atggcctgga ccgttctcct cctcggcctc ctctctcact gcacaggctc tgtgacctcc 60

tatgtgctga ctcagccacc ctcggtgtca gggtctcctg gacagtcgat 110

<210> 99

<211> 62

<212> DNA

<213> Artificial Sequence

<220>

<223> 12E10VL2, PCR primer

<400> 99

tcatgagttt gggggctttg cctgggtgct gttggtacca ggagacatag ttataaccac 60

caacgtcact gctggttcca gtgcaggaga tggtgatcga ctgtccagga 110

<210> 100

<211> 110

<212> DNA

<213> Artificial Sequence

<220>

<223> 12E10VL3, PCR primer

<400> 100

cccccaaact catgatttat gagggcagta aacggccctc aggggtttct aatcgcttct 60

ctggctccaa gtctggcaac acggcctccc tgaccatctc tgggctccag 110

<210> 101

<211> 102

<212> DNA

<213> Artificial Sequence

<220>

<223> 12E10VL4, PCR primer

<400> 101

taggacggtc agcttggtcc ctccgccgaa cacccgagtg cttctggttg tatatgagct 60

gcagtaataa tcagcctcgt cctcagcctg gagcccagag at 102

<210> 102

<211> 31

<212> DNA

<213> Artificial Sequence

<220>

<223> 12E10VLS, PCR primer

<400> 102

atcaagcttc caccatggcc tggaccgttc t 31

<210> 103

<211> 36

<212> DNA

<213> Artificial Sequence

<220>

<223> 12E10VLA, PCR primer

<400> 103

ctaggatccg ggctgaccta ggacggtcag cttggt 36

<210> 104

<211> 387

<212> DNA

<213> Mus

<220>

<221> CDS

<222> (1)...(387)

<223> 12E10L, L chain V region

<310>

<400> 104

atg gcc tgg acc gtt ctc ctc ctc ggc ctc ctc tct cac tgc aca ggc 48

Met Ala Trp Thr Val Leu Leu Leu Gly Leu Leu Ser His Cys Thr Gly

1               5                   10                  15

tct gtg acc tcc tat gtg ctg act cag cca ccc tcg gtg tca ggg tct 96

Ser Val Thr Ser Tyr Val Leu Thr Gln Pro Pro Ser Val Ser Gly Ser

            20                  25                  30

cct gga cag tcg atc acc atc tcc tgc act gga acc agc agt gac gtt 144

Pro Gly Gln Ser Ile Thr Ile Ser Cys Thr Gly Thr Ser Ser Asp Val

        35                  40                  45

ggt ggt tat aac tat gtc tcc tgg tac caa cag cac cca ggc aaa gcc 192

Gly Gly Tyr Asn Tyr Val Ser Trp Tyr Gln Gln His Pro Gly Lys Ala

    50                  55                  60

ccc aaa ctc atg att tat gag ggc agt aaa cgg ccc tca ggg gtt tct 240

Pro Lys Leu Met Ile Tyr Glu Gly Ser Lys Arg Pro Ser Gly Val Ser

65                  70                  75                  80

aat cgc ttc tct ggc tcc aag tct ggc aac acg gcc tcc ctg acc atc 288

Asn Arg Phe Ser Gly Ser Lys Ser Gly Asn Thr Ala Ser Leu Thr Ile

                85                  90                  95

tct ggg ctc cag gct gag gac gag gct gat tat tac tgc agc tca tat 336

Ser Gly Leu Gln Ala Glu Asp Glu Ala Asp Tyr Tyr Cys Ser Ser Tyr

100                    105                    110

Aca acc aga agc act cgg gtg ttc ggc gga ggg acc aag ctg acc gtc  384

Thr Thr Arg Ser Thr Arg Val Phe Gly Gly Gly Thr Lys Leu Thr Val

        115                    120                    125

cta                                                              387

Leu

<210> 105

<211> 24

<212> DNA

<213> Artificial Sequence

<220>

<221> CDS

<222> (1)...(24)

<223> FLAG, reader sequence

<400> 105

gac tac aag gat gac gac gat aag      24

Asp Tyr Lys Asp Asp Asp Asp Lys

<210> 106

<211> 30

<212> DNA

<213> Artificial Sequence

<220>

<223> 12E10S, PCR primer

<400> 106

tatgaattcc accatgaaac atctgtggtt  30

<210> 107

<211> 38

<212> DNA

<213> Artificial Sequence

<220>

<223> DB2, PCR primer

<400> 107

taggagctac cgcctccacc tgaggagaca gtgaccat 38

<210> 108

<211> 44

<212> DNA

<213> Artificial Sequence

<220>

<223> DB1, PCR primer

<400> 108

gtctcctcag gtggaggcgg tagctcctat gtgctgactc agcc 44

<210> 109

<211> 59

<212> DNA

<213> Artificial Sequence

<220>

<223> 12E10FA, PCR primer

<400> 109

attgcggccg cttatcactt atcgtcgtca tccttgtagt ctaggacggt cagcttggt 59

<210> 110

<211> 792
<212> DNA
<213> Artificial Sequence
<220>
<221> CDS
<222> (11)...(778)
<223> 12E10, Single chain Fv
<400> 110
gaattccacc atg aaa cat ctg tgg ttc ttc ctt ctc ctg gtg gca gct 49
Met Lys His Leu Trp Phe Phe Leu Leu Leu Val Ala Ala
1               5                   10
ccc aga tgg gtc ctg tcc cag gtg cag ctg cag cag tcg ggc cca gga 97
Pro Arg Trp Val Leu Ser Gln Val Gln Leu Gln Gln Ser Gly Pro Gly
    15                  20                  25
ctg gtg aag cct tcg gag acc ctg tcc ctc acc tgc act gtc tct ggt 145
Leu Val Lys Pro Ser Glu Thr Leu Ser Leu Thr Cys Thr Val Ser Gly
30                  35                  40                  45
gac tcc atc agt agt tac tac tgg agc tgg att cgg cag ccc cca ggg 193
Asp Ser Ile Ser Ser Tyr Tyr Trp Ser Trp Ile Arg Gln Pro Pro Gly
                50                  55                  60
aag gga ctg gag tgg att ggg tat atc tat tac agt ggg agc acc aac 241
Lys Gly Leu Glu Trp Ile Gly Tyr Ile Tyr Tyr Ser Gly Ser Thr Asn
            65                  70                  75
tac aac ccc tcc ctc aag agt cga gtc acc ata tca gta gac acg tcc 289
Tyr Asn Pro Ser Leu Lys Ser Arg Val Thr Ile Ser Val Asp Thr Ser
        80                  85                  90
aag agc cag ttc tcc ctg aag ctg agc tct gtg acc gcc gca gac acg 337
Lys Ser Gln Phe Ser Leu Lys Leu Ser Ser Val Thr Ala Ala Asp Thr

95                  100                 105

gcc gtg tat tac tgt gcg aga ggg cgg tac ttc gat gtc tgg ggc cgt   385

Ala Val Tyr Tyr Cys Ala Arg Gly Arg Tyr Phe Asp Val Trp Gly Arg

110                 115                 120                 125

ggc acc atg gtc act gtc tcc tca ggt gga ggc ggt agc tcc tat gtg   433

Gly Thr Met Val Thr Val Ser Ser Gly Gly Gly Gly Ser Ser Tyr Val

                130                 135                 140

ctg act cag cca ccc tcg gtg tca ggg tct cct gga cag tcg atc acc   481

Leu Thr Gln Pro Pro Ser Val Ser Gly Ser Pro Gly Gln Ser Ile Thr

            145                 150                 155

atc tcc tgc act gga acc agc agt gac gtt ggt ggt tat aac tat gtc   529

Ile Ser Cys Thr Gly Thr Ser Ser Asp Val Gly Gly Tyr Asn Tyr Val

        160                 165                 170

tcc tgg tac caa cag cac cca ggc aaa gcc ccc aaa ctc atg att tat   577

Ser Trp Tyr Gln Gln His Pro Gly Lys Ala Pro Lys Leu Met Ile Tyr

    175                 180                 185

gag ggc agt aaa cgg ccc tca ggg gtt tct aat cgc ttc tct ggc tcc   625

Glu Gly Ser Lys Arg Pro Ser Gly Val Ser Asn Arg Phe Ser Gly Ser

190                 195                 200                 205

aag tct ggc aac acg gcc tcc ctg acc atc tct ggg ctc cag gct gag   673

Lys Ser Gly Asn Thr Ala Ser Leu Thr Ile Ser Gly Leu Gln Ala Glu

                210                 215                 220

gac gag gct gat tat tac tgc agc tca tat aca acc aga agc act cgg   721

Asp Glu Ala Asp Tyr Tyr Cys Ser Ser Tyr Thr Thr Arg Ser Thr Arg

            225                 230                 235

gtg ttc ggc gga ggg acc aag ctg acc gtc cta gac tac aag gat gac   769

Val Phe Gly Gly Gly Thr Lys Leu Thr Val Leu Asp Tyr Lys Asp Asp

        240                 245                 250

gac gat aag tgataagcgg ccgc 792
Asp Asp Lys
    255

<210> 111
<211> 62
<212> DNA
<213> Artificial Sequence
<220>
<223> sc4.3, PCR primer
<400> 111
ggtggctgag tcagcacata ggacgatccg ccaccacccg aaccaccacc acccgaacca 60
cc 62

<210> 112
<211> 61
<212> DNA
<213> Artificial Sequence
<220>
<223> sc1.3, PCR primer
<400> 112
gcaccatggt cactgtctcc tcaggtggtg gtggttcggg tggtggtggt tcgggtggtg 60
g 61

<210> 113
<211> 822
<212> DNA
<213> Artificial Sequence

<220>

<221> CDS

<222> (11)...(807)

<223> sc12E10, Single chain Fv

<400> 113

gaattccacc atg aaa cat ctg tgg ttc ttc ctt ctc ctg gtg gca gct 49

Met Lys His Leu Trp Phe Phe Leu Leu Leu Val Ala Ala

1               5                   10

ccc aga tgg gtc ctg tcc cag gtg cag ctg cag cag tcg ggc cca gga 97

Pro Arg Trp Val Leu Ser Gln Val Gln Leu Gln Gln Ser Gly Pro Gly

    15                  20                  25

ctg gtg aag cct tcg gag acc ctg tcc ctc acc tgc act gtc tct ggt 145

Leu Val Lys Pro Ser Glu Thr Leu Ser Leu Thr Cys Thr Val Ser Gly

30                  35                  40                  45

gac tcc atc agt agt tac tac tgg agc tgg att cgg cag ccc cca ggg 193

Asp Ser Ile Ser Ser Tyr Tyr Trp Ser Trp Ile Arg Gln Pro Pro Gly

                50                  55                  60

aag gga ctg gag tgg att ggg tat atc tat tac agt ggg agc acc aac 241

Lys Gly Leu Glu Trp Ile Gly Tyr Ile Tyr Tyr Ser Gly Ser Thr Asn

            65                  70                  75

tac aac ccc tcc ctc aag agt cga gtc acc ata tca gta gac acg tcc 289

Tyr Asn Pro Ser Leu Lys Ser Arg Val Thr Ile Ser Val Asp Thr Ser

        80                  85                  90

aag agc cag ttc tcc ctg aag ctg agc tct gtg acc gcc gca gac acg 337

Lys Ser Gln Phe Ser Leu Lys Leu Ser Ser Val Thr Ala Ala Asp Thr

    95                  100                 105

gcc gtg tat tac tgt gcg aga ggg cgg tac ttc gat gtc tgg ggc cgt 385

Ala Val Tyr Tyr Cys Ala Arg Gly Arg Tyr Phe Asp Val Trp Gly Arg

110                 115                 120                 125

ggc acc atg gtc act gtc tcc tca ggt ggt ggt ggt tcg ggt ggt ggt   433

Gly Thr Met Val Thr Val Ser Ser Gly Gly Gly Gly Ser Gly Gly Gly

                130                 135                 140

ggt tcg ggt ggt ggc gga tcg tcc tat gtg ctg act cag cca ccc tcg   481

Gly Ser Gly Gly Gly Gly Ser Ser Tyr Val Leu Thr Gln Pro Pro Ser

            145                 150                 155

gtg tca ggg tct cct gga cag tcg atc acc atc tcc tgc act gga acc   529

Val Ser Gly Ser Pro Gly Gln Ser Ile Thr Ile Ser Cys Thr Gly Thr

        160                 165                 170

agc agt gac gtt ggt ggt tat aac tat gtc tcc tgg tac caa cag cac   577

Ser Ser Asp Val Gly Gly Tyr Asn Tyr Val Ser Trp Tyr Gln Gln His

    175                 180                 185

cca ggc aaa gcc ccc aaa ctc atg att tat gag ggc agt aaa cgg ccc   625

Pro Gly Lys Ala Pro Lys Leu Met Ile Tyr Glu Gly Ser Lys Arg Pro

190                 195                 200                 205

tca ggg gtt tct aat cgc ttc tct ggc tcc aag tct ggc aac acg gcc   673

Ser Gly Val Ser Asn Arg Phe Ser Gly Ser Lys Ser Gly Asn Thr Ala

                210                 215                 220

tcc ctg acc atc tct ggg ctc cag gct gag gac gag gct gat tat tac   721

Ser Leu Thr Ile Ser Gly Leu Gln Ala Glu Asp Glu Ala Asp Tyr Tyr

            225                 230                 235

tgc agc tca tat aca acc aga agc act cgg gtg ttc ggc gga ggg acc   769

Cys Ser Ser Tyr Thr Thr Arg Ser Thr Arg Val Phe Gly Gly Gly Thr

        240                 245                 250

aag ctg acc gtc cta gac tac aag gat gac gac gat aag tgataagcgg   818

Lys Leu Thr Val Leu Asp Tyr Lys Asp Asp Asp Asp Lys

    255                 260                 265

ccgc 822

## Description

### TECHNICAL FIELD

This invention relates to modified antibodies containing two or more H chain V regions and two or more L chain V regions of a monoclonal antibody which show an agonist activity by crosslinking a cell surface molecule(s) or intracellular molecule(s). The modified antibodies have an agonist activity of transducing a signal into cells by crosslinking a cell surface molecule(s) and are useful as a medicine for various purposes.

### BACKGROUND ART

JP-A 9-295999 discloses the preparation of a specific monoclonal antibody using a splenic stromal cell line as a sensitizing antigen aiming at developing specific antibodies that can recognize the aforementioned splenic stromal cells and the preparation of novel monoclonal antibodies that recognize mouse Integrin Associated Protein (mouse LAP) as an antigen. JP-A. 9-295999 also discloses that the monoclonal antibodies are capable of inducing apoptosis of myeloid cells.

WO99/1297 discloses monoclonal antibodies whose antigen is human Integrin Associated Protein (hereinafter referred to as human IAP; amino acid sequence and nucleotide sequence thereof are described in J. Cell Biol., 123, 485-496, 1993; see also Journal of Cell Science, 108, 3419-3425, 1995) and which are capable of inducing apoptosis of human nucleated blood cells (myeloid cell and lymphocyte) having said human IAP. These monoclonal antibodies are referred to antibody MABL-1 and antibody MABL-2, and hybridomas producing these antibodies are also referred to MABL-1 (FERM BP-6100) and MABL-2 (FERM BP-6101), respectively.

Japanese Patent Application 11-63557 describes the preparation of single chain Fvs having single chain Fv regions from the monoclonal antibodies whose antigen is human IAP. The single chain Fvs are capable of inducing apoptosis of nucleated blood cells having human IAP.

The monoclonal antibody recognizing IAP as an antigen induces apoptosis of nucleated blood cells having human IAP, but it also causes hemagglutination in vitro. It indicates that the administration of a large amount of the monoclonal antibody recognizing IAP as an antigen may result in a side effect such as hemagglutination.

The inventors made intensive research for utilizing the monoclonal antibodies against human IAP as therapeutic agent of blood diseases and obtained single chain Fvs having the single chain Fv region capable of inducing apoptosis of nucleated blood cells having human IAP.

On the other hand modified antibodies, especially antibodies with lowered molecular size, for example, single chain Fvs were developed to improve permeability into tissues and tumors by lowering molecular size and to produce by a recombinant method. Recently the dimers of single chain Fvs, especially bispecific-dimers have been used for crosslinking cells. Typical examples of such dimers are hetero-dimers of single chain Fvs recognizing antigens of cancer cells and antigens of host cells like NK cells and neutrophils (Kipriyanov et al., Int. J. Cancer, 77, 9763-9772, 1998). They were produced by construction technique of single chain Fv as modified antibodies, which are more effective in treating cancers by inducing intercellular crosslinking. It has been thought that the intercellular crosslinking is induced by antibodies and their fragments (e.g. Fab fragment), bispecific modified antibodies and even dimers of single chain Fvs, which are monospecific.

As antibodies capable of transducing a signal by crosslinking a cell surface molecule(s), there are known an antibody against EPO receptor involved in cell differentiation and proliferation (JP-A 2000-95800), an antibody against MuSK receptor (Xie et al., Nature Biotech. 15, 768-771, 1997) and others. However there have been no reports on modified antibodies with lowered molecular size.

Noticing that single chain Fv monomers derived from antibody MABL-1 and antibody MABL-2 do not induce apoptosis of cells while single chain Fv dimers induce apoptosis of cells having IAP, the inventors discovered that they crosslink (dimerize) IAP receptor on cell surface, thereby a signal is transduced into the cells and, as a result, apoptosis is induced. This suggests that monospecific single chain Fv dimers crosslink a cell surface molecule(s) (e.g. receptor) and transduce a signal like a ligand, thereby serving as an agonist.

Focusing on the intercellular crosslinking, it was discovered that the above-mentioned single chain Fv dimers do not cause hemagglutination while the above-mentioned monoclonal antibodies do. The same result was also observed with single chain bivalent antibodies (single chain polypeptides containing two H chain V regions and two L chain V regions). This suggests that monoclonal antibodies may form intercellular crosslinking while modified antibodies like single chain Fv dimers and single chain bivalent antibodies crosslink a cell surface molecule(s) but do not form intercellular crosslinking.

Based on those observations the inventors have newly discovered that modified antibodies such as single chain Fv dimers and single chain bivalent antibodies crosslink a cell surface molecule(s) or intercellular molecule(s) of the same cell, in addition to known intercellular crosslinking, and are suitable as a ligand to the molecule(s) (especially as a ligand which mimics the action of natural ligand).

Discovering further that an antibody molecule (whole IgG) can be modified into single chain Fv dimers, single chain bivalent antibodies and the like which crosslink a cell surface molecule(s), thereby reducing side effects caused by intercellular crosslinking and providing new medicines inducing only desired effect on the cell, the inventors completed the invention. The modified antibodies of the invention have remarkably high activity compared with natural ligands such as TPO, EPO or G-CSF, or whole antibodies (IgG) having the same V region as the modified antibodies. They have an improved permeability into tissues due to the lowered molecular size compared with antibody molecules and the lack of constant regions.

### DISCLOSURE OF INVENTION

An object of this invention is to provide low molecular-sized agonist modified antibodies which contain two or more H chain V regions and two or more L chain V regions of monoclonal antibodies and have an agonist action by crosslinking a cell surface molecule(s) or intracellular molecule (s) .

Therefore, this invention relates the modified antibodies which contain two or more H chain V regions and two or more L chain V regions, preferably 2 to 6 each, especially preferably 2 to 4 each, most preferably two each, and show an agonist activity by crosslinking a cell surface molecule(s) or intracellular molecule(s).

The "modified antibodies" in the specification mean any substances which contain two or more H chain V regions and two or more L chain V regions, wherein said V regions are combined directly or via linker through covalent bond or non-covalent bond. For example, polypeptides and compounds produced by combining each V region of antibody through a peptide linker or a chemical crosslinking agent and the like. Two or more H chain V regions and two or more L chain V regions used in the invention can be derived from the same antibody or from different antibodies.

Preferable examples of modified antibodies of the invention are multimers such as dimers, trimers or tetramers of single chain Fv containing an H chain V region and an L chain V region, or single chain polypeptides containing two or more H chain V regions and two or more L chain V regions. When the modified antibodies of the invention are multimers of single chain Fv such as dimers, trimers, tetramers and the like containing an H chain V region and an L chain V region, it is preferable that the H chain V region and L chain V region existing in the same chain are not associated to form an antigen-binding site.

More preferable examples are dimers of the single chain Fv which contains an H chain V region and an L chain V region, or a single chain polypeptide containing two H chain V regions and two L chain V regions. The H chain V region and L chain V region are connected preferably through a linker in the modified antibodies.

"Agonist action" in the specification means a biological action occurring in the cell(s) into which a signal is transduced by crosslinking a cell surface molecule(s) or intracellular molecule(s), for example, apoptosis induction, cell proliferation induction, cell differentiation induction, cell division induction or cell cycle regulation action.

ED50 of the agonist action in the invention is determined by known methods for measuring agonist action. Examples are to detect agonist specific cell death or cell proliferation, to detect expression of proteins specific to cell differentiation (e.g. specific antigens) or to measure a kinase activity specific to cell cycle. ED50 is a dose needed for achieving 50% reaction of the maximum activity set as 100% in the dose-reaction curve.

Preferable modified antibodies of the invention have an agonist action (ED50) equivalent to or better than that of an antibody having the same antigen-binding region as the modified antibody, namely the whole antibody like IgG (hereinafter "parent antibody" ) having the same pair of H chain V region and L chain V region as the pair of H chain V region and L chain V region forming antigen-biding region of the modified antibody. More preferable are those having an agonist action (ED50) more than two times higher than that of parent antibody, further preferably more than 5 times, most preferably more than 10 times. The invention includes modified antibodies with an agonist action containing H chain V region and L chain V region forming the same antigen-binding region as parent antibody which binds to target cell surface molecule(s) or intracellular molecule(s) but has no agonist action to the molecule.

The compounds containing two or more H chain V regions and two or more L chain V regions of the invention can be any compounds which contain two or more H chain V regions and two or more L chain V regions of antibody and show an agonist action (ED50) equivalent to or better than that of a natural ligand binding to a cell surface molecule(s) or intracellular molecule(s). Preferable are those having an agonist action (ED50) more than two times higher than that of a natural ligand, more preferably more than 5 times, most preferably more than 10 times.

The "compounds" mentioned here include not only modified antibodies of the invention but also any compounds containing two or more, preferably from 2 to 6, more preferably from 2 to 4, most preferably 2 antigen-binding regions such as whole antibodies or F(ab')₂.

The modified antibodies or compounds of the invention containing two or more H chain V regions and two or more L chain V regions of antibody have preferably no substantial intercellular adhesion action. When the H chain V region and L chain V region of the modified antibodies of the invention are derived from the same antibody, those are preferable with an intercellular adhesion action (ED50) not more than 1/10 compared with the original antibody.

ED50 of intercellular adhesion action in the invention is determined by known methods for measuring agonist action, for example, by the measurement of agglomeration action of cells expressing said cell surface molecule such as hemagglutination test.

The invention relates to DNAs which code for the modified antibodies.

The invention relates to animal cells or microorganisms which produce the modified antibodies.

The invention relates to use of the modified antibody as an agonist.

The invention relates to a method of transducing a signal into cells by crosslinking cell surface molecule or intracellular molecule using the modified antibody and thereby inducing an agonist action of cells such as apoptosis induction, cell proliferation induction, cell differentiation induction, cell division induction or cell cycle regulation action.

The invention relates to a medicine containing the modified antibody.

The invention relates to use of the modified antibody as a medicine.

The invention relates to a method of screening or measuring the modified antibody, which contains two or more H chain V regions and two or more L chain V regions of antibody and shows an agonist action by crosslinking cell surface molecule or intracellular molecule, that comprises 1) to prepare a modified antibody containing two or more H chain V regions and two or more L chain V regions of antibody and binding specifically to said molecule, 2) to contact the modified antibody with cells expressing said molecule and 3) to measure an agonist action which occurs in the cells caused by crosslinking said molecule. The method of measurement is useful for the quality control in producing the modified antibodies of the invention as a medicine and other purposes.

The above-mentioned single chain Fv dimer includes a dimer by non-covalent bond, a dimer by a covalent bond through a crosslinking radical and a dimer through a crosslinking reagent (an antibody, an antibody fragment, or bivalent modified antibody). Conventional crosslinking radicals used for crosslinking peptides can be used as the crosslinking radicals to form the dimers. Examples are disulfide crosslinking by cysteine residue, other crosslinking radicals such as C₄ - C₁₀ alkylene (e.g. tetramethylene, pentamethylene, hexamethylene, heptamethylene and octamethylene, etc.) or C₄ - C₁₀ alkenylene (cis/trans -3-butenylene, cis/trans-2-pentenylene, cis/trans-3-pentenylene, cis/trans-3-hexenylene, etc.).

Moreover, the crosslinking reagent which can combine with a single chain Fv is, for example, an amino acid sequence which can optionally be introduced into Fv, for example, an antibody against FLAG sequence and the like or a fragment thereof, or a modified antibody originated from the antibody, for example, single chain Fv.

The invention also relates to a method of inducing an agonist action to cells by administering the first ligand and the second ligand which combine with a cell surface molecule(s) or intracellular molecule(s), and administering a substance which combine with the first and the second ligands and crosslink the first and second ligands. The first ligand and the second ligand can be any things which contain a biding site to said molecule and can induce an agonist action by being crosslinked. Preferable examples are monovalent modified antibodies, such as the same or different single chain Fv monomer, a fragment of antibody etc. The substance to crosslink the above-mentioned ligand can be any things that induce an agonist action to the cells by crosslinking the first ligand and the second ligand. Preferable examples are antibodies, fragments of antibodies, (Fab)₂ or bivalent modified antibodies. Examples of bivalent antibodies are (Fab)₂, dimers of single chain Fv containing one H chain V region and one L chain V region and single chain polypeptides containing two H chain V regions and two L chain V regions. The method is effective for exploring receptors that transduce a signal into cells by crosslinking, is expected to be employed for DDS to deliver a medicine to target cells and is also useful as a drug administration system which suppresses side effect and allows a medicine to become effective at desired time and for desired period.

The modified antibodies of this invention can be any things which contain L chain V region and H chain V region of antibody (e.g. antibody MABL-1, antibody MABL-2, antibody 12B5, antibody 12E10 etc.) and which specifically recognize the cell surface molecule(s) or intracellular molecule(s), for example, a protein (a receptor or a protein involved in signal transduction), or a sugar chain of the above-mentioned protein or of a cell membrane protein and crosslink said cell surface molecule(s), thereby transduce a signal into cells. Modified antibodies in which a part of amino acid sequence of V region has been altered are included.

Depending upon the characteristics of cell surface molecule or intracellular molecule to be combined, for example, the structure of molecule or the action mechanism, the modified antibodies can be mono-specific or multi-specific like bi-specific. When the modified antibody is combined with a receptor molecule which homodimerizes and transduces a signal into the cells (e.g. erythropoietin receptor, thrombopoietin receptor, G-CSF receptor, SCF receptor, EGF receptor, IAP(CD47) and the like), mono-specific modified antibody is preferable. When it is combined with a receptor molecule which heterodimerizes and transduces a signal into the cells (e.g. IL-6 receptor, LIF receptor, IL-11 receptor), bi-specific modified antibody is preferable. When it is combined with a receptor molecule which heterotrimerizes and transduces a signal into the cells (e.g. IL-2 receptor, CNTF receptor, OSM receptor), tri-specific modified antibody is preferable. A method for producing bi-specific single chain Fv dimers is described in WO9413804 and the like.

The present invention also relates to modified antibodies whose H chain V region and/or L chain V region is H chain V region derived from human antibody and/or L chain V region derived from human antibody. The H chain V region and/or L chain V region derived from human antibody can be obtained by screening human nomoclonal antibody's library as described in WO99/10494. The H chain V region and L chain V region derived from human monoclonal antibodies are also included.

The present invention further relates to modified antibodies whose H chain V regions and/or L chain V regions are humanized H chain V regions and/or humanized L chain V regions. Specifically, the humanized modified antibodies consist of the humanized L chain V region which comprises framework regions (FR) derived from an L chain V region of human monoclonal antibody and complementarity determining regions (hereinafter "CDR") derived from an L chain V region of non-human mammalian (e.g. mouse, rat, bovine, sheep, ape) monoclonal antibody and/or the humanized H chain V region which comprises FR derived from an H chain V region of human monoclonal antibody and CDR derived from an H chain V region of non-human mammalian (e.g. mouse, rat, bovine, sheep, ape) monoclonal antibody. In this case, the amino acid sequence of CDR and FR may be partially altered, e.g. deleted, replaced or added.

H chain V regions and/or L chain V regions of the modified antibodies of the invention can be H chain V regions and/or L chain V regions derived from monoclonal antibodies of animals other than human (such as mouse, rat, bovine, sheep, ape, chicken and the like). In this case, the amino acid sequence of CDR and FR may be partially altered, e.g. deleted, replaced or added.

The invention also relates to DNAs encoding the various modified antibodies as mentioned above and genetic engineering techniques for producing recombinant vectors comprising the DNAs.

The invention also relates to host cells transformed with the recombinant vectors. Examples of host cells are animal cells such as human cells, mouse cells or the like and microorganisms such as E. coli, Bacillus subtilis, yeast or the like.

The invention relates to a process for producing the modified antibodies, which comprises culturing the above-mentioned hosts and extracting the modified antibodies from the culture thereof.

The present invention further relates to a process for producing a dimer of the single chain Fv which comprises culturing host animal cells producing the single chain Fv in a serum-free medium to secrete the single chain Fv into the medium and isolating the dimer of the single chain Fv formed in the medium.

The present invention also relates to the use of the modified antibodies as an agonist. That is, it relates to the signal-transduction agonist which comprises as an active ingredient the modified antibody obtained as mentioned above. Since the modified antibodies used in the invention are those that crosslink a cell surface molecule(s) or intracellular molecule(s) and induce signal transduction, the molecule can be any molecule that is oligomerized, e.g. dimerized, by combining with the ligand and thereby transduce a signal into cells.

Such cell surface molecule includes hormone receptors and cytokine receptors. The hormone receptor includes, for example, estrogen receptor. The cytokine receptor and the like include hematopoietic factor receptor, lymphokine receptor, growth factor receptor, differentiation control factor receptor and the like. Examples of cytokine receptors are erythropoietin (EPO) receptor, thrombopoietin (TPO) receptor, granulocyte colony stimulating factor (G-CSF) receptor, macrophage colony stimulating factor (M-CSF) receptor, granular macrophage colony stimulating factor (GMCSF) receptor, tumor necrosis factor (TNF) receptor, interleukin-1 (IL-1) receptor, interleukin-2 (IL-2) receptor, interleukin-3 (IL-3) receptor, interleukin-4 (IL-4) receptor, interleukin-5 (IL-5) receptor, interleukin-6 (IL-6) receptor, interleukin-7 (IL-7) receptor, interleukin-9 (IL-9) receptor, interleukin-10 (IL-10) receptor, interleukin-11 (IL-11) receptor, interleukin-12 (IL-12) receptor, interleukin-13 (IL-13) receptor, interleukin-15 (IL-15) receptor, interferon-alpha (IFN-alpha) receptor, interferon-beta (IFN-beta) receptor, interferon-gamma (IFN-gamma) receptor, growth hormone (GH) receptor, insulin receptor, blood stem cell proliferation factor (SCF) receptor, vascular endothelial growth factor (VEGF) receptor, epidermal cell growth factor (EGF) receptor, nerve growth factor (NGF) receptor, fibroblast growth factor (FGF) receptor, platelet-derived growth factor (PDGF) receptor, transforming growth factor-beta (TGF-beta) receptor, leukocyte migration inhibitory factor (LIF) receptor, ciliary neurotrophic factor (CNTF) receptor, oncostatin M (OSM) receptor, Notch family receptor and the like.

The intracellular surface molecule includes TAK1, TAB1 and the like. TAK1 and TAB1 act in signal transduction pathway of TGF-β, activate MAP kinase by forming hetero-dimer and transduce a series of signals. Many cancer cells have mutation of TGF-β receptor, which represses the growth of cancer, and, therefore, the signal of TGF-β is not transduced. The modified antibodies, which can transduce a signal by crosslinking TAK1 and TAB1, can induce the signal of TGF-β through an agonistic action by combining with TAK1/TAB1. Such modified antibodies of the invention can inhibit the growth of TGF-β resistant cancer cells and provide a new method for cancer therapy. Other examples of intracellular molecule are transcription factor E2F homodimer and E2F/DP1 hetero-dimer having cell proliferation action. The modified antibodies of the invention can induce an agonist action also on those molecules, and therefore can be used for the treatment of various cell-proliferation-related diseases. The modified antibodies of the invention can induce an agonist action by crosslinking intracellular factor involved in apoptosis-induction-related signal transduction and therefore can induce apoptosis cell death of cancer cells or autoimmune-disease-related cells.

To achieve the interaction of the modified antibodies of the invention with intracellular molecule, peptides with cell-membrane-permeation-ability (e.g. Pegelin, Penetratin) can be used to transport the modified antibodies into the cells (Martine Mazel et al, Doxorubicin-peptide conjugates overcome multidrug resistance. AntiCancer Drugs 2001, 12, Dccrossi D. et al., The third helix of the antennapedia homeodomain translocates through biological membranes, J. Biol. Chem. 1994, 269, 10444-10450).

Therefore, the pharmaceutical preparations containing the agonist modified antibody as an active ingredient are useful as preventives and/or remedies etc. for various diseases such as cancers, inflammation, hormone disorders, blood diseases and autoimmune diseases.

Oligomers which can be formed by receptor proteins can be homo-oligomers or hetero-oligomers, and any oligomers such as dimers, trimers and tetramers. It is known for example that erythropoietin receptor, thrombopoietin receptor, G-CSF receptor, SCF receptor, EGF receptor and the like form homo-dimers, that IL-6 receptor, LIF receptor and IL-11 receptor form hetero-dimers and that IL-2 receptor, CNTF receptor, OSM receptor form hetero-trimers.

The modified antibodies of the present invention comprise two or more H chain V regions and two or more L chain V regions derived from monoclonal antibodies. The structure of the modified antibodies may be a dimer of single chain Fv comprising one H chain V region and one L chain V region or a polypeptide comprising two H chain V regions and two L chain V regions. In the modified antibodies of the invention, the V regions of H chain and L chain are preferably linked through a peptide linker which consists of one or more amino acids. The resulting modified antibodies contain variable regions of antibodies and bind to the antigen with the same specificity as that of the original monoclonal antibodies.

### H chain V region

In the present invention, the H chain V region derived from an antibody recognizes a cell surface molecule(s) or intracellular molecule(s), for example, a protein (a receptor or a signal-transduction-related protein) or a sugar chain of the protein or on cell membrane and oligomerizes, for example, dimerizes through crosslinking said molecule, and thereby transduces a signal into the cells. The H chain V region of the invention includes H chain V regions derived from a mammal (e.g. human, mouse, rat, bovine, sheep, ape etc.) and H chain V regions having partially modified amino acid sequences of the H chain V regions. More preferable is a humanized H chain V region containing FR of H chain V region of a human monoclonal antibody and CDR of H chain V region of a mouse monoclonal antibody. Also preferable is an H chain V region having an amino acid sequence derived from a human, which can be produced by recombination technique. The H chain V region of the invention may be a fragment of aforementioned H chain V region, which fragment preserves the antigen binding capacity.

### L chain V region

In the present invention, the L chain V region recognizes a cell surface molecule(s) or intracellular molecule(s), for example, a protein (a receptor or a signal-transduction-related protein) or a sugar chain of the protein or on cell membrane and oligomerizes, for example, dimerizes through crosslinking said molecule, and thereby transduces a signal into the cells. The L chain V region of the invention includes L chain V regions derived from a mammal (e.g. human, mouse, rat, bovine, sheep, ape etc.) and L chain V regions having partially modified amino acid sequences of the L chain V regions. More preferable is a humanized L chain V region containing FR of L chain V region of human monoclonal antibody and CDR of L chain V region of mouse monoclonal antibodies. Also preferable is an L chain V region having an amino acid sequence derived from a human antibody, which can be produced by recombination technique. The L chain V regions of the invention may be fragments of L chain V region, which fragments preserve the antigen binding capacity.

### Complementarity determining region (CDR)

Each V region of L chain and H chain forms an antigen-binding site. The variable region of the L and H chains is composed of comparatively conserved four common framework regions linked to three hypervariable regions or complementarity determining regions (CDR) (Kabat, E.A. et al., "Sequences of Protein of Immunological Interest", US Dept. Health and Human Services, 1983).

Major portions in the four framework regions (FRs) form β-sheet structures and thus three CDRs form a loop. CDRs may form a part of the β-sheet structure in certain cases. The three CDRs are held sterically close position to each other by FR, which contributes to the formation of the antigen-binding site together with three CDRs.

These CDRs can be identified by comparing the amino acid sequence of V region of the obtained antibody with known amino acid sequences of V regions of known antibodies according to the empirical rule in Kabat, E.A. et al., "Sequences of Protein of Immunological Interest".

### Single chain Fv

A single chain Fv is a polypeptide monomer comprising an H chain V region and an L chain V region linked each other which are derived from monoclonal antibodies. The resulting single chain Fvs contain variable regions of the parent monoclonal antibodies and preserve the complementarity determining region thereof, and therefore the single chain Fvs bind to the antigen by the same specificity as that of the parent monoclonal antibodies (JP-Appl. 11-63557). A part of the variable region and/or CDR of the single chain Fv of the invention or a part of the amino acid sequence thereof may be partially altered, for example, deleted, replaced or added. The H chain V region and L chain V region composing the single chain Fv of the invention are mentioned before and may be linked directly or through a linker, preferably a peptide linker. The constitution of the single chain Fv may be [H chain V region]-[L chain V region] or [L chain V regionl-[H chain V region]. In the present invention, it is possible to make the single chain Fv to form a dimer, a trimer or a tetramer, from which the modified antibody of the invention can be formed.

### Single chain modified antibody

The single chain modified antibodies of the present invention comprising two or more H chain V regions and two or more L chain V regions, preferably each two to four, especially preferable each two, comprise two or more H chain V regions and L chain V regions as mentioned above. Each region of the peptide should be arranged such that the modified single chain antibody forms a specific steric structure, concretely mimicking a steric structure formed by the dimer of single chain Fv. For instance, the V regions are arranged in the order of the following manner:
[H chain V region]-[L chain V region]-[H chain V region]-[1 chain V region]; or
[L chain V region]-[H chain V region]-[L chain V region]-[H chain V region],
   wherein these regions are connected through a peptide linker, respectively.

### Linker

In this invention, the linkers for the connection between the H chain V region and the L chain V region may be any peptide linker which can be introduced by the genetic engineering procedure or any linker chemically synthesized. For instance, linkers disclosed in literatures, e.g. Protein Engineering, 9(3), 299-305, 1996 may be used in the invention. These linkers can be the same or different in the same molecule. If peptide linkers are required, the following are cited as example linkers:
Ser
Gly-Ser
Gly-Gly-Ser
Ser-Gly-Gly
Gly-Gly-Gly-Ser
Ser-Gly-Gly-Gly
Gly-Gly-Gly-Gly-Ser
Ser-Gly-Gly-Gly-Gly
Gly-Gly-Gly-Gly-Gly-Ser
Ser-Gly-Gly-Gly-Gly-Gly
Gly-Gly-Gly-Gly-Gly-Gly-Ser
Ser-Gly-Gly-Gly-Gly-Gly-Gly
(Gly-Gly-Gly-Gly-Ser)ₙ and
(Ser-Gly-Gly-Gly-Gly)ₙ
wherein n is an integer not less than one. Preferable length of the linker peptide varies dependent upon the receptor to be the antigen, in the case of single chain Fvs, the range of 1 to 20 amino acids is normally preferable. In the case of single chain modified antibodies comprising two or more H chain V regions and two or more L chain V regions, the peptide linkers connecting those forming the same antigen binding site comprising [H chain V region]-[L chain V region] (or [L chain V region]-[H chain V region]) have lengths of 1 - 30 amino acids, preferably 1 - 20 amino acids, more preferably 3 - 18 amino acids. The peptide linkers connecting those not forming the same antigen biding site comprising [H chain V region]-[L chain V region] or ([L chain V region] - [H chain V region]) have lengths of 1 - 40 amino acids, preferably 3 - 30 amino acids, more preferably 5 - 20 amino acids. The method for introducing those linkers will be described in the explanation for DNA construction coding for modified antibodies of the invention.

The chemically synthesized linkers, i.e. the chemical crosslinking agents, according to the invention can be any linkers conventionally employed for the linkage of peptides. Examples of the linkers may include N-hydroxy succinimide (NHS), disuccinimidyl suberate (DSS), bis(sulfosuccinimidyl)suberate (BS³), dithiobis(succinimidyl propionate) (DSP), dithiobis(sulfosuccinimidyl propionate) (DTSSP), ethylene glycolbis (succinimidyl succinate) (EGS), ethylene glycolbis (sulfosuccinimidyl succinate) (sulfo-EGS), disuccinimidyl tartrate (DST), disulfosuccinimidyl tartrate (sulfo-DST), bis[2-(succinimido oxycarbonyloxy)ethyl]sulfone (BSOCOES), bis[2-(sulfosuccinimido oxycarbonyloxy) ethyl]sulfone (sulfo-BSOCOES) or the like. These are commercially available. It is preferable for the chemically synthesized linkers to have the length equivalent to that of peptide linkers.

To form a dimer of the single chain Fv it is preferable to select a linker suitable to dimerize in the solution such as culture medium more than 20%, preferably more than 50%, more preferably more than 80%, most preferably more than 90% of the single chain Fv produced in the host cells. Specifically, preferable is a linker composed of 2 to 12 amino acids, preferably 3 to 10 amino acids or other linkers corresponding thereto.

### Preparation of Modified antibodies

The modified antibodies can be produced by connecting, through the aforementioned linker, an H chain V region and an L chain V region derived from known or novel monoclonal antibodies specifically binding to a cell surface molecule(s). As examples of the single chain Fvs are cited MABL1-scFv and MABL2-scFv comprising the H chain V region and the L chain V region derived from the antibody MABL-1 and the antibody MABL-2, respectively. As examples of the single chain polypeptides comprising two H chain V regions and two L chain V regions are cited MABL1-sc(Fv)₂ and MABL2-SC (Fv)₂ comprising the H chain V region and the L chain V region derived from the aforementioned antibodies.

For the preparation of the polypeptide, a signal peptide may be attached to N-terminal of the polypeptide if the polypeptide is desired to be a secretory peptide. A well-known amino acid sequence useful for the purification of polypeptide such as the FLAG sequence may be attached for the efficient purification of the polypeptide. In this case a dimer can be formed by using anti-FLAG antibody.

For the preparation of the modified antibody of the invention, it is necessary to obtain a DNA, i.e. a DNA encoding the single chain Fv or a DNA encoding reconstructed single chain polypeptide. These DNAs, especially for MABL1-scFv, MABL2-scFv, MABL1-sc(Fv)₂ and/or MABL2-sc(Fv)₂ are obtainable from the DNAs encoding the H chain V region and the L chain V region derived from said Fv. They are also obtainable by polymerase chain reaction (PCR) method using those DNA as a template and amplifying the part of DNA contained therein encoding desired amino acid sequence with the aid of a pair of primers corresponding to both ends thereof.

In the case where each V region having partially modified amino acid sequence is desired, the V regions in which one or some amino acids are modified, i.e. deleted, replaced or added can be obtained by a procedure known in the art using PCR. A part of the amino acid sequence in the V region is preferably modified by the PCR known in the art in order to prepare the modified antibody which is sufficiently active against the specific antigen.

For the determination of primers for the PCR amplification, it is necessary to decide the type of the H chain and L chain of the desired antibodies. In the case of antibody MABL-1 and the antibody MABL-2 it has been reported, however, that the antibody MABL-1 has κ type L chains and γ1 type H chains and the antibody MABL-2 has κ type L chains and γ2a type H chains (JP-Appl. 11-63557). For the PCR amplification of the DNA encoding the H chain and L chain of the antibody MABL-1 and/or the antibody MABL-2, primers described in Jones, S.T. et al., Bio/Technology, 9, 88-89, 1991 may be employed.

For the amplification of the L chain V regions of the antibody MABL-1 and the antibody MABL-2 by PCR, 5'-end and 3'-end oligonucleotide primers are decided as aforementioned. In the same manner, 5'-end and 3'-end oligonucleotide primers are decided for the amplification of the H chain V regions of the antibody MARL-1 and the antibody MABL-2.

In embodiments of the invention, the 5'-end primers which contain a sequence "GANTC" providing the restriction enzyme Hinf I recognition site at the neighborhood of 5'-terminal thereof are used and the 3'-end primers which contain a nucleotide sequence "CCCGGG" providing the XmaI recognition site at the neighborhood of 5'-terminal thereof are used. Other restriction enzyme recognition site may be used instead of these sites as long as they are used for subcloning a desired DNA fragment into a cloning vector.

Specifically designed PCR primers are employed to provide suitable nucleotide sequences at 5'-end and 3'-end of the cDNAs encoding the V regions of the antibodies MABL-1 and MABL-2 so that the cDNAs are readily inserted into an expression vector and appropriately function in the expression vector (e.g. this invention devises to increase translation efficiency by inserting Kozak sequence). The V regions of the antibodies MAEL-1 and MABL-2 obtained by amplifying by PCR using these primers are inserted into HEF expression vector containing the desired human C region (see WO92/19759). The cloned DNAs can be sequenced by using any conventional process, for example, by the automatic DNA sequencer (Applied Biosystems).

A linker such as a peptide linker can be introduced into the modified antibody of the invention in the following manner. Primers which have partially complementary sequence with the primers for the H chain V regions and the L chain V regions as described above and which code for the N-terminal or the C-terminal of the linker are designed. Then, the PCR procedure can be carried out using these primers to prepare a DNA encoding the peptide linker having desired amino acid sequence and length. The DNAs encoding the H chain V region and the L chain V region can be connected through the resulting DNA to produce the DNA encoding the modified antibody of the invention which has the desired peptide linker. Once the DNA encoding one of the modified antibodies is prepared, the DNAs encoding the modified antibodies with or without the desired peptide linker can readily be produced by designing various primers for the linker and then carrying out the PCR using the primers and the aforementioned DNA as a template.

Each V region of the modified antibody of the present invention can be humanized by using conventional techniques (e.g. Sato, K. et al., Cancer Res., 53, 1-6 (1993)). Once a DNA encoding each of humanized Fvs is prepared, a humanized single chain Fv, a fragment of the humanized single chain Fv, a humanized monoclonal antibody and a fragment of the humanized monoclonal antibody can readily be produced according to conventional methods. Preferably, amino acid sequences of the V regions thereof may be partially modified, if necessary.

Furthermore, a DNA derived from other mammalian origin, for example a DNA encoding each of V regions of human antibody, can be produced in the same manner as used to produce DNA encoding the H chain V region and the L chain V region derived from mouse by conventional methods as mentioned in the above. The resulting DNA can be used to prepare an H chain V region and an L chain V region of other mammal, especially derived from human antibody, a single chain Fv derived from human and a fragment thereof, and a monoclonal antibody of human origin and a fragment thereof.

When the modified antibodies of the invention is bi-specific modified antibodies, they can be produced by known methods (for example, the method described in WO9413804).

As mentioned above, when the aimed DNAs encoding the V regions of the modified antibodies and the V regions of the humanized modified antibodies are prepared, the expression vectors containing them and hosts transformed with the vectors can be obtained according to conventional methods. Further, the hosts can be cultured according to a conventional method to produce the reconstructed single chain Fv, the reconstructed humanized single chain Fv, the humanized monoclonal antibodies and fragments thereof. They can be isolated from cells or a medium and can be purified into a homogeneous mass. For this purpose any isolation and purification methods conventionally used for proteins, e.g. chromatography, ultra-filtration, salting-cut and dialysis, may be employed in combination, if necessary, without limitation thereto.

When the reconstructed single chain Fv of the present invention is produced by culturing an animal cell such as COS7 cells or CHO cells, preferably CHO cells, in a serum-free medium, the dimer of said single chain Fv formed in the medium can be stably recovered and purified in a high yield. Thus purified dimer can be stably preserved for a long period. The serum-free medium employed in the invention may be any medium conventionally used for the production of a recombinant protein without limit thereto.

For the production of the modified antibodies of the present invention, any expression systems can be employed, for example, eukaryotic cells such as animal cells, e.g., established mammalian cell lines, filamentous fungi and yeast, and prokaryotic cells such as bacterial cells e.g., E. coli. Preferably, the modified antibodies of the invention are expressed in mammalian cells, for example COS7 cells or CHO cells.

In these cases, conventional promoters useful for the expression in mammalian cells can be used. Preferably, human cytomegalovirus (HCMV) immediate early promoter is used. Expression vectors containing the HCMV promoter include HCMV-VH-HCγ 1, HCMV-VL-HCK and the like which are derived from pSV2neo (WO92/19759).

Additionally, other promoters for gene expression in mammal cell which may be used in the invention include virus promoters derived form retrovirus, polyoma virus, adenovirus and simian virus 40 (SV40) and promoters derived from mammal such as human polypeptide-chain elongation factor-1α (HEF-1α). SV40 promoter can easily be used according to the method of Mulligan, R.C., et al. (Nature 277, 108-114 (1979)) and HEF-1α promoter can also be used according to the methods of Mizushima, S. et al. (Nucleic Acids Research, 18, 5322 (1990)).

Replication origin (ori) which can be used in the invention includes ori derived from SV40, polyoma virus, adenovirus, bovine papilloma virus (BPV) and the like. An expression vector may contain, as a selection marker, phosphotransferase APH (3') II or I (neo) gene, thymidine kinase (TK) gene, E. coli xanthine-guanine phosphoribosyl transferase (Ecogpt) gene or dihydrofolate reductase (DHFR) gene.

The antigen-binding activity of the modified antibody prepared in the above can be evaluated by a conventional method such as radio immunoassay (RIA), enzyme-linked immunosorbent assay (ELISA) or surface plasmon resonance. It can also be evaluated using the binding-inhibitory ability of original antibodies as an index, for example in terms of the absence or presence of concentration-dependent inhibition of the binding of said monoclonal antibody to the antigen.

More in detail, animal cells transformed with an expression vector containing a DNA encoding the modified antibody of the invention, e.g., COS7 cells or CHO cells, are cultured. The cultured cells and/or the supernatant of the medium or the modified antibody purified from them are used to determine the binding to antigen. As a control is used a supernatant of the culture medium in which cells transformed only with the expression vector were cultured. In the case of an antigen, for example, the antibody MABL-1 and the antibody MABL-2, a test sample of the modified antibody of the invention or the supernatant of the control is added to mouse leukemia cell line, L1210 cells, expressing human IAP and then an assay such as the flow cytometry is carried out to evaluate the antigen-binding activity.

In vitro evaluation of the signal transduction effect (apoptosis-inducing effect in the cases of the antibody MAEL-1 and the antibody MABL-2) is performed in the following manner: A test sample of the above modified antibody is added to the cells which are expressing the antibody or cells into which the gene for the antibody has been introduced, and is evaluated by the change caused by the signal transduction, for example, whether cell death is induced in a manner specific to the human IAP-antigen, using conventional methods.

In vivo evaluation of the apoptosis-inducing effect, for example, in the case where the modified antibody recognizes human IAP (e.g. modified antibodies derived from the antibody MABL-1 and the antibody MABL-2) is carried out in the following manner: A mouse model of human myeloma is prepared. To the mice is intravenously administered the monoclonal antibody or the modified antibody of the invention, which induces apoptosis of nucleated blood cells having IAP. To mice of a control group is administered PBS alone. The induction of apoptosis is evaluated in terms of antitumor effect based on the change of human IgG content in serum of the mice and their survival time.

As mentioned above the modified antibodies of the invention can be obtained by preparing modified antibodies which contain two or more H chain V regions and two or more L chain V regions and specifically bind to target cell surface molecule or intracellular molecule and screening the modified antibodies by in vivo or in vitro evaluation as mentioned in the above.

The modified antibodies of the invention, which comprises two or more H chain V regions and two or more L chain V regions, preferably each two to four, more preferably each two, may be a dimer of the single chain Fv comprising one H chain V region and one L chain V region, or a single chain polypeptide in which two or more H chain V regions and two or more L chain V regions are connected. It is considered that owing to such construction the peptide mimics three dimensional structure of a natural ligand and therefore retains an excellent antigen-binding property and agonist activity.

The modified antibodies of the invention have a remarkably lowered molecular size compared with antibody molecule (whole IgG), and, therefore, a superior permeability into tissues and tumors and a higher activity than original agonist monoclonal antibodies. Therefore, proper selection of the parent antibody makes it possible to transduce various signals into cells and to induce various actions in the cells such as apoptosis induction, cell proliferation induction, cell differentiation induction, cell division induction or cell cycle regulation action. The pharmaceutical preparations containing them are useful for treating diseases curable by inducing signal transduction, for example cancers, inflammation, hormone disorders, autoimmune diseases as well as blood dyscrasia, for example, leukemia, malignant lymphoma, aplastic anemia, myelodysplasia syndrome and polycythemia vera. It is further expected that the antibody of the invention can be used as a contrast agent by RI-labeling. The effect can be enhanced by attaching to a RI-compound or a toxin.

### BEST MODE FOR WORKING THE INVENTION

The present invention will concretely be illustrated in reference to the following examples, which in no way limit the scope of the invention.

For illustrating the production process of the modified antibodies of the invention, examples of producing single chain Fvs are shown below. Mouse antibodies against human IAP, MABL-1 and MABL-2 were used in the examples of producing the modified antibodies. Hybridomas MABL-1 and MABL-2 producing them respectively were internationally deposited as FERM BP-6100 and FERM BP-6101 with the National Institute of Bioscience and Human Technology, Agency of Industrial Science and Technology, Minister of International Trade and Industry (1-3 Higasi 1-chome, Tsukuba-shi, Ibaraki-ken, Japan), an authorized depository for microorganisms, on September 11, 1997.

### Examples

### Example 1 (Cloning of DNAs encoding V region of mouse monoclonal antibodies to human IAP)

DNAs encoding variable regions of the mouse monoclonal antibodies to human IAP, MAB1-1 and MABL-2, were cloned as follows.

### 1.1 Preparation of messenger RNA (mRNA)

mRNAs of the hybridomas MABL-1 and MABL-2 were obtained by using mRNA Purification Kit (Pharmacia Biotech).

### 1.2 Synthesis of double-stranded cDNA

Double-stranded cDNA was synthesized from about 1 µg of the mRNA using Marathon cDNA Amplification Kit (CLONTECH) and an adapter was linked thereto.

### 1.3 PCR Amplification of genes encoding variable regions of an antibody by

PCR was carried out using Thermal Cycler (PERKIN ELMER).

### (1) Amplification of a gene coding for L chain V region of MABL-1

Primers used for the PCR method are Adapter Primer-1 (CLONTECH) shown in SEQ ID No. 1, which hybridizes to a partial sequence of the adapter, and MKC (Mouse Kappa Constant) primer (Bio/Technology, 9, 88-89, 1991) shown in SEQ ID No. 2, which hybridizes to the mouse kappa type L chain V region.

50 µl of the PCR solution contains 5 µl of 10 × PCR Buffer II, 2 mM MgCl₂, 0.16 mM dNTPs (dATP, dGTP, dCTP and dTTP) , 2.5 units of a DNA polymerase, AmpliTaq Gold (PERKIN ELMER), 0.2 µM of the adapter primer of SEQ ID No. 1, 0.2 µM of the MKC primer of SEQ ID No. 2 and 0.1 µg of the double-stranded cDNA derived from MABL-1. The solution was preheated at 94°C of the initial temperature for 9 minutes and then heated at 94°C for 1 minute, at 60°C for 1 minute and at 72°C for 1 minute 20 seconds in order. This temperature cycle was repeated 35 times and then the reaction mixture was further heated at 72°C for 10 minutes.

### (2) Amplification of cDNA encoding H chain V region of MABL-1

The Adapter Primer-1 shown in SEQ ID No. 1 and MHC-γ1 (Mouse Heavy Constant) primer (Bio/Technology, 9, 88-89, 1991) shown in SEQ ID No. 3 were used as primers for PCR.

The amplification of cDNA was performed according to the method of the amplification of the L chain V region gene, which was described in Example 1.3-(1), except for using 0.2 µM of the MHC-γ1 primer instead of 0.2 µM of the MKC primer.

### (3) Amplification of cDNA encoding L chain V region of MABL-2

The Adapter Primer-1 of SEQ ID No. 1 and the MKC primer of SEQ ID No. 2 were used as primers for PCR.

The amplification of cDNA was carried out according to the method of the amplification of the L chain V region gene of MABL-1 which was described in Example 1.3-(1), except for using 0.1 µg of the double-stranded cDNA derived from MAB1-2 instead of 0.1 µg of the double-stranded cDNA from MABL-1.

### (4) Amplification of cDNA encoding H chain V region of MABL-2

The Adapter Primer-1 of SEQ ID No. 1 and MHC-γ2a primer (Bio/Technology, 9, 88-89, 1991) shown in SEQ ID No. 4 were used as primers for PCR.

The amplification of cDNA was performed according to the method of the amplification of the L chain V region gene, which was described in Example 1.3-(3), except for using 0.2 µM of the MHC-γ2a primer instead of 0.2 µM of the MKC primer.

### 1.4 Purification of PCR products

The DNA fragment amplified by PCR as described above was purified using the QIAquick PCR Purification Kit (QIAGEN) and dissolved in 10 mM Tris-HCl (pH 8.0) containing 1 mM EDTA.

### 1.5 Ligation and Transformation

About 140 ng of the DNA fragment comprising the gene encoding the mouse kappa type L chain V region derived from MABL-1 as prepared above was ligated with 50 ng of pGEM-T Easy vector (Promega) in the reaction buffer comprising 30 mM Tris-HCl (pH 7.8), 10 mM MgCl₂, 10 mM dithiothreitol, 1 mM ATP and 3 units of T4 DNA Ligase (Promega) at 15°C for 3 hours.

Then, 1 µl of the reaction mixture was added to 50 µl of E. coli DH5α competent cells (Toyobo Inc.) and the cells were stored on ice for 30 minutes, incubated at 42°C for 1 minute and stored on ice for 2 minutes again. 100 µl of SOC medium (GIBCO BRL) was added. The cells of E. coli were plated on LB (Molecular Cloning: A Laboratory Manual, Sambrook et al., Cold Spring Harbor Laboratory Press, 1989) agar medium containing 100 µg/ml of ampicillin (SIGMA) and cultured at 37°C overnight to obtain the transformant of E. coli.

The transformant was cultured in 3 ml of LB medium containing 50 µg/ml of ampicillin at 37°C overnight and the plasmid DNA was prepared from the culture using the QIAprep Spin Miniprep Kit (QIAGEN).

The resulting plasmid comprising the gene encoding the mouse kappa type L chain V region derived from the hybridoma MABL-1 was designated as pGEM-M1L.

According to the same manner as described above, a plasmid comprising the gene encoding the mouse H chain V region derived from the hybridoma MABL-1 was prepared from the purified DNA fragment and designated as pGEM-M1H.

A plasmid comprising the gene encoding the mouse kappa type L chain V region derived from the hybridoma MABL-2 was prepared from the purified DNA fragment and designated as pGEM-M2L.

A plasmid comprising the gene encoding the mouse H chain V region derived from the hybridoma MABL-2 was prepared from the purified DNA fragment and designated as pGEM-M2H.

### Example 2 (DNA Sequencing)

The nucleotide sequence of the cDNA encoding region in the aforementioned plasmids was determined using Auto DNA Sequencer (Applied Biosystem) and ABI PRISM Dye Terminator Cycle Sequencing Ready Reaction Kit (Applied Biosystem) according to the manufacturer's protocol.

The nucleotide sequence of the gene encoding the L chain V region from the mouse antibody MABL-1, which is included in the plasmid pGEM-M1L, is shown in SEQ ID No. 5.

The nucleotide sequence of the gene encoding the H chain V region from the mouse antibody MABL-1, which is included in the plasmid pGEM-M1H, is shown in SEQ ID No. 6.

The nucleotide sequence of the gene encoding the L chain V region from the mouse antibody MABL-2, which is included in the plasmid pGEM-M2L, is shown in SEQ ID No. 7.

The nucleotide sequence of the gene encoding the H chain V region from the mouse antibody MABL-2, which is included in the plasmid pGEM-M2H, is shown in SEQ ID No. 8.

### Example 3 (Determination of CDR)

The V regions of L chain and H chain generally have a similarity in their structures and each four framework regions therein are linked by three hypervariable regions, i.e., complementarity determining regions (CDR). An amino acid sequence of the framework is relatively well conserved, while an amino acid sequence of CDR has extremely high variation (Kabat, E.A., et al., "Sequences of Proteins of Immunological Interest", US Dept. Health and Human Services, 1983) .

On the basis of these facts, the amino acid sequences of the variable regions from the mouse monoclonal antibodies to human IAP were applied to the database of amino acid sequences of the antibodies made by Kabat et al. to investigate the homology. The CDR regions were determined based on the homology as shown in Table 1.

**Table 1**

| Plasmid | SEQ ID No. | CDR(1) | CDR(2) | CDR(3) |
|---|---|---|---|---|
| pGEM-M1L | 5 | 43-58 | 74-80 | 113-121 |
| pGEM-M1H | 6 | 50-54 | 69-85 | 118-125 |
| pGFM-M2L | 7 | 43-58 | 74-80 | 113-121 |
| pGEM-M2H | 8 | 50-54 | 69-85 | 118-125 |

### Example 4 (Identification of Cloned cDNA Expression (Preparation of Chimera MABL-1 antibody and Chimera MABL-2 antibody.)

### 4.1 Preparation of vectors expressing chimera MABL-1 antibody

cDNA clones, pGEM-M1L and pGEM-M1H, encoding the V regions of the L chain and the H chain of the mouse antibody MABL-1, respectively, were modified by the PCR method and introduced into the HEF expression vector (WO92/19759) to prepare vectors expressing chimera MABL-1 antibody.

A forward primer MLS (SEQ ID No. 9) for the L chain V region and a forward primer MHS (SEQ ID No. 10) for the H chain V region were designed to hybridize to a DNA encoding the beginning of the leader sequence of each V region and to contain the Kozak consensus sequence (J. Mol. Biol., 196, 947-950, 1987) and HindIII restriction enzyme site. A reverse primer MLAS (SEQ ID No. 11) for the L chain V region and a reverse primer MHAS (SEQ ID No. 12) for the H chain V region were designed to hybridize to a DNA encoding the end of the J region and to contain the splice donor sequence and BamHI restriction enzyme site.

100 µl of a PCR solution comprising 10 µl of 10 × PCR Buffer II, 2 mM MgCl₂, 0.16 mM dNTPs (dATP, dGTP, dCTP and dTTP), 5 units of DNA polymerase AmpliTaq Gold, 0.4 µM each of primers and 8 ng of the template DNA (pGEM-M1L or pGEM-M1H) was preheated at 94°C of the initial temperature for 9 minutes and then heated at 94°C for 1 minute, at 60°C for 1 minute and at 72°C for 1 minute 20 seconds in order. This temperature cycle was repeated 35 times and then the reaction mixture was further heated at 72°C for 10 minutes.

The PCR product was purified using the QIAquick PCR Purification Kit (QIAGEN) and then digested with HindIII and BamHI. The product from the L chain V region was cloned into the HEF expression vector, HEF-κ and the product from the H chain V region was cloned into the HEF expression vector, HEF-γ. After DNA sequencing, plasmids containing a DNA fragment with a correct DNA sequence are designated as HEF-M1L and HEF-M1H, respectively.

### 4.2 Preparation of vectors expressing chimera MABL-2 antibodies

Modification and cloning of cDNA were performed in the same manner described in Example 4.1 except for using pGEM-M2L and pGEM-M2H as template DNA instead of pGEM-M1L and pGEM-M1H. After DNA sequencing, plasmids containing DNA fragments with correct DNA sequences are designated as HEF-M2L and HEF-M2H, respectively.

### 4.3 Transfection to COS7 cells

The aforementioned expression vectors were tested in COS7 cells to observe the transient expression of the chimera MABL-1 and MABL-2 antibodies.

### (1) Transfection with genes for the chimera MABL-1 antibody

COS7 cells were co-transformed with the HEF-M1L and HEF-M1H vectors by electroporation using the Gene Pulser apparatus (BioRad). Each DNA (10 µg) and 0.8 ml of PBS with 1 × 10⁷ cells/ml were added to a cuvette. The mixture was treated with pulse at 1.5 kV, 25 µF of electric capacity.

After the restoration for 10 minutes at a room temperature, the electroporated cells were transferred into DMEM culture medium (GIBCO BRL) containing 10% γ-globulin-free fetal bovine serum. After culturing for 72 hours, the supernatant was collected, centrifuged to remove cell fragments and recovered.

### (2) Transfection with genes coding for the chimera MABL-2 antibody

The co-transfection to COS7 cells with the genes coding for the chimera MABL-2 antibody was carried out in the same manner as described in Example 4.3-(1) except for using the HEF-M2L and HEF-M2H vectors instead of the HEF-M1L and HEF-M1H vectors. The supernatant was recovered in the same manner.

### 4.4 Flow cytometry

Flow cytometry was performed using the aforementioned culture supernatant of COS7 cells to measure binding to the antigen. The culture supernatant of the COS7 cells expressing the chimera MABL-1 antibody or the COS7 cells expressing the chimera MABL-2 antibody, or human IgG antibody (SIGMA) as a control was added to 4 × 10⁵ cells of mouse leukemia cell line L1210 expressing human IAP and incubated on ice. After washing, the FITC-labeled anti-human IgG antibody (Cappel) was added thereto. After incubating and washing, the fluorescence intensity thereof was measured using the FACScan apparatus (BECTON DICKINSON).

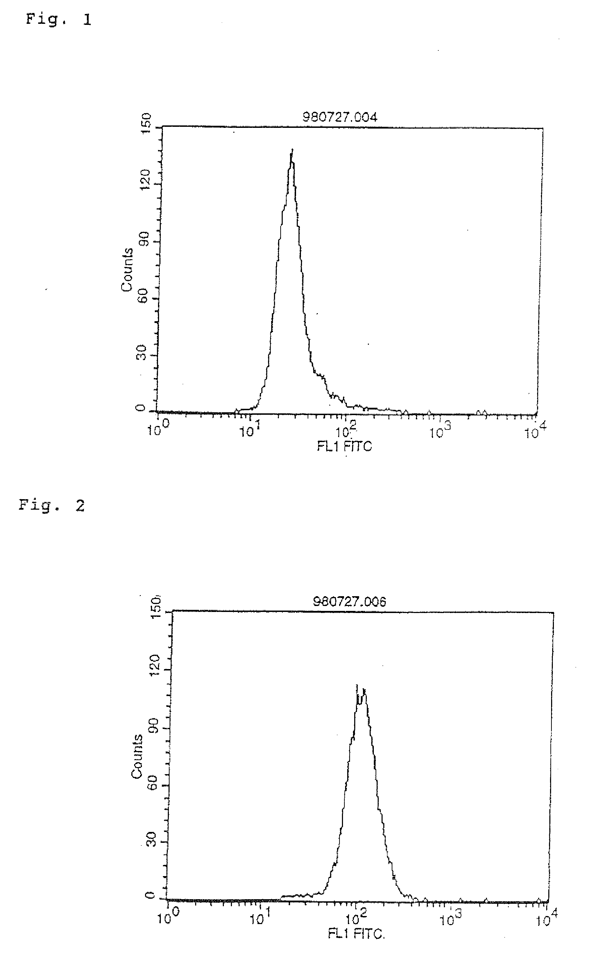

Since the chimera MABL-1 and MABL-2 antibodies were specifically bound to L1210 cells expressing human IAP, it is confirmed that these chimera antibodies have proper structures of the V regions of the mouse monoclonal antibodies MAB1-1 and MABL-2, respectively (Figs. 1-3).

### Example 5 (Preparation of reconstructed Single chain Fv (scFv) of the antibody MABL-1 and antibody MABL-2)

### 5.1. Preparation of reconstructed single chain Fv of antibody MABL-1

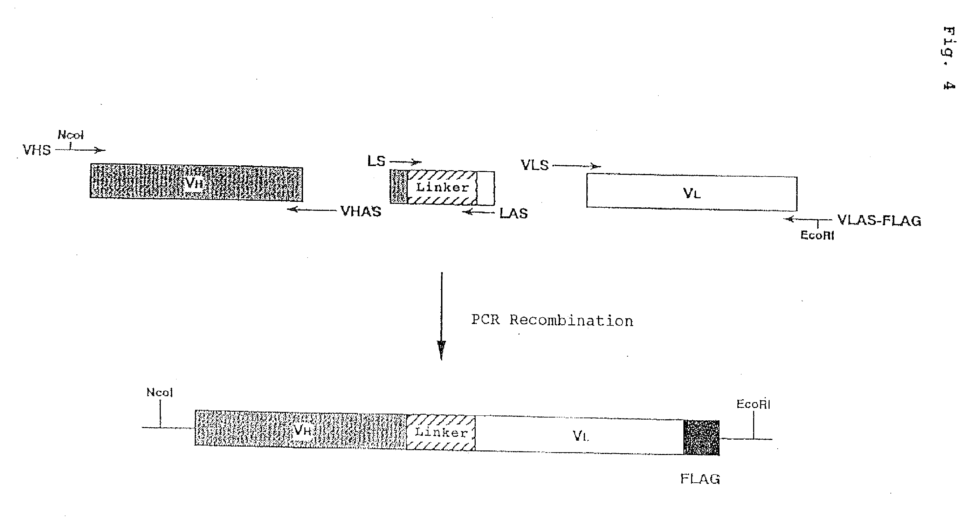

The reconstructed single chain Fv of antibody MABL-1 was prepared as follows. The H chain V region and the L chain V of antibody MABL-1, and a linker were respectively amplified by the PCR method and were connected to produce the reconstructed single chain Fv of antibody MABL-1. The production method is illustrated in Fig. 4. Six primers (A-F) were employed for the production of the single chain Fv of antibody MABL-1. Primers A, C and E have a sense sequence and primers B, D and F have an antisense sequence.

The forward primer VHS for the H chain V region (Primer A, SEQ ID No. 13) was designed to hybridize to a DNA encoding the N-terminal of the H chain V region and to contain NcoI restriction enzyme recognition site. The reverse primer VHAS for H chain V region (Primer B, SEQ ID No. 14) was designed to hybridize to a DNA coding the C-terminal of the H chain V region and to overlap with the linker.

The forward primer LS for the linker (Primer C, SEQ ID No. 15) was designed to hybridize to a DNA encoding the N-terminal of the linker and to overlap with a DNA encoding the C-terminal of the H chain V region. The reverse primer LAS for the linker (Primer D, SEQ ID No. 16) was designed to hybridize to a DNA encoding the C-terminal of the linker and to overlap with a DNA encoding the N-terminal of the L chain V region.

The forward primer VLS for the L chain V region (Primer E, SEQ ID No. 17) was designed to hybridize to a DNA encoding the C-terminal of the linker and to overlap with a DNA encoding the N-terminal of the L chain V region. The reverse primer VLAS-FLAG for L chain V region (Primer F, SEQ ID No. 18) was designed to hybridize to a DNA encoding the C-terminal of the L chain V region and to have a sequence encoding the FLAG peptide (Hopp. T. P. et al., Bio/Technology, 6, 1204-1210, 1988), two stop codons and EcoRI restriction enzyme recognition site.

In the first PCR step, three reactions, A-B, C-D and E-F, were carried out and PCR products thereof were purified. Three PCR products obtained from the first PCR step were assembled by their complementarity. Then, the primers A and F were added and the full length DNA encoding the reconstructed single chain Fv of antibody MABL-1 was amplified (Second PCR). In the first PCR, the plasmid pGEM-M1H encoding the H chain V region of antibody MABL-1 (see Example 2), a plasmid pSC-DP1 which comprises a DNA sequence encoding a linker region comprising: Gly Gly Gly Gly Ser Gly Gly Gly Gly Ser Gly Gly Gly Gly Ser (SEQ ID No. 19) (Huston, J.S., et al., Proc. Natl. Acad. Sci. USA, 85, 5879-5883, 1988) and the plasmid pGEM-M1L encoding the L chain V region of antibody MABL-1 (see Example 2) were employed as templ ate, respectively.

50 µl of the solution for the first PCR step comprises 5 µl of 10 × PCR Buffer II, 2 mM MgCl₂, 0.16 mM dNTPs, 2.5 units of DNA polymerase, AmpliTaq Gold (PERKIN ELMER), 0.4 µM each of primers and 5 ng each of template DNA. The PCR solution was preheated at 94°C of the initial temperature for 9 minutes and then heated at 94°C for 1 minute, at 65°C for 1 minute and at 72°C for 1 minute and 20 seconds in order. This temperature cycle was repeated 35 times and then the reaction mixture was further heated at 72°C for 7 minutes.

The PCR products A-B (371bp), C-D (63bp) and E-F (384bp) were purified using the QIAquick PCR Purification Kit (QIAGEN) and were assembled in the second PCR. In the second PCR, 98 µl of a PCR solution comprising 120 ng of the first PCR product A-B, 20 ng of the PCR product C-D and 120 ng of the PCR product E-F, 10 µl of 10 × PCR Buffer II, 2mM MgCl₂, 0.16 mM dNTPs, 5 units of DNA polymerase AmpliTaq Gold (PERKIN ELMER) was preheated at 94°C of the initial temperature for 8 minutes and then heated at 94°C for 2 minutes, at 65°C for 2 minutes and at 72°C for 2 minutes in order. This temperature cycle was repeated twice and then 0.4 µM each of primers A and F were added into the reaction, respectively. The mixture was preheated at 94°C of the initial temperature for 1 minutes and then heated at 94°C for 1 minute, at 65°C for 1 minute and at 72°C for 1 minute and 20 seconds in order. This temperature cycle was repeated 35 times and then the reaction mixture was further heated at 72°C for 7 minutes.

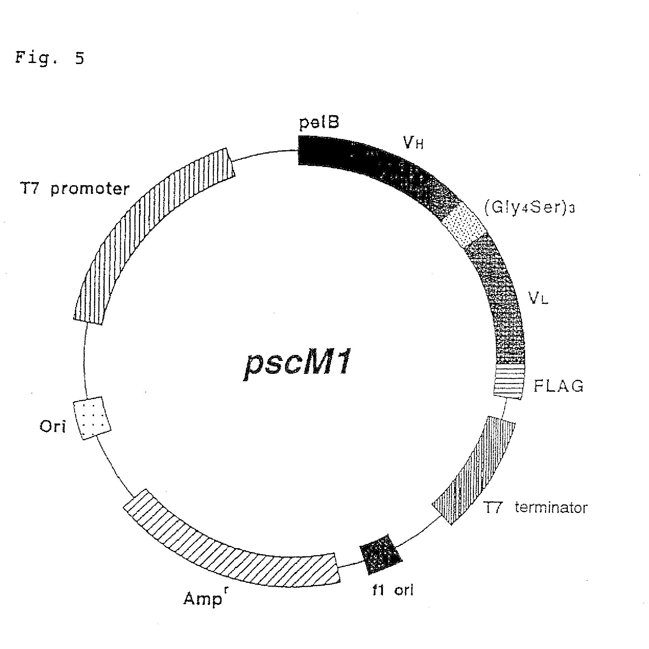

A DNA fragment of 843 bp produced by the second PCR was purified and digested by NcoI and EcoRI. The resultant DNA fragment was cloned into pSCFVT7 vector. The expression vector pSCFVT7 contains a pelB signal sequence suitable for E. coli periplasmic expression system (Lei, S.P., et al., J. Bacteriology, 169, 4379-4383, 1987). After the DNA sequencing, the plasmid containing the DNA fragment encoding correct amino acid sequence of the reconstructed single chain Fv of antibody MABL-1 is designated as "pscM1" (see Fig. 5). The nucleotide sequence and the amino acid sequence of the reconstructed single chain Fv of antibody MABL-1 contained in the plasmid pscM1 are shown in SEQ ID No. 20.

The pscM1 vector was modified by the PCR method to prepare a vector expressing the reconstructed single chain Fv of antibody MABL-1 in mammalian cells. The resultant DNA fragment was introduced into pCHO1 expression vector. This expression vector, pCHO1, was constructed by digesting DHFR-ΔE-rvH-PM1-f (WO92/19759) with EcoRI and SmaI to eliminate the antibody gene and connecting the EcoRI-NotI-BamHI Adapter (Takara Shuzo) thereto.

As a forward primer for PCR, Sal-VHS primer shown in SEQ ID No. 21 was designed to hybridize to a DNA encoding the N-terminal of the H chain V region and to contain SalI restriction enzyme recognition site. As a reverse primer for PCR, FRH1anti primer shown in SEQ ID No. 22 was designed to hybridize to a DNA encoding the end of the first framework sequence.

100 µl of PCR solution comprising 10 µl of 10 × PCR Buffer II, 2 mM MgCl₂, 0.16 mM dNTPs, 5 units of the DNA polymerase, AmpliTaq Gold, 0.4 µl M each of primer and 8 ng of the template DNA (pscM1) was preheated at 95°C of the initial temperature for 9 minutes and then heated at 95°C for 1 minute, at 60°C for 1 minute and at 72°C for 1 minute and 20 seconds in order. This temperature cycle was repeated 35 times and then the reaction mixture was further heated at 72°C for 7 minutes.

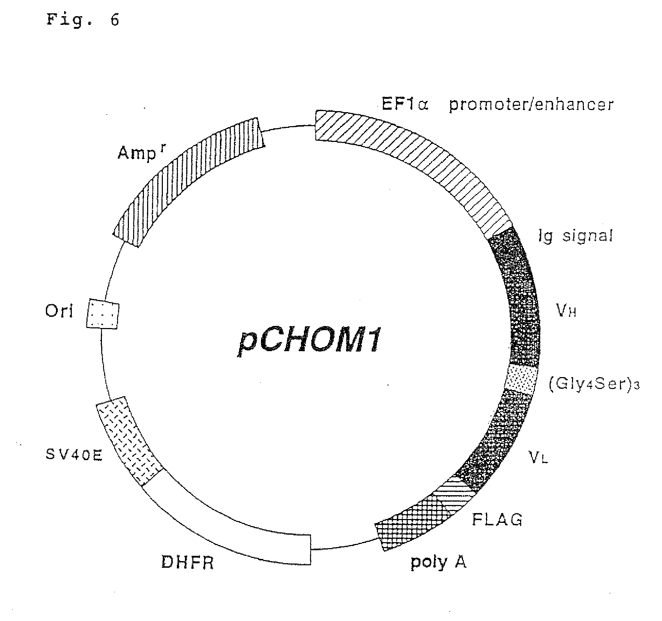

The PCR product was purified using the QIAquick PCR Purification Kit (QIAGEN) and digested by SalI and MboII to obtain a DNA fragment encoding the N-terminal of the reconstructed single chain Fv of antibody MABL-1 The pscM1 vector was digested by MboII and EcoRI to obtain a DNA fragment encoding the C-terminal of the reconstructed single chain Fv of antibody MABL-1. The SalI-MboII DNA fragment and the MboII-EcoRI DNA fragment were cloned into pCHO1-Igs vector. After DNA sequencing, the plasmid comprising the desired DNA sequence was designated as "pCHOM1" (see Fig. 6). The expression vector, pCHO1-Igs, contains a mouse IgG1 signal sequence suitable for the secretion-expression system in mammalian cells (Nature, 322, 323-327, 1988). The nucleotide sequence and the amino acid sequence of the reconstructed single chain Fv of antibody MABL-1 contained in the plasmid pCHOM1 are shown in SEQ ID No. 23.

### 5.2 Preparation of reconstructed single chain Fv of antibody MABL-2

The reconstructed single chain Fv of antibody MABL-2 was prepared in accordance with the aforementioned Example 5.1. Employed in the first PCR step were plasmid pGEM-M2H encoding the H chain V region of MABL-2 (see Example 2) instead of pGEM-M1H and plasmid pGEM-M2L encoding the L chain V region of MABL-2 (see Example 2) instead of pGEM-M1L, to obtain a plasmid pscM2 which comprises a DNA fragment encoding the desired amino acid sequence of the single chain Fv of antibody MABL-2. The nucleotide sequence and the amino acid sequence of the reconstructed single chain Fv of antibody MABL-2 contained in the plasmid pscM2 are shown in SEQ ID No. 24.

The pscM2 vector was modified by the PCR method to prepare a vector, pCHOM2, for the expression in mammalian cells which contains the DNA fragment encoding the correct amino acid sequence of reconstructed the single chain Fv of antibody MABL-2. The nucleotide sequence and the amino acid sequence of the reconstructed single chain Fv of antibody MABL-2 contained in the plasmid pCHOM2 are shown in SEQ ID No. 25.

### 5.3 Transfection to COS7 cells

The pCHOM2 vector was tested in COS7 cells to observe the transient expression of the reconstructed single chain Fv of antibody MABL-2.

The COS7 cells were transformed with the pCHOM2 vector by electroporation using the Gene Pulser apparatus (BioRad). The DNA (10 µg) and 0.8 ml of PBS with 1 × 10⁷ cells/ml were added to a cuvette. The mixture was treated with pulse at 1.5 kV, 25 µF of electric capacity.

After the restoration for 10 minutes at a room temperature, the electroporated cells were transferred into IMDM culture medium (GIBCO BRL) containing 10% fetal bovine serum. After culturing for 72 hours, the supernatant was collected, centrifuged to remove cell fragments and recovered.

### 5.4 Detection of the reconstructed single chain Fv of antibody MABL-2 in culture supernatants of COS7 cells

The existence of the single chain Fv of antibody MABL-2 in the culture supernatant of COS7 cells which had been transfected with the pCHOM2 vector was confirmed by the Western Blotting method.

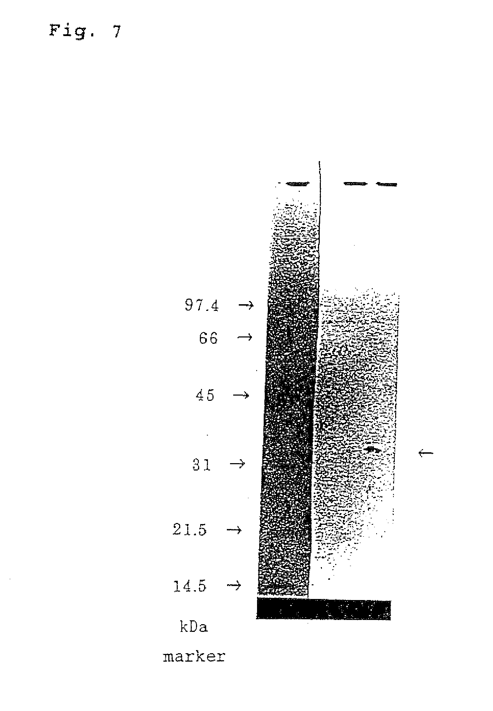

The culture supernatant of COS7 cells transfected with the pCHOM2 vector and the culture supernatant of COS7 cells transfected with the pCHO1 as a control were subjected to SDS electrophoresis and transferred to REINFORCED NC membrane (Schleicher & Schuell). The membrane was blocked with 5% skim milk (Morinaga Nyu-gyo), washed with 0.05% Tween 20-PBS and mixed with an anti-FLAG antibody (SIGMA). The membrane was incubated at room temperature, washed and mixed with alkaline phosphatase-conjugated mouse IgG antibody (Zymed). After incubating and washing at room temperature, the substrate solution (Kirkegaard Perry Laboratories) was added to develop color (Fig. 7).

A FLAG-peptide-specific protein was detected only in the culture supernatant of the pCHOM2 vector-introduced COS7 cells and thus it is confirmed that the reconstructed single chain Fv of antibody MABL-2 was secreted in this culture supernatant.

### 5.5 Flow cytometry

Flow cytometry was performed using the aforementioned COS7 cells culture supernatant to measure the binding to the antigen. The culture supernatant of the COS7 cells expressing the reconstructed single chain Fv of antibody MABL-2 or the culture supernatant of COS7 cells transformed with pCHO1 vector as a control was added to 2 × 10⁵ cells of the mouse leukemia cell line L1210 expressing human Integrin Associated Protein (IAP) or the cell line L1210 transformed with pCOS1 as a control. After incubating on ice and washing, the mouse anti-FLAG antibody (SIGMA) was added. Then the cells were incubated and washed. Then, the FITC labeled anti-mouse IgG antibody (BECTON DICKINSON) was added thereto and the cells were incubated and washed again. Subsequently, the fluorescence intensity was measured using the FACScan apparatus (BECTON DICKINSON).

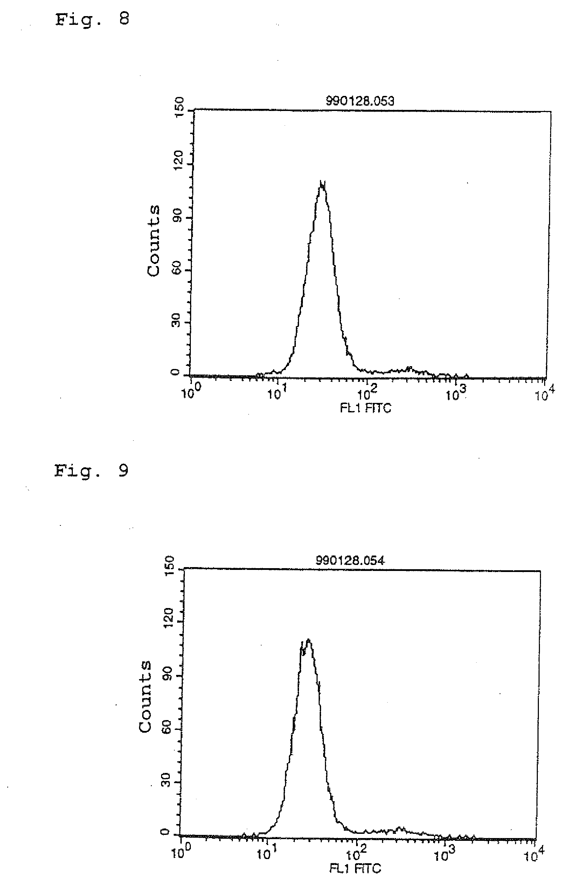

Since the single chain Fv of antibody MABL-2 was specifically bound to L1210 cells expressing human IAP, it is confirmed that the reconstructed single chain Fv of antibody MABL-2 has an affinity to human Integrin Associated Protein (IAP) (see Figs. 8-11).

### 5.6 Competitive ELISA

The binding activity of the reconstructed single chain Fv of antibody MABL-2 was measured based on the inhibiting activity against the binding of mouse monoclonal antibodies to the antigen.

The anti-FLAG antibody adjusted to 1 µg/ml was added to each well on 96-well plate and incubated at 37°C for 2 hours. After washing, blocking was performed with 1% BSA-PBS. After incubating and washing at a room temperature, the culture supernatant of COS7 cells into which the secretion-type human IAP antigen gene (SEQ ID No. 26) had been introduced was diluted with PBS into twofold volume and added to each well. After incubating and washing at a room temperature, a mixture of 50 µl of the biotinized MABL-2 antibody adjusted to 100 ng/ml and 50 µl of sequentially diluted supernatant of the COS7 cells expressing the reconstructed single chain Fv of antibody MABL-2 were added into each well. After incubating and washing at a room temperature, the alkaline phosphatase-conjugated streptoavidin (Zymed) was added into each well. After incubating and washing at a room temperature, the substrate solution (SIGMA) was added and absorbance of the reaction mixture in each well was measured at 405 nm.

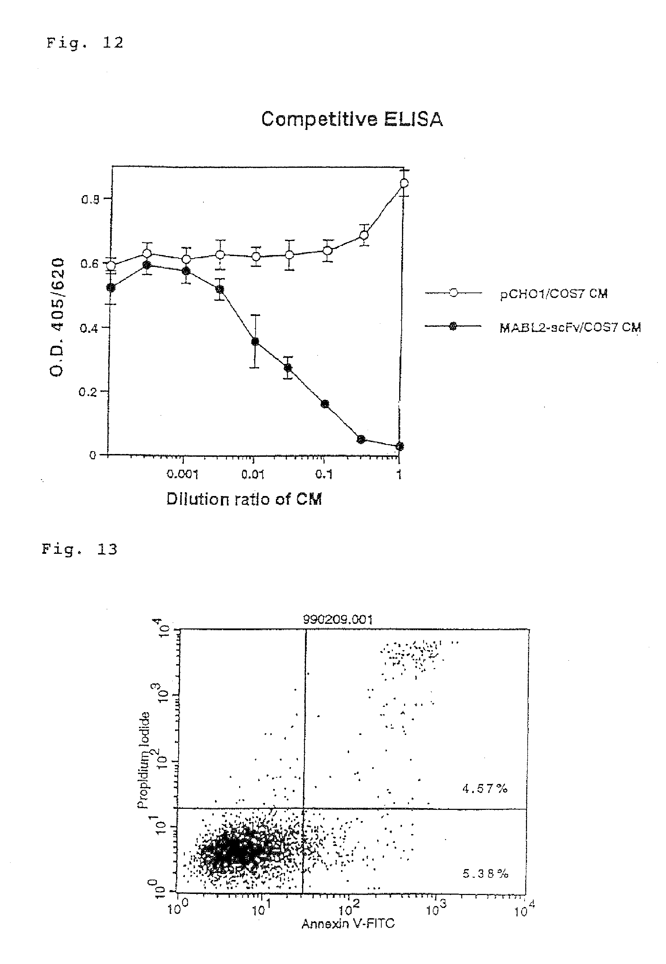

The results revealed that the reconstructed single chain Fv of antibody MABL-2 (MABL2-scFv) evidently inhibited concentration-dependently the binding of the mouse antibody MABL-2 to human IAP antigen in comparison with the culture supernatant of the PCHO1-introduced COS7 cells as a control (Fig. 12). Accordingly, it is suggested that the reconstructed single chain Fv of antibody MABL-2 has the correct structure of each of the V regions from the mouse monoclonal antibody MABL-2.

### 5.7 Apoptosis-inducing Effect in vitro

An apoptosis-inducing action of the reconstructed single chain Fv of antibody MABL-2 was examined by Annexin-V staining (Boehringer Mannheim) using the L1210 cells transfected with human IAP gene, the L1210 cells transfected with the pCOS1 vector as a control and CCRF-CEM cells.

To each 1 × 10⁵ cells of the above cells was added the culture supernatant of the COS7 cells expressing the reconstructed single chain Fv of antibody MABL-2 or the culture supernatant of COS7 cells transfected with the pCHO1 vector as a control at 50% final concentration and the mixtures were cultured for 24 hours. Then, the Annexin-V staining was performed and the fluorescence intensity was measured using the FACScan apparatus (BECTON DICKINSON).

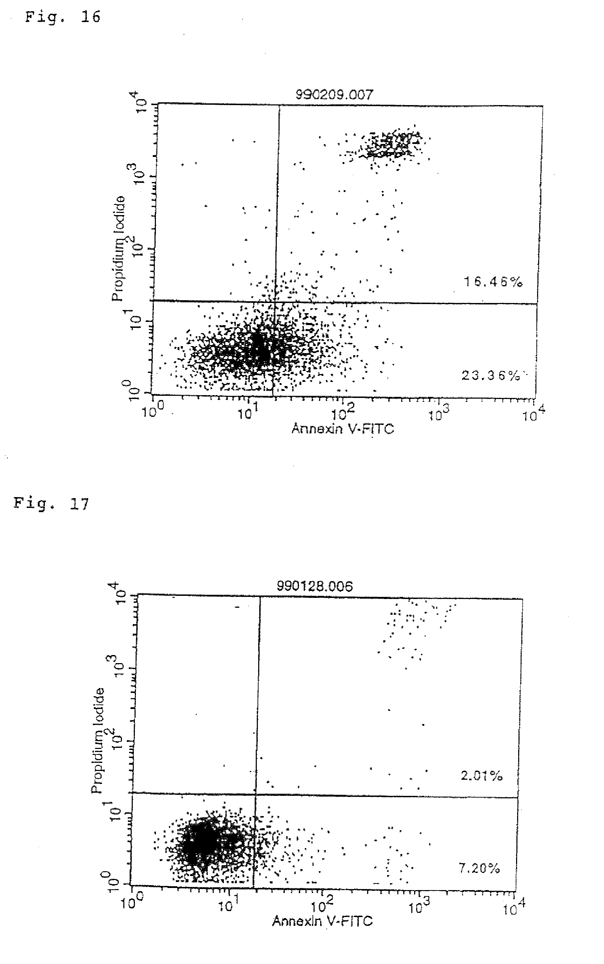

Results of the Annexin-V staining are shown in Figs. 13-18, respectively. Dots in the left-lower region represent living cells and dots in the right-lower region represent cells at the early stage of apoptosis and dots in the right-upper region represent cells at the late stage of apoptosis. The results show that the reconstructed single chain Fv of antibody MABL-2 (MABL2-scFv) remarkably induced cell death of L1210 cells specific to human IAP antigen (Figs. 13-16) and that the reconstructed single chain Fv also induced remarkable cell death of CCRF-CEM cells in comparison with the control (Figs. 17-18).

### 5.8 Expression of MABL-2 derived single chain Fv in CHO cells

CHO cells were transfected with the pCHOM2 vector to establish a CHO cell line which constantly expresses the single chain Fv (polypeptide) derived from the antibody MABL-2.

CHO cells were transformed with the pCHOM2 vector by the electroporation using the Gene Pulser apparatus (BioRad). A mixture of DNA (10 µg) and 0.7 ml of PBS with CHO cells (1 × 10⁷ cells/ml) was added to a cuvette. The mixture was treated with pulse at 1.5 kV, 25 µF of electric capacity. After the restoration for 10 minutes at a room temperature, the electroporated cells were transferred into nucleic acid free α-MEM medium (GIBCO BRL) containing 10% fetal bovine serum and cultured. The expression of desired protein in the resultant clones was confirmed by SDS-PAGE and a clone with a high expression level was selected as a cell line producing the single chain Fv derived from the antibody MABL-2. The cell line was cultured in serum-free medium CHO-S-SFM II (GIBCO BRL) containing 10 nM methotrexate (SIGMA). Then, the culture supernatant was collected, centrifuged to remove cell fragments and recovered.

### 5.9 Purification of MABL-2 derived single chain Fv produced in CHO cells

The culture supernatant of the CHO cell line expressing the single chain Fv obtained in Example 5.8 was concentrated up to twenty times using a cartridge for the artificial dialysis (PAN130SF, ASAHI MEDICALS). The concentrated solution was stored at -20°C and thawed on purification.

Purification of the single chain Fv from the culture supernatant of the CHO cells was performed using three kinds of chromatography, i.e., Blue-sepharose, a hydroxyapatite and a gel filtration.

### (1) Blue-sepharose column chromatography

The concentrated supernatant was diluted to ten times with 20 mM acetate buffer (pH 6.0) and centrifuged to remove insoluble materials (10000 × rpm, 30 minutes). The supernatant was applied onto a Blue-sepharose column (20 ml) equilibrated with the same buffer. After washing the column with the same buffer, proteins adsorbed in the column were eluted by a stepwise gradient of NaCl in the same buffer, 0.1, 0.2, 0.3, 0.5 and up to 1.0 M. The pass-through fraction and each eluted fraction were analyzed by SDS-PAGE. The fractions in which the single chain Fv were confirmed (the fractions eluted at 0.1 to 0.3M NaCl) were pooled and concentrated up to approximately 20 times using CentriPrep-10 (AMICON).

### (2) Hydroxyapatite

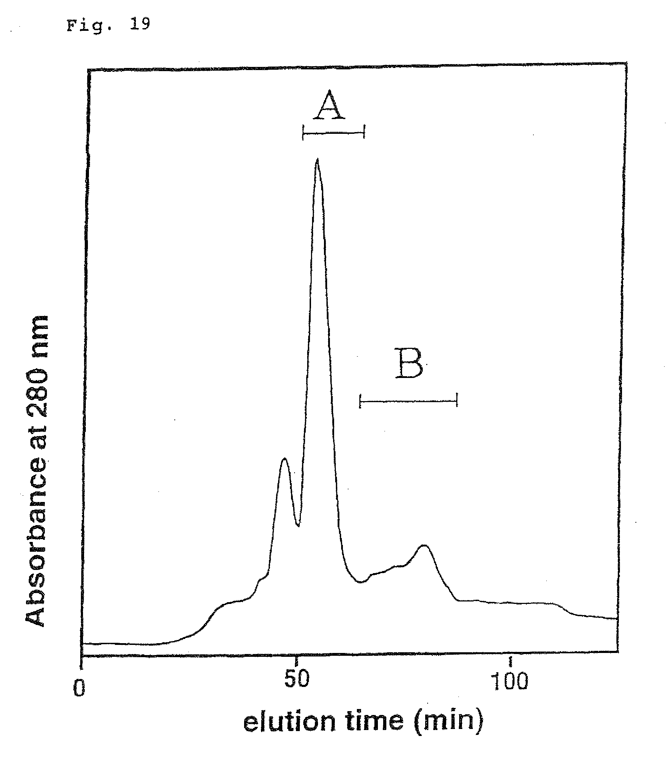

The concentrated solution obtained in (1) was diluted to 10 times with 10 mM phosphate buffer (pH 7.0) and applied onto the hydroxyapatite column (20 ml, BIORAD). The column was washed with 60 ml of 10 mM phosphate buffer (pH 7.0). Then, proteins adsorbed in the column were eluted by a linear gradient of sodium phosphate buffer up to 200 mM (see Fig. 19). The analysis of each fraction by SDS-PAGE confirmed the single chain Fv in fraction A and fraction B.

### (3) Gel filtration

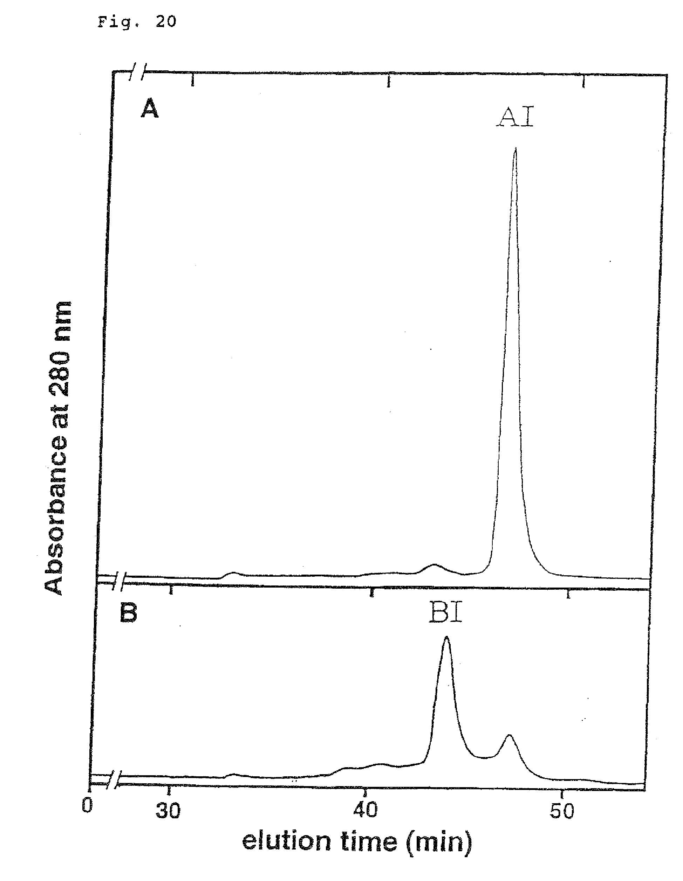

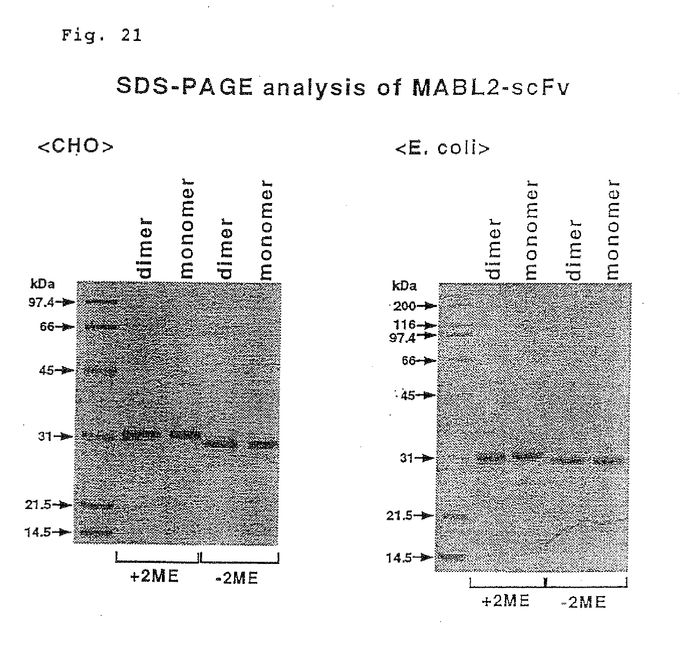

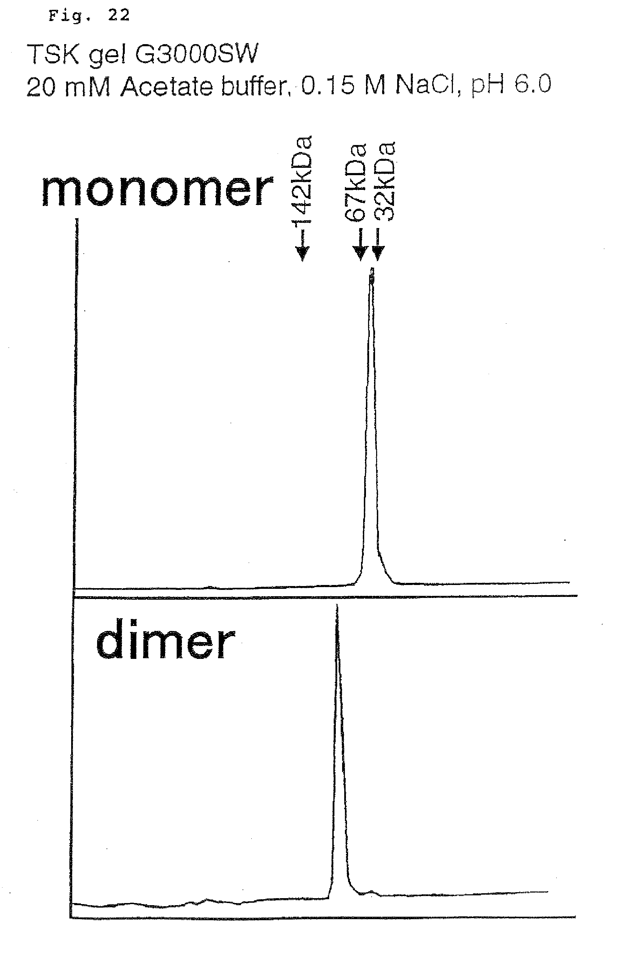

Each of fractions A and B in (2) was separately concentrated with CentriPrep-10 and applied onto TSKgel G3000SWG column (21.5 × 600 mm) equilibrated with 20 mM acetate buffer (pH 6.0) containing 0.15 M NaCl. Chromatograms are shown in Fig. 20. The analysis of the fractions by SDS-PAGE confirmed that both major peaks (AI and BI) are of desired single chain Fv. In the gel filtration analysis, the fraction A was eluted at 36 kDa of apparent molecular weight and the fraction B was eluted at 76 kDa. The purified single chain Fvs (AI, BI) were analyzed with 15% SDS polyacrylamide gel. Samples were treated in the absence or presence of a reductant and the electrophoresis was carried out in accordance with the Laemmli's method. Then the protein was stained with Coomassie Brilliant Blue. As shown in Fig. 21, both AI and BI gave a single band at 35 kDa of apparent molecular weight, regardless of the absence or presence of the reductant. From the above, it is concluded that AI is a monomer of the single chain Fv and BI is a non-covalently bound dimer of the single chain Fv. The gel filtration analysis of the fractions AI and BI with TSKgel G3000SW column (7.5 × 60 mm) revealed that a peak of the monomer is detected only in the fraction AI and a peak of the dimer is detected only in the fraction BI (Fig. 22). The dimer fraction (fraction BI) accounted for 4 period of total single chain Fvs. More than 90% of the dimer in the dimer fraction was stably preserved for more than a month at 4°C.

### 5.10 Construction of vector expressing single chain Fv derived from antibody MABL-2 in E. coli cell

The pscM2 vector was modified by the PCR method to prepare a vector effectively expressing the single chain Fv from the antibody MABL-2 in E. coli cells. The resultant DNA fragment was introduced into pSCFVT7 expression vector.

As a forward primer for PCR, Nde-VHSm02 primer shown in SEQ ID No. 27 was designed to hybridize to a DNA encoding the N-terminal of the H chain V region and to contain a start codon and NdeI restriction enzyme recognition site. As a reverse primer for PCR, VLAS primer shown in SEQ ID No. 28 was designed to hybridize to a DNA encoding the C-terminal of the L chain V region and to contain two stop codons and EcoRI restriction enzyme recognition site. The forward primer, Nde-VHSm02, comprises five point mutations in the part hybridizing to the DNA encoding the N-terminal of the H chain V region for the effective expression in E. coli.

100 µl of a PCR solution comprising 10 µl of 10 x PCR Buffer #1, 1 mM MgCl₂, 0.2 mM dNTPs, 5 units of KOD DNA polymerase (all from TOYOBO), 1 µM of each primer and 100 ng of a template DNA (pscM2) was heated at 98°C for 15 seconds, at 65°C for 2 seconds and at 74°C for 30 seconds in order. This temperature cycle was repeated 25 times.

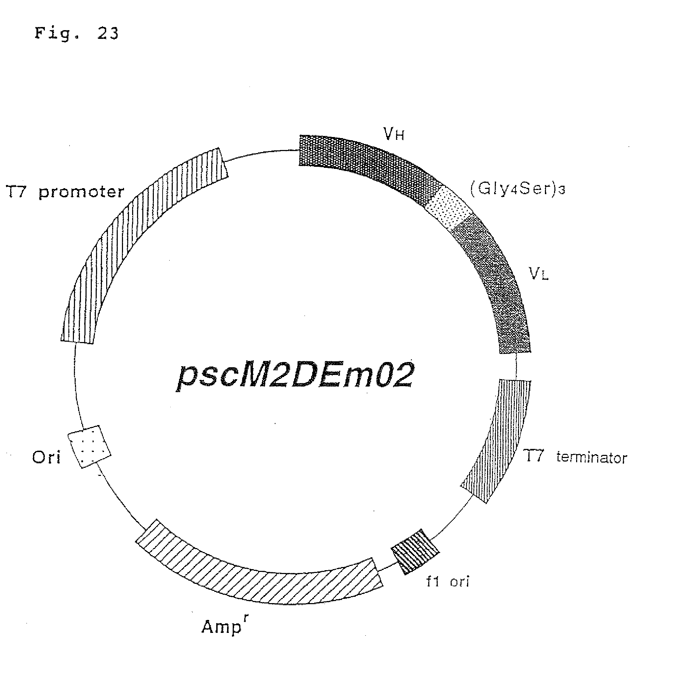

The PCR product was purified using the QIAquick PCR Purification Kit (QIAGEN) and digested by NdeI and EcoRI, and then the resulting DNA fragment was cloned into pSCFVT7 vector, from which pelB signal sequence had been eliminated by the digestion with NdeI and EcoRI. After DNA sequencing, the resulting plasmid comprising a DNA fragment with the desired DNA sequence is designated as "pscM2DEm02" (see Fig. 23). The nucleotide sequence and the amino acid sequence of the single chain Fv derived from the antibody MABL-2 contained in the plasmid pscM2DEm02 are shown in SEQ ID No. 29.

### 5.11 Expression of single chain Fv derived from antibody MABL-2 in E. coli cells

E. coli BL21(DE3)pLysS (STRATAGENE) was transformed with pscM2DEm02 vector to obtain a strain of E. coli expressing the single chain Fv derived from antibody MABL-2. The resulting clones were examined for the expression of the desired protein using SDS-PAGE, and a clone with a high expression level was selected as a strain producing the single chain Fv derived from antibody MABL-2.

### 5.12 Purification of single chain Fv derived from antibody MABL-2 produced in E.coli

A single colony of E. coli obtained by the transformation was cultured in 3 ml of LB medium at 28°C for 7 hours and then in 70 ml of LB medium at 28°C overnight. This pre-culture was transplanted to 7 L of LB medium and cultured at 28°C with stirring at 300 rpm using the Jar-fermenter. When an absorbance of the medium reached O.D.=1.5, the bacteria were induced with 1 mM IPTG and then cultured for 3 hours.

The culture medium was centrifuged (10000 × g, 10 minutes) and the precipitated bacteria were recovered. To the bacteria was added 50 mM Tris-HCl buffer (pH 8.0) containing 5 mM EDTA, 0.1 M NaCl and 1% Triton X-100 and the bacteria were disrupted by ultrasonication (out put: 4, duty cycle: 70%, 1 minute × 10 times). The suspension of disrupted bacteria was centrifuged (12000 × g, 10 minutes) to precipitate inclusion body. Isolated inclusion body was mixed with 50 mM Tris-HCl buffer (pH 8.0) containing 5 mM EDTA, 0.1 M Nacl and 4% Triton X-100, treated by ultrasonication (out put: 4, duty cycle: 50%, 30 seconds × 2 times) again and centrifuged (12000 × g, 10 minutes) to isolate the desired protein as precipitate and to remove containment proteins included in the supernatant.

The inclusion body comprising the desired protein was lysed in 50 mM Tris-HCl buffer (pH 8.0) containing 6 M Urea, 5 mM EDTA and 0.1 M NaCl and applied onto Sephacryl S-300 gel filtration column (5 × 90 cm, Amersharm Pharmacia) equilibrated with 50 mM Tris-HCl buffer (pH 8.0) containing 4M Urea, 5 mM EDTA, 0.1 M NaCl and 10 mM mercaptoethanol at a flow rate of 5 ml/minutes to remove associated single chain Fvs with high-molecular weight. The obtained fractions were analyzed with SDS-PAGE and the fractions with high purity of the protein were diluted with the buffer used in the gel filtration up to O.D₂₈₀=0.25. Then, the fractions were dialyzed three times against 50 nM Tris-HCl buffer (pH 8.0) containing 5 mM EDTA, 0.1 M NaCl, 0.5 M Arg, 2 mM glutathione in the reduced form and 0.2 mM glutathione in the oxidized form in order for the protein to be refolded. Further, the fraction was dialyzed three times against 20 mM acetate buffer (pH 6.0) containing 0.15 M NaCl to exchange the buffer.

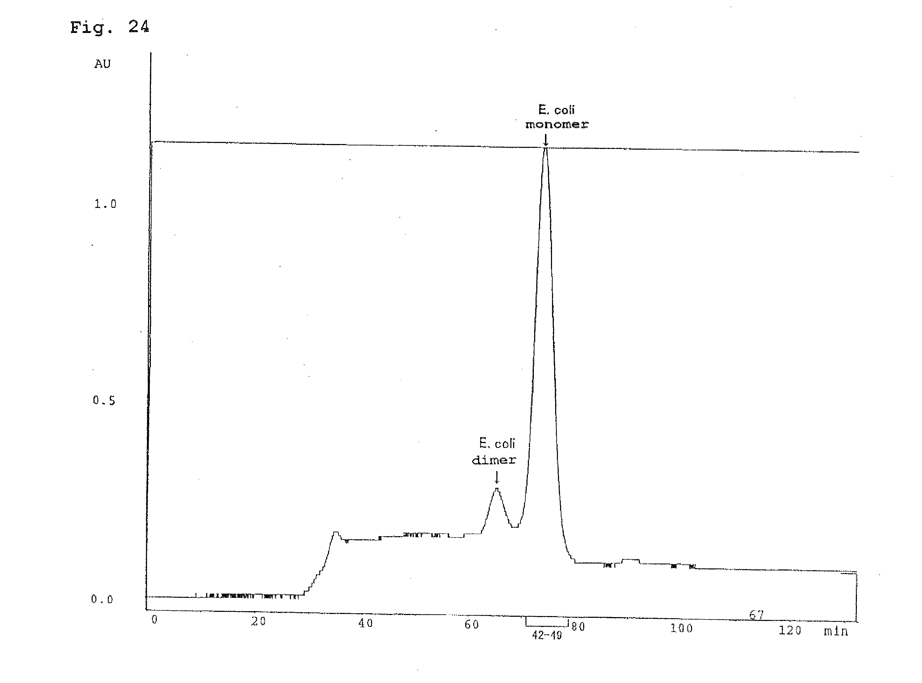

The dialysate product was applied onto Superdex 200 pg gel filtration column (2.6 × 60 cm, Amersharm Pharmacia) equilibrated with 20 mM acetate buffer (pH 6.0) containing 0.15 M NaCl to remove a small amount of high molecular weight protein which was intermolecularly crosslinked by S-S bonds. As shown in Fig. 24, two peaks, major and sub peaks, were eluted after broad peaks which are expectedly attributed to an aggregate with a high molecular weight. The analysis by SDS-PAGE (see Fig. 21) and the elution positions of the two peaks in the gel filtration analysis suggest that the major peak is of the monomer of the single chain Fv and the sub peak is of the non-covalently bound dimer of the single chain Fv. The non-covalently bound dimer accounted for 4 percent of total single chain Fvs.

### 5.13 Apoptosis-inducing activity in vitro of single chain Fv derived from antibody MABL-2

An apoptosis-inducing action of the single chain Fv from antibody MABL-2 (MABL2-scFv) produced by the CHO cells and E. coli was examined according to two protocols by Annexin-V staining (Boehringer Mannheim) using the L1210 cells (hIAP/L1210) into which human IAP gene had been introduced.

In the first protocol sample antibodies at the final concentration of 3 µg/ml were added to 5 × 10⁴ cells of hIAP/L1210 cell line and cultured for 24 hours. Sample antibodies, i.e., the monomer and the dimer of the single chain Fv of MABL-2 from the CHO cells obtained in Example 5.9, the monomer and the dimer of the single chain Fv of MABL-2 from E. coli obtained in Example 5.12, and the mouse IgG antibody as a control were analyzed. After culturing, the Annexin-V staining was carried out and the fluorescence intensity thereof was measured using the FACScan apparatus (BECTON DICKINSON).

In the second protocol sample antibodies at the final concentration of 3 µg/ml were added to 5 × 10⁴ cells of hIAP/L1210 cell line, cultured for 2 hours and mixed with anti-FLAG antibody (SIGMA) at the final concentration of 15 µg/ml and further cultured for 22 hours. Sample antibodies of the monomer of the single chain Fv of MABL-2 from the CHO cells obtained in Example 5.9 and the mouse IgG antibody as a control were analyzed. After culturing, the Annexin-V staining was carried out and the fluorescence intensity thereof was measured using the FACScan apparatus.

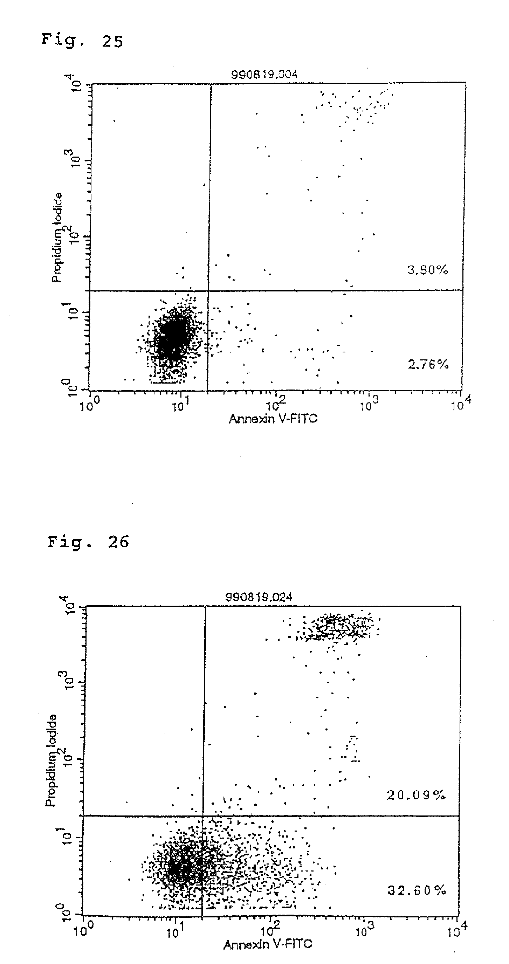

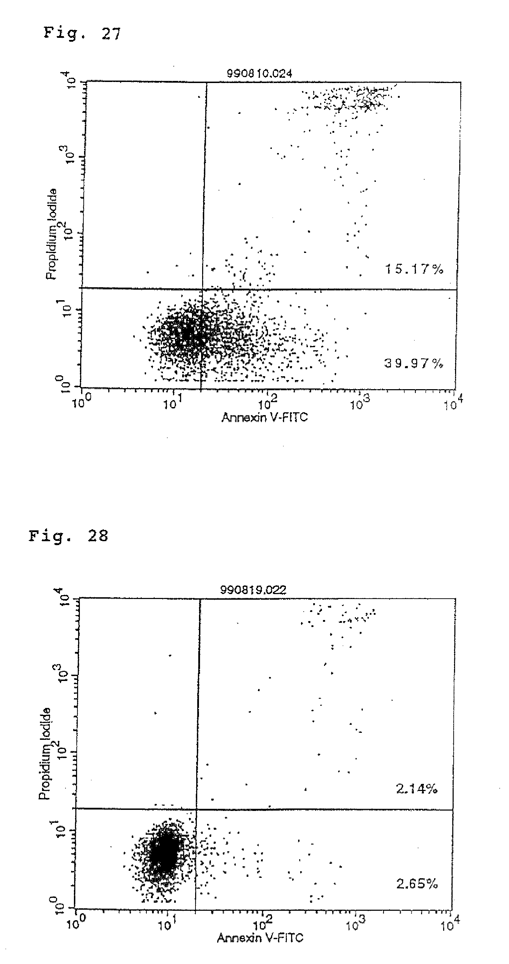

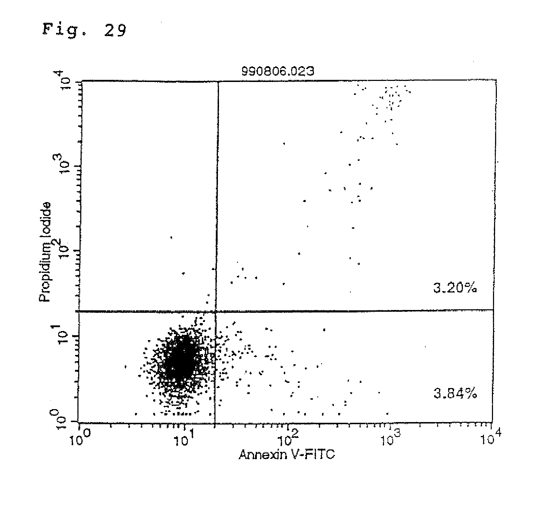

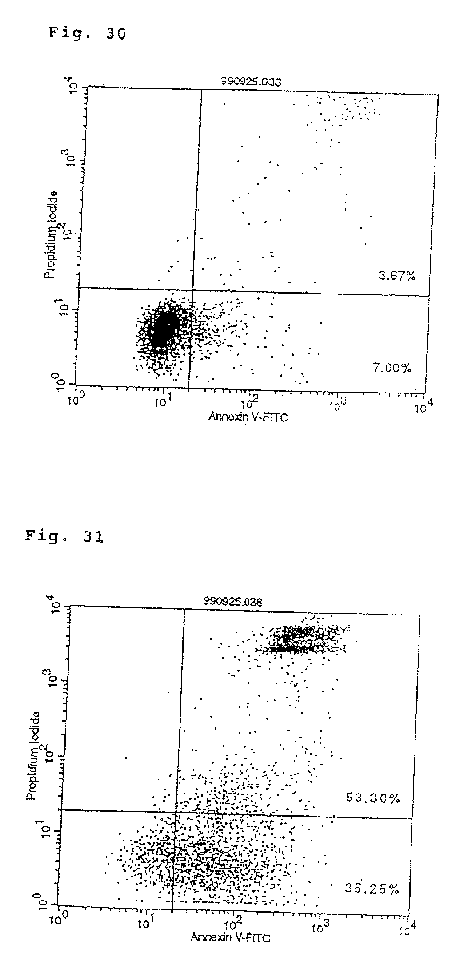

Results of the analysis by the Annexin-V staining are shown in Figs. 25-31. The results show that the dimers of the single chain Fv polypeptide of MABL-2 produced in the CHO cells and E. coli remarkably induced cell death (Figs. 26, 27) in comparison with the control (Fig. 25), while no apoptosis-inducing action was observed in the monomers of the single chain Fv polypeptide of MABL-2 produced in the CHO cells and E. coli (Figs. 28, 29). When anti-FLAG antibody was used together, the monomer of the single chain Fv polypeptide derived from antibody MABL-2 produced in the CHO cells induced remarkably cell death (Fig. 31) in comparison with the control (Fig. 30).

### 5.14 Antitumor effect of the monomer and the dimer of scFv/CHO polypeptide with a model mouse of human myeloma

### (1) Quantitative measurement of human IgG in mouse serum

Measurement of human IgG (M protein) produced by human myeloma cell and contained in mouse serum was carried out by the following ELISA. 100 µL of goat anti-human IgG antibody (BIOSOURCE, Lot#7902) diluted to 1 µg/mL with 0.1% bicarbonate buffer (pH 9.6) was added to each well on 96 wells plate (Nunc) and incubated at 4°C overnight so that the antibody was immobilized. After blocking, 100 µL of the stepwisely diluted mouse serum or human IgG (CAPPEL, Lot#00915) as a standard was added to each well and incubated for 2 hours at a room temperature. After washing, 100 µL of alkaline phasphatase-labeled anti-human IgG antibody (BIOSOURCE, Lot#6202) which had been diluted to 5000 times was added, and incubation was carried out for 1 hour at a room temperature. After washing, a substrate solution was added. After incubation, absorbance at 405 nm was measured using the MICROPLATE READER Model 3550 (BioRad). The concentration of human IgG in the mouse serum was calculated based on the calibration curve obtained from the absorbance values of human IgG as the standard.

### (2) Preparation of antibodies for administration

The monomer and the dimer of the scFv/CHO polypeptide were respectively diluted to 0.4 mg/mL or 0.25 mg/mL with sterile filtered PBS(-) on the day of administration to prepare samples for the administration.

### (3) Preparation of a mouse model of human myeloma

A mouse model of human myeloma was prepared as follows. KPMM2 cells passaged in vivo (JP-Appl. 7-236475) by SCID mouse (Japan Clare) were suspended in RPMI1640 medium (GIBCO-BRL) containing 10% fetal bovine serum (GIBCO-BRL) and adjusted to 3 × 10⁷ cells/mL. 200 µL of the KPMM2 cell suspension (6 × 10⁶ cells/mouse) was transplanted to the SCID mouse (male, 6 week-old) via caudal vein thereof, which had been subcutaneously injected with the asialo GM1 antibody (WAKO JUNYAKU, 1 vial dissolved in 5 mL) a day before the transplantation.

### (4) Administration of antibodies

The samples of the antibodies prepared in (2), the monomer (250 µL) and the dimer (400 µL), were administered to the model mice of human myeloma prepared in (3) via caudal vein thereof. The administration was started from three days after the transplantation of KPMM2 cells and was carried out twice a day for three days. As a control, 200 µL of sterile filtered PBS(-) was likewise administered twice a day for three days via caudal vein. Each group consisted of seven mice.

### (5) Evaluation of antitumor effect of the monomer and the dimer of scFv/CHO polypeptide with the model mouse of human myeloma

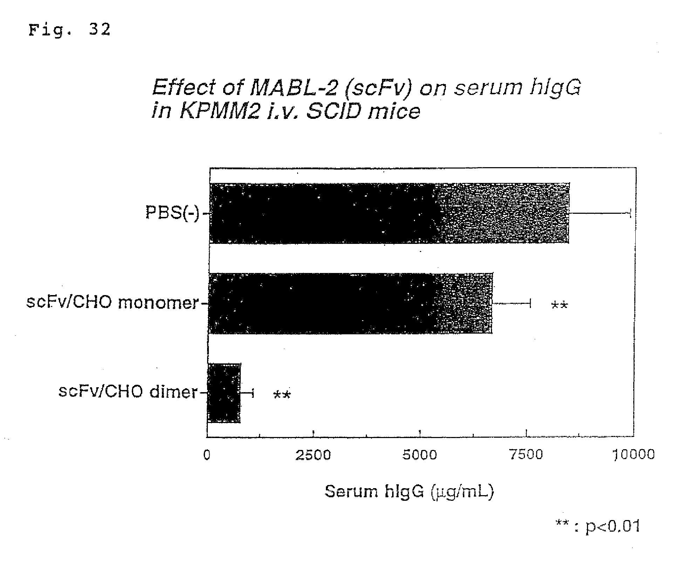

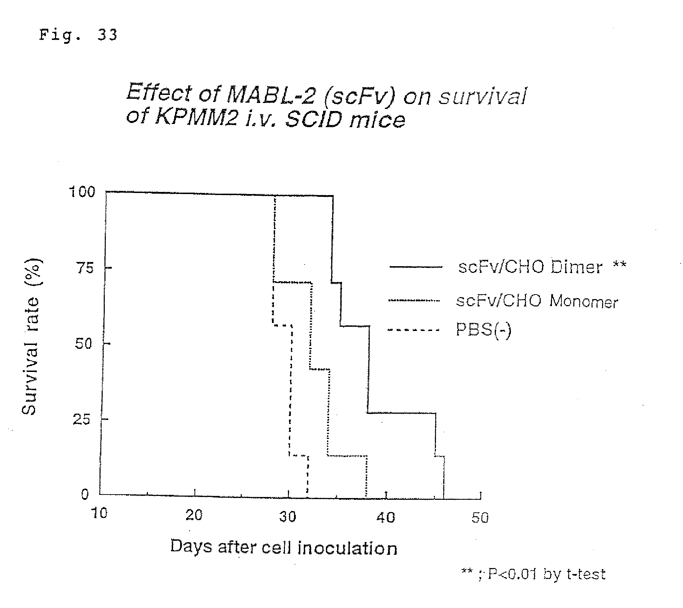

The antitumor effect of the monomer and the dimer of scFv/CHO polypeptide with the model mice of human myeloma was evaluated in terms of the change of human IgG (M protein) concentration in the mouse serum and survival time of the mice. The change of human IgG concentration was determined by measuring it in the mouse serum collected at 24 days after the transplantation of KPMM2 cells by ELISA described in the above (1). The amount of serum human IgG (M protein) in the serum of the PBS(-)-administered group (control) increased to about 8500 µg/mL, whereas the amount of human IgG of the scFv/CHO dimer-administered group was remarkably low, that is, as low as one-tenth or less than that of the control group. Thus, the results show that the dimer of scFv/CHO strongly inhibits the growth of the KPMM2 cells (Fig. 32). As shown in Fig. 33, a remarkable elongation of the survival time was observed in the scFv/CHO dimer-administered group in comparison with the PBS(-)-administered group.

From the above, it is confirmed that the dimer of scFv/CHO has an antitumor effect for the human myeloma model mice. It is considered that the antitumor effect of the dimer of scFv/CHO, the modified antibody of the invention, results from the apoptosis-inducing action of the modified antibody.

### 5.15 Hemagglutination Test

Hemagglutination test and determination of hemagglutination were carried out in accordance with "Immuno-Biochemical Investigation", Zoku-Seikagaku Jikken Koza, edited by the Biochemical Society of Japan, published by Tokyo Kagaku Dojin.

Blood was taken from a healthy donor using heparin-treated syringes and washed with PBS(-) three times, and then erythrocyte suspension with a final concentration of 2% in PBS(-) was prepared. Test samples were the antibody MABL-2, the monomer and the dimer of the single chain Fv polypeptide produced by the CHO cells, and the monomer and the dimer of the single chain Fv polypeptide produced by E. coli, and the control was mouse IgG (ZYMED). For the investigation of the hemagglutination effect, round bottom 96-well plates available from Falcon were used. 50 µL per well of the aforementioned antibody samples and 50 µL of the 2% erythrocyte suspension were added and mixed in the well. After incubation for 2 hours at 37°C, the reaction mixtures were stored at 4°C overnight and the hemagglutination thereof was determined. As a control, 50 µL per well of PBS(-) was used and the hemagglutination test was carried out in the same manner. The mouse IgG and antibody MABL-2 were employed at 0.01, 0.1, 1.0, 10.0 or 100.0 µg/mL of the final concentration of the antibodies. The single chain Fvs were employed at 0.004, 0.04, 0.4, 4.0, 40.0 or 80.0 µg/mL of the final concentration and further at 160.0 µg/mL only in the case of the dimer of the polypeptide produced by E. coli. Results are shown in the Table 2. In the case of antibody MABL-2, the hemagglutination was observed at a concentration of more than 0.1 µg/mL, whereas no hemagglutination was observed for both the monomer and the dimer of the single chain Fv.

**Table 2 Hemagglutination Test**

| | Control | 0.01 | 0.1 | 1 | 10 | 100 | µg/mL | | |
|---|---|---|---|---|---|---|---|---|---|
| mlgG | - | - | - | - | - | - | | | |
| MABL-2 (intact) | - | - | + | +++ | +++ | ++ | | | |

| | Control | 0.004 | 0.04 | 0.4 | 4 | 40 | 80 | µg/mL | |
|---|---|---|---|---|---|---|---|---|---|
| scFv/CHO | | | | | | | | | |
| monomer | - | - | - | - | - | - | - | | |
| scFv/CHO | | | | | | | | | |
| dimer | - | - | - | - | - | - | - | | |

| | Control | 0.004 | 0.04 | 0.4 | 4 | 40 | 80 | 160 | µg/mL |
|---|---|---|---|---|---|---|---|---|---|
| scFv/E.coli | | | | | | | | | |
| monomer | - | - | - | - | - | - | - | | |
| scFv/E.coli | | | | | | | | | |
| dimer | - | - | - | - | - | - | - | - | |

### Example 6 Modified antibody sc(Fv)₂ comprising two H chain V regions and two L chain V regions and antibody MABL-2 scFvs having linkers with different length

### 6.1 Construction of plasmid expressing antibody MABL-2 sc(Fv)₂

For the preparation of a plasmid expressing the modified antibody [sc(Fv)₂] which comprises two H chain V regions and two L chain V regions derived from the antibody MABL-2, the aforementioned pCHOM2, which comprises the DNA encoding scfv derived from the MABL-2 described above, was modified by the PCR method as mentioned below and the resulting DNA fragment was introduced into pCHOM2.

Primers employed for the PCR are EF1 primer (SEQ ID NO: 30) as a sense primer, which is designed to hybridize to a DNA encoding EF1α, and an antisense primer (SEQ ID NO: 19), which is designed to hybridize to the DNA encoding C-terminal of the L chain V region and to contain a DNA sequence coding for a linker regions, and VLLAS primer containing SalI restriction enzyme recognition site (SEQ ID NO 31).

100 µl of the PCR solution comprises 10 µl of 10 × PCR Buffer #1, 1 mM MgCl₂, 0.2 mM dNTPs (dATP, dGTP, dCTP and dTTP), 5 units of KOD DNA polymerase (Toyobo, Inc.), 1 µM of each primer and 100 ng of the template DNA (pCHOM2). The PCR solution was heated at 94°C for 30 seconds, at 50°C for 30 seconds and at 74°C for 1 minute in order. This temperature cycle was repeated 30 times.

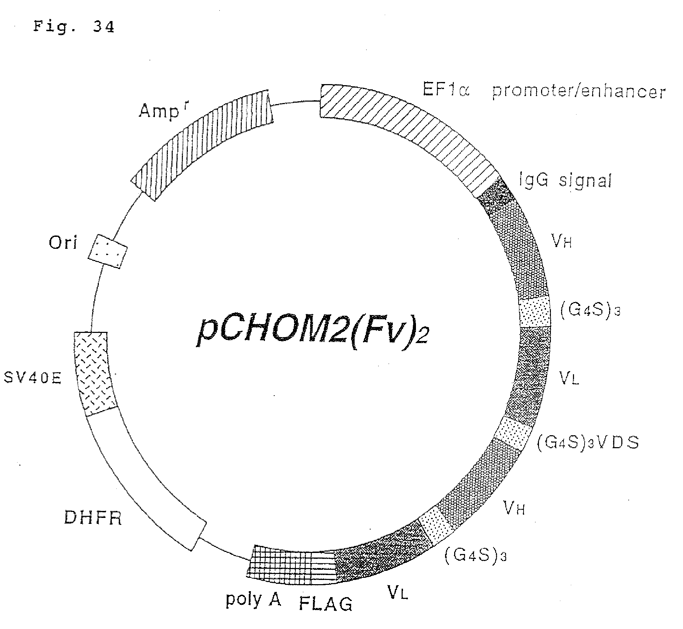

The PCR product was purified using the QIAquick PCR Purification Kit (QIAGEN) and digested by SalI. The resultant DNA fragment was cloned into pBluescript KS⁺ vector (Toyobo, Inc.). After DNA sequencing, a plasmid comprising the desired DNA sequence was digested by SalI and the obtained DNA fragment was connected using Rapid DNA Ligation Kit(BOEHRINGER MANNHEIM) to pCHOM2 digested by SalI. After DNA sequencing, a plasmid comprising the desired DNA sequence is designated as "pCHOM2 (Fv)₂" (see Fig. 34). The Nucleotide sequence and the amino acid sequence of the antibody MABL-2 sc(Fv)₂ region contained in the plasmid pCHOM2 (F_{V})₂ are shown in SEQ ID No. 32.

### 6.2 Preparation of Plasmid expressing antibody MABL-2 scFvs having linkers with various length

The scFv containing linkers with different length and the V regions which are designed in the order of [H chain] - [L chain] (hereinafter "HL") or [L chain] - [H chain] (hereinafter "LH") were prepared using, as a template, cDNAs encoding the H chain and the L chain derived from the MABL-2 as mentioned below.

To construct HL type scFv the PCR procedure was carried out using pCHOM2 (Fv)₂ as a template. In the PCR step, a pair of CFHL-F1 primer (SEW ID NO: 33) and CFHL-R2 primer (SEQ ID NO: 34) or a pair of CFHL-F2 primer (SEQ ID NO: 35) and CFHL-R1 primer (SEQ ID NO: 36) and KOD polymerase were employed. The PCR procedure was carried out by repeating 30 times the temperature cycle consisting of 94°C for 30 seconds, 60°C for 30 seconds and 72°C for 1 minute in order to produce a cDNA for the H chain containing a leader sequence at 5'-end or a cDNA for the L chain containing FLAG sequence at 3'-end thereof. The resultant cDNAs for the H chain and the L chain were mixed and PCR was carried out by repeating 5 times the temperature cycle consisting of 94°C for 30 seconds, 60°C for 30 seconds and 72°C for 1 minute in order using the mixture as templates and the KOD polymerase. To the reaction mixture were added CFHL-F1 and CFHL-R1 primers and then the PCR reaction was performed by repeating 30 times of the aforementioned temperature cycle to produce a cDNA for HL-0 type without a linker.

To construct LH type scFv, the PCR reaction was carried out using, as a template, pGEM-M2L and pGEM-M2H which contain cDNAs encoding the L chain V region and the H chain V region from the antibody MABL-2, respectively (see JP- Appl. 11-63557). A pair of T7 primer (SEQ ID NO: 37) and CFLH-R2 primer (SEQ ID NO: 38) or a pair of CFLH-F2 primer (SEQ ID NO: 39) and CFLH-R1 (SEQ ID NO: 40) and the KOD polymerase (Toyobo Inc.) were employed. The PCR reaction was performed by repeating 30 times the temperature cycle consisting of 94°C for 30 seconds, 60°C for 30 seconds and 72°C for 1 minute in sequential order to produce a cDNA of an L chain containing a leader sequence at 5'-end or a cDNA of an H chain containing FLAG sequence at 3'-end thereof. The resultant cDNAs of the L chain and the H chain were mixed and PCR was carried out using this mixture as templates and the KOD polymerase by repeating 5 times the temperature cycle consisting of 94°C for 30 seconds, 60°C for 30 seconds and 72°C for 1 minute in order. To the reaction mixture were added T7 and CFLH-R1 primers and the reaction was performed by repeating 30 times of the aforementioned temperature cycle. The reaction product was used as a template and PCR was carried out using a pair of CFLH-F4 primer SEQ ID NO: 41) and CFLH-R1 primer by repeating 30 times the temperature cycle consisting of 94°C for 30 seconds, 60°C for 30 seconds and 72°C for 1 minute in order to produce a cDNA of LH-0 type without a linker.

The resultant cDNAs of LH-0 and HL-0 types were digested by EcoRI and BamHI restriction enzymes (Takara Shuzo) and the digested cDNAs were introduced into an expression plasmid INPEP4 for mammalian cells using Ligation High (Toyobo Inc.), respectively. Competent E coli JM109 (Nippon Gene) was transformed with each plasmid and the desired plasmids were isolated from the transformed E. coli using QIAGEN Plasmid Maxi Kit (QUIAGEN). Thus plasmids pCF2LH-0 and pCF2HL-0 were prepared.

To construct the expression plasmids of HL type containing linkers with different size, pCF2HL-0, as a template, and CFHL-X3 (SEQ ID NO: 42), CFHL-X4 (SEQ ID NO: 43), CFHL-X5 (SEQ ID NO: 44), CFHL-X6 (SEQ ID NO: 45) or CFHL-X7 (SEQ ID NO: 46), as a sense primer, and BGH-1 (SEQ ID NO: 47) primer, as an antisense primer, which is complementary with the vector sequence were employed. PCR reaction was carried out using the KOD polymerase by repeating 30 times the temperature cycle consisting of 94°C for 30 seconds, 60°C for 30 seconds and 72°C for 1 minute in order and the reaction products were digested by restriction enzymes XhoI and BamHI (Takara Shuzo). The digested fragments were introduced between XhoI and BamHI sites in the pCF2HL-0 using Ligation High (Toyobo Inc.), respectively. Competent E. coli JM109 was transformed with each plasmid and the desired plasmid were isolated from the transformed E. coli by using Qiagen Plasmid Maxi kit. Thus expression plasmids pCF2HL-3, pCF2HL-4, pCF2HL-5, pCF2HL-6 and pCF2HL-7 were prepared.

To construct expression plasmid for the transient expression in COS7 cells the plasmids pCF2HL-0, pCF2HL-3, pCF2HL-4, pCF2HL-5, pCF2HL-6 and pCF2HL-7 were digested by restriction enzymes EcoRI and BamHI (Takara Shuzo) and the resultant fragments of approximately 800 bp were purified with agarose gel electrophoresis. The obtained fragments were introduced between EcoRI and BamHI sites in an expression plasmid pCOS1 for the expression in mammalian cells by using Ligation High (Toyobo Inc.), respectively. Competent E. coli DH5α (Toyobo Inc.) was transformed with each plasmid and the desired plasmids were isolated from the transformed E. coli using Qiagen Plasmid Maxi kit. Thus the expression plasmids CF2HL-0/pCOS1, CF2HL-3/pCOS1, CF2HL-4/pCOS1, CF2HL-5/pCOS1, CF2HL-6/pCOS1 and CF2HL-7/pCOS1 were prepared.

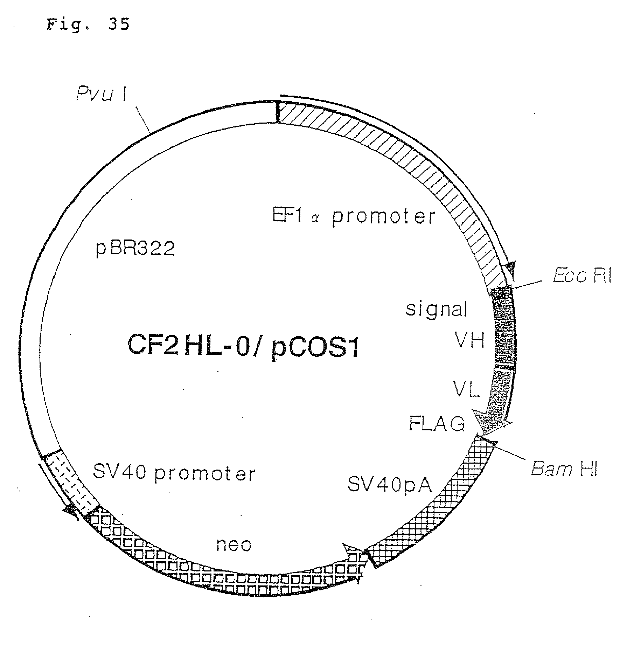

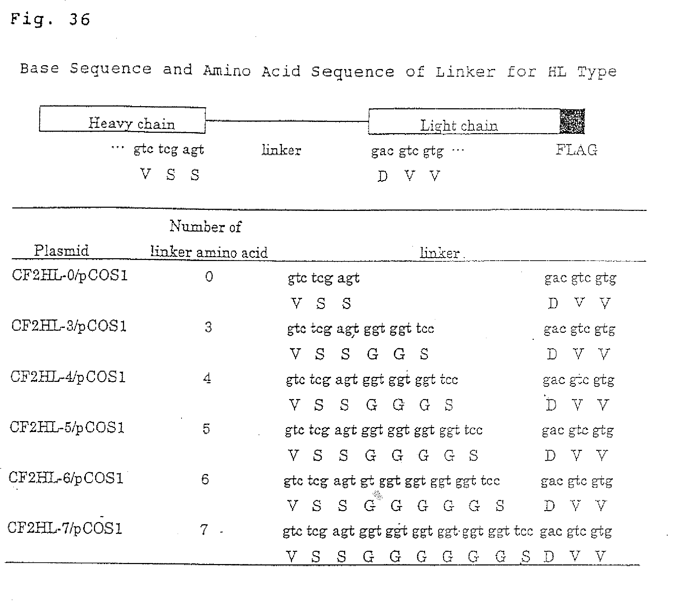

As a typical example of these plasmids, the construction of the plasmid CF2HL-0/pCOS1 is illustrated in Fig. 35 and the nucleotide sequence and the amino acid sequence of MABL2-scFv <HL-0> contained in the plasmid are shown in SEQ ID No. 48. Nucleotide sequences and amino acid sequences of the linker regions in these plasmids are also shown in Fig. 36.

To construct the expression plasmids of LH type containing linkers with different size, pCF2LH-0, as a template, and CFLH-X3 (SEQ ID NO: 49), CFLH-X4 (SEQ ID NO: 50), CFLH-X5 (SEQ ID NO: 51), CFLH-X6 (SEQ ID NO: 52) or CFLH-X7 (SEQ ID NO: 53), as a sense primer, and BGH-1 primer, as an antisense primer, which is complementary with the vector sequence were employed. PCR reaction was carried out using the KOD polymerase by repeating 30 times the temperature cycle consisting of 94°C for 30 seconds, 60°C for 30 seconds and 72°C for 1 minute in order and the reaction products were digested by restriction enzymes XhoI and BamHI. The digested fragments were introduced into the pCF2LH-0 between XhoI and BamHI sites using Ligation High, respectively. Competent E. coli DH5α (Toyobo Inc.) was transformed with each plasmid and the desired plasmids were isolated from the transformed E. coli using Qiagen Plasmid Maxi kit. Thus expression plasmids pCF2LH-3, pCF2LH-4, pCF2LH-5, pCF2LH-6 and pCF2LH-7 were prepared.

To construct expression plasmid for the transient expression in COS7 cells the plasmids pCF2LH-0, pCF2LH-3, pCF2LH-4, pCF2LH-5, pCF2LH-6 and pCF2LH-7 were digested by restriction enzymes EcoRI and BamHI (Takara Shuzo) and the resultant fragments of approximately 800 bp were purified with agarose gel electrophoresis. The obtained fragments were introduced between XhoI and BamHI sites in an expression plasmid pCOS1 for the expression in mammalian cells by using the Ligation High, respectively. Competent E. coli DH5α (Toyobo Inc.) was transformed with each plasmid and the desired plasmids were isolated from the transformed E. coli using the Qiagen Plasmid Maxi kit. Consequently, the expression plasmids CF2LH-0/pCOS1, CF2LH-3/pCOS1, CF2LH-4/pCOS1, CF2LH-5/pCCS1, CF2LH-6/pCOS1 and CF2LH-7/pCOS1 were prepared.

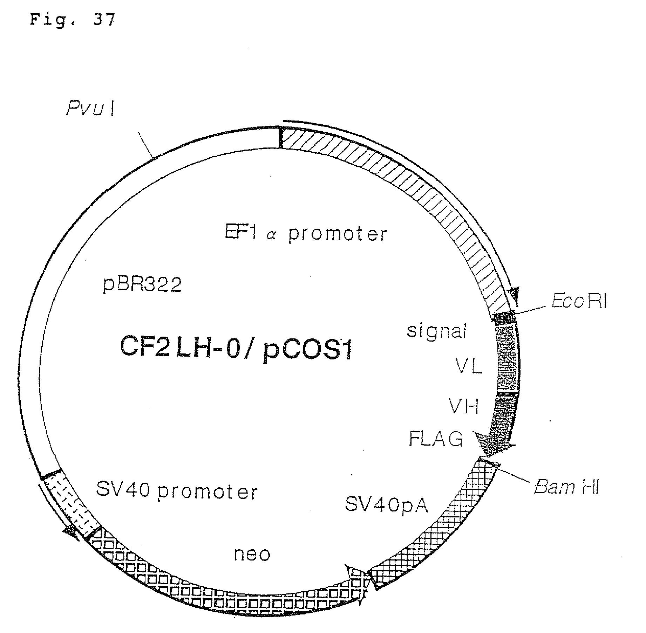

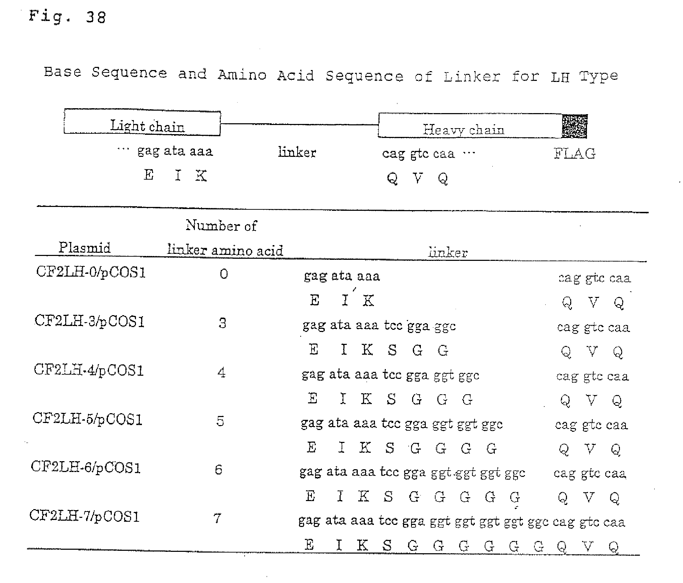

As a typical example of these plasmids, the construction of the plasmid CF2LH-0/pCOS1 is illustrated in Fig. 37 and the nucleotide sequence and the amino acid sequence of MABL2-scFv <LH-0> contained in the plasmid are shown in SEQ ID No. 54. Nucleotide sequences and amino acid sequences of the linker regions in these plasmids are also shown in Fig. 38.

### 6.3 Expression of scFvs and sc (Fv)₂ in COS7 cells

### (1) Preparation of culture supernatant using serum-containing culture medium

The HL type and LH type of scFvs and sc(Fv)₂ were transiently expressed in COS7 cells (JCRB9127, Japan Health Sciences Foundation). COS7 cells were subcultured in DMEM media (GIBCO BRL) containing 10% fetal bovine serum (HyClone) at 37°C in carbon dioxide atmosphere incubator. The COS7 cells were transfected with CF2HL-0, 3 ∼ 7/pCOS1, or CF2LH-0, 3 ∼ 7/pCOS1 prepared in Example 6.2 or pCHOM2(Fv)₂ vectors by electroporation using the Gene Pulser apparatus (BioRad). The DNA (10 µg) and 0.25 ml of 2 × 10⁷ cells/ml in DMEM culture medium containing 10% FBS and 5 mM BES (SIGMA) were added to a cuvette. After standing for 10 minutes the mixtures were treated with pulse at 0.17kV, 950µF of electric capacity. After the restoration for 10 minutes at room temperature, the electroporated cells were transferred into the DMEM culture medium (10%FBS) in 75 cm³ flask. After culturing for 72 hours, the culture supernatant was collected and centrifuged to remove cell fragments. The culture supernatant was subjected to the filtration using 0.22 µm bottle top filter (FALCON) to obtain the culture supernatant (hereinafter "CM").

### (2) Preparation of culture supernatant using serum-free culture medium

Cells transfected in the same manner as (1) were transferred to the DMEM medium (10% FBS) in 75 cm³ flask and cultured overnight. After the culture, the supernatant was discarded and the cells were washed with PBS and then added to CHO-S-SFM II medium (GIBCO BRL). After culturing for 72 hours, the culture supernatant was collected, centrifuged to remove cell fragments and filtered using 0.22 µm bottle top filter (FALCON) to obtain CM.

### 6.4 Detection of scFvs and sc (Fv)₂ in CM of COS7

The various MABL2-scFVs and sc(Fv)₂ in CM of COS7 prepared in the aforementioned Example 6.3 (2) were detected by Western Blotting method.

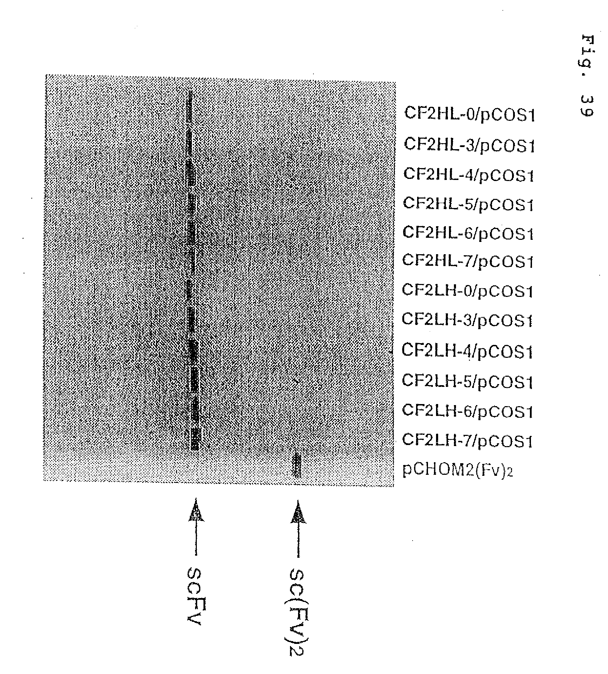

Each CM of COS7 was subjected to SDS-PAGE electrophoresis and transferred to REINFORCED NC membrane (Schleicher & Schuell). The membrane was blocked with 5% skim milk (Morinaga Nyu-gyo) and washed with TBS. Then an anti-FLAG antibody (SIGMA) was added thereto. The membrane was incubated at room temperature and washed. A peroxidase labeled mouse IgG antibody (Jackson Immuno Research) was added. After incubating and washing at room temperature, the substrate solution (Kirkegaard Perry Laboratories) was added to develop color (Fig. 39).

### 6.5 Flow cytometry

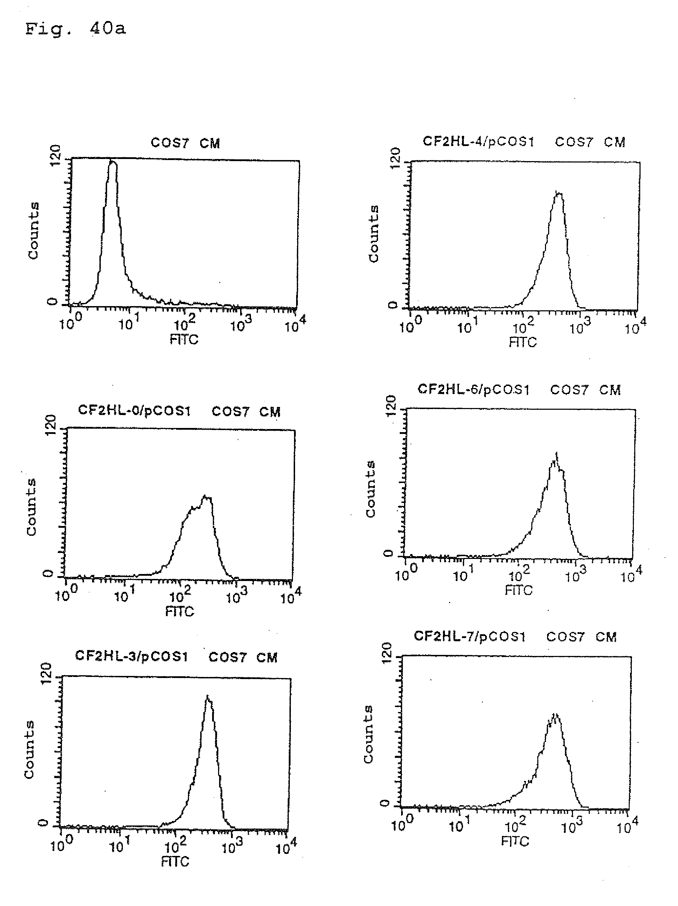

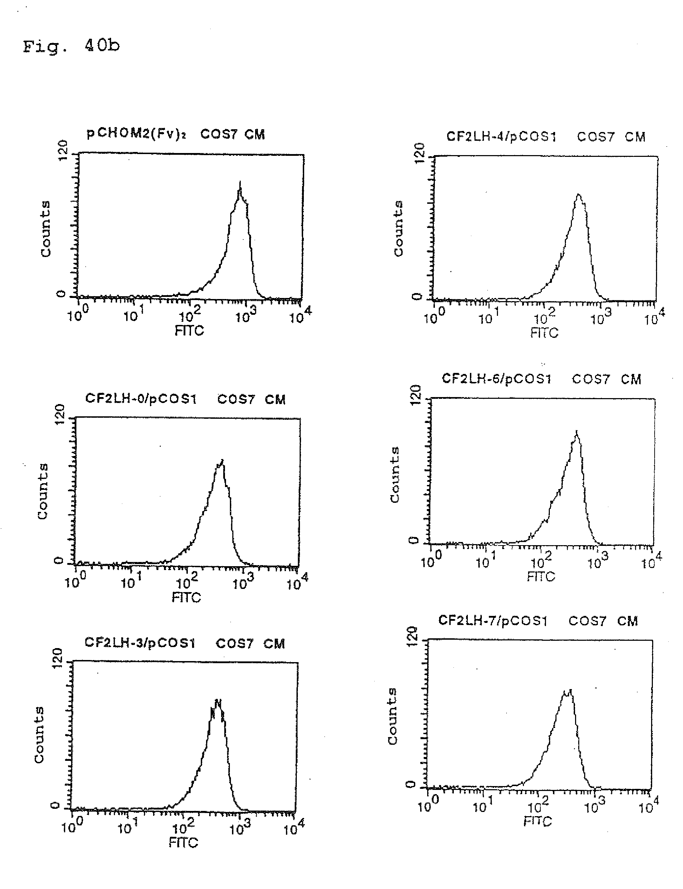

Flow cytometry was performed using the culture supernatants of COS7 cells prepared in Example 6.3 (1) to measure the binding of the MABL2-scFVs and sc(Fv)₂ to human Integrin Associated Protein (IAP) antigen. The culture supernatants to be tested or a culture supernatant of COS7 cells as a control was added to 2 × 10⁵ cells of the mouse leukemia cell line L1210 expressing human IAP. After incubating on ice and washing, 10 µg/mL of the mouse anti-FLAG antibody (SIGMA) was added and then the cells were incubated and washed. Then, the FITC labeled anti-mouse IgG antibody (BECTON DICKINSON) was added thereto and the cells were incubated and washed again. The fluorescence intensity was measured using the FACScan apparatus (BECTON DICKINSON). The results of the flow cytometry show that the MABL2-scFvs having linkers with different length and the sc(Fv)₂ in the culture supernatants of COS7 have high affinity to human IAP (see Figs. 40a and 40b).

### 6.6 Apoptosis-inducing Effect in vitro

An apoptosis-inducing action of the culture supernatants of COS7 prepared in Example 6.3 (1) was examined by Annexin-V staining (Boehringer Mannheim) using the L1210 cells transfected with human IAP gene (hIAP/L1210).

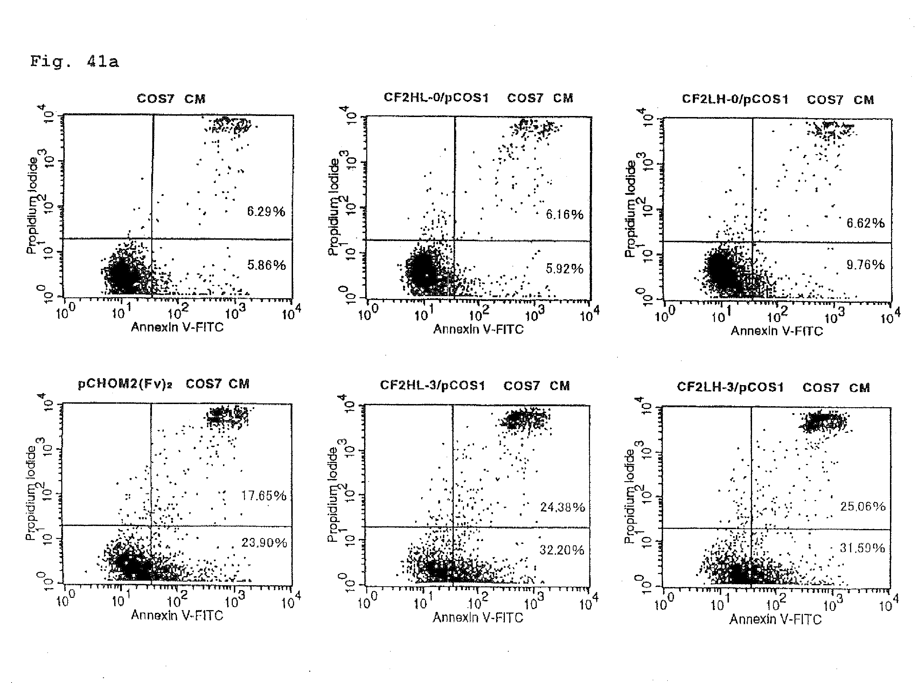

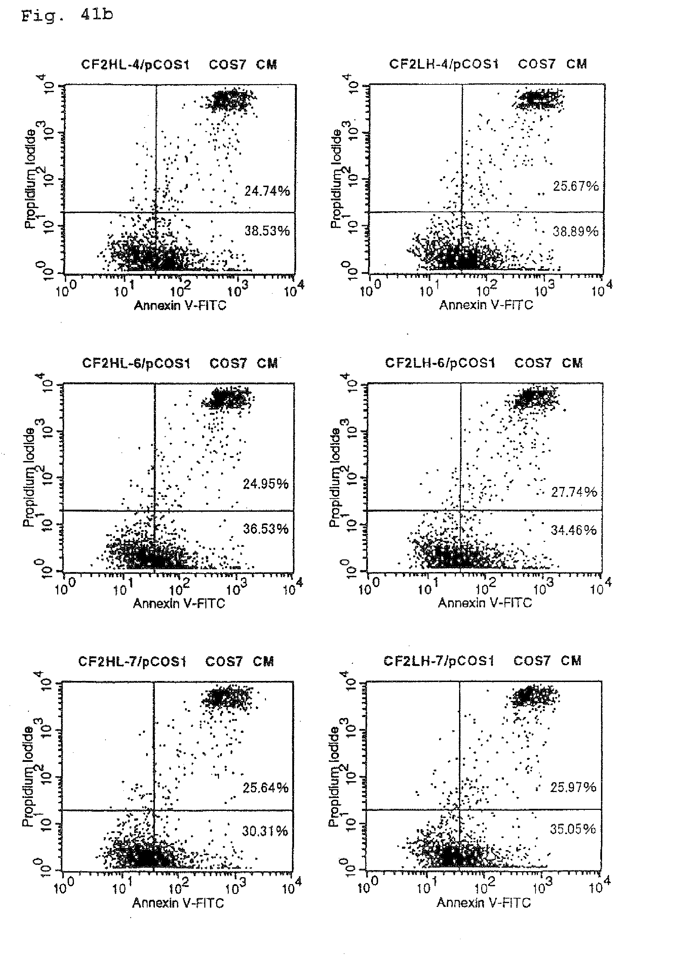

To 5 × 10⁴ cells of the hIAP/L1210 cells were added the culture supernatants of COS7 cells transfected with each vectors or a culture supernatant of COS7 cells as a control at 10% of the final concentration and the mixtures were cultured for 24 hours. Then, the Annexin-V/PI staining was performed and the fluorescence intensity was measured using the FACScan apparatus (BECTON DICKINSON). The results revealed that scFvs <HL3, 4, 6, 7, LH3, 4, 6, 7> and sc(Fv)₂ in CM of COS7 induced remarkable cell death of hIAP/L1210 cells. These results are shown in Fig. 41.

### 6.7 Construction of vectors for the expression of scFvs and sc(Fv)₂ in CHO cells

To isolate and purify MABL2-scFvs and sc(Fv)₂ from culture supernatant, the expression vectors for expressing in CHO cells were constructed as below.

The EcoRI-BamHI fragments of pCF2HL-0, 3 ~ 7, and pCF2LH-0, 3 ~ 7 prepared in Example 6.2 were introduced between EcoRI and BamHI sites in an expression vector pCHO1 for CHO cells using the Ligation High. Competent E. coli DH5α was transformed with them. The plasmids were isolated from the transformed E. coli using QIAGEN Plasmid Midi kit (QIAGEN) to prepare expression plasmids pCHOM2HL-0, 3 ~ 7, and pCHOM2LH-0, 3 ~ 7.

### 6.8 Production of CHO cells expressing MABL2-scFvs <HL-0, 3 ~ 7>, MABL2-scFvs <LH-0, 3 ~ 7> and sc(Fv)₂ and preparation of the culture supernatants thereof

CHO cells were transformed with each of the expression, plasmids pCHOM2HL-0, 3 ~ 7, and pCHOM2LH-0, 3 ~ 7, constructed in Example 6.7 and pCHOM2(Fv)₂ vector to prepare the CHO cells constantly expressing each modified antibody. As a typical example thereof, the production of the CHO cells constantly expressing MABL2-scFv <HL-5> or sc(Fv)₂ is illustrated as follows.

The expression plasmids pCHOM2HL-5 and pCHOM2(Fv)₂ were linearized by digesting with a restriction enzyme PvuI and subjected to transfection to CHO cells by electroporation using Gene Pulser apparatus (BioRad). The DNA (10 µg) and 0.75 ml of PBS with 1 × 10⁷ cells/ml were added to a cuvette and treated with pulse at 1.5 kV, 25 µF of electric capacity. After the restoration for 10 minutes at room temperature, the electroporated cells were transferred into nucleic acid-containing α-MEM culture medium (GIBCO BRL) containing 10% fetal bovine serum and cultured. After culturing overnight, the supernatant was discarded. The cells were washed with PBS and added to nucleic acid-free α-MEM culture medium (GIBCO BRL) containing 10% fetal bovine serum. After culturing for two weeks, the cells were cultured in a medium containing 10 nM (final concentration) methotrexate (SIGMA), then 50 nM and 100 nM methotrexate. The resultant cells were cultured in serum-free CHO-S-SFM II medium (GIBCO BRL) in a roller bottle. The culture supernatant was collected, centrifuged to remove cell fragments and filtered using a filter with 0.22 µm of pore size to obtain CM, respectively.

According to the above, CHO cells which constantly express MABL2-scFvs <HL-0, -3, -4, -6, -7> and <LH-0, -3, - 4, -5, -6, -7> and CMs thereof were obtained.

### 6.9 Purification of dimer of MABL2-scFv <HL-5> and sc(Fv)₂

The MABL2-scFv <HL-5> and the sc(Fv)₂ were purified from CMs prepared in Example 6.8 by two types of purification method as below.

### <Purification Method

HL-5 and sc(Fv)₂ were purified by the anti-FLAG antibody affinity column chromatography utilizing the FLAG sequence located at C-terminal of the polypeptides and by gel filtration. One liter of CM as obtained in 6.8 was applied onto a column (7.9ml) prepared with anti-FLAG M2 Affinity gel (SIGMA) equilibrated with 50 mM Tris-HCl buffer (TBS, pH 7.5) containing 150 mM NaCl. After washing the column with TBS, the scFv was eluted by 0.1 M glycine-HCl buffer, pH 3.5. The resultant fractions were analyzed by SDS-PAGE and the elution of the scFv was confirmed. The scFv fraction was mixed with Tween 20 up to 0.01% of the final concentration and concentrated using Centricon-10 (MILITPORE). The concentrate was applied onto TSKgel G3000SWG column (7.5 × 600 mm) equilibrated with 20 mM acetate buffer (pH 6.0) containing 150 mM NaCl and 0.01% Tween 20. At 0.4 mL/minute of the flow rate, the scFv was detected by the absorption at 280 nm. The HL-5 was eluted as the major fraction in the position of the dimer and the sc(Fv)₂ was eluted in the position of the monomer.

### <Purification Method

HL-5 and sc(Fv)₂ were purified using three steps comprising ion exchange chromatography, hydroxyapatite and gel filtration. In the ion exchange chromatography, Q sepharose fast flow column (Pharmacia) was employed for HL-5 and SP-sepharose fast flow column was employed for sc(Fv)₂. In and after the second step, HL-5 and sc(Fv)₂ were processed by the same procedure.

### First step for HL-5

CM of HL-5 was diluted to two times with 20 mM Tris-HCl buffer (pH 9.0) containing 0.02% Tween 20 and then the pH was adjusted to 9.0 with 1 M Tris. The solution was applied onto Q Sepharose fast flow column equilibrated with 20 mM Tris-HCl buffer (pH 8.5) containing 0.02% Tween 20. A polypeptide adsorbed to the column was eluted by a linear gradient of NaCl in the same buffer, from 0.1 to 0.55 M. Monitoring the eluted fractions by SDS-PAGE, the fractions containing HL-5 were collected and subjected to hydroxyapatite of the second step.

### First step for sc(Fv)₂

CM of the sc(Fv)₂ was diluted to two times with 20mM acetate buffer (pH 5.5) containing 0.02% Tween 20 and its pH was adjusted to 5.5 with 1 M acetic acid. The solution was applied onto a SP-Sepharose fast flow column equilibrated with 20 mM acetate buffer (pH 5.5) containing 0.02% Tween 20. A polypeptide adsorbed to the column was eluted by a linear gradient of NaCl in the buffer, from 0 to 0.5 M. Monitoring the eluted fractions by SDS-PAGE, the fractions containing the sc(Fv)₂ were collected and subjected to hydroxyapatite of the second step.

### Second step: Hydroxyapatite chromatography of HL-5 and sc(Fv)₂

The fractions of HL-5 and sc(Fv)₂ obtained in the first step were separately applied onto the hydroxyapatite column (Type I, BIORAD) equilibrated with 10 mM phosphate buffer containing 0.02% Tween 20, pH 7.0. After washing the column with the same buffer, polypeptides adsorbed to the column were eluted by a linear gradient of the phosphate buffer up to 0.5 M. Monitoring the eluted fractions by SDS-PAGE, the fractions containing the desired polypeptides were collected.

### Third step: Gel filtration of HL-5 and sc(Fv)₂

Each fraction obtained at the second step was separately concentrated with CentriPrep-10 (MILIPORE) and applied onto a Superdex 200 column (2.6 × 60 cm, Pharmacia) equilibrated with 20 mM acetate buffer (pH 6.0) containing 0.02% Tween 20 and 0.15 M NaCl. HL-5 was eluted in the position of the dimer, and sc(Fv)HL-5 and sc(Fv)₂ were eluted in the position of the monomer as a major peek respectively.

Since the monomer of HL-5 was hardly detected by both purification methods, it is proved that the dimers of single chain Fvs are formed in high yields when the linker for the single chain Fv contains around 5 amino acids. Furthermore, the dimer of HL-5 and the sc(Fv)₂ were stably preserved for a month at 4°C after the purification.

### 6.10 Evaluation of the binding activity of purified dimer of scFv <HL-5> and sc(Fv)₂ against antigen

Flow cytometry was performed using the purified dimer of MABL2-scFv <HL-5> and the purified sc(Fv)₂ in order to evaluate the binding to human Integrin Associated Protein (IAP) antigen. 10µg/ml of the purified dimer of MABL2-scFv <HL-5>, the purified sc(Fv)₂, the antibody MABL-2 as a positive control or a mouse IgG (Zymed) as a negative control was added to 2 × 10⁵ cells of the mouse leukemia cell line L1210 expressing human IAP (hIAP/L1210) or the cell line L1210 transformed with pCOS1 (pCOS1/L1210) as a control. After incubating on ice and washing, 10µg/mL of the mouse anti-FLAG antibody (SIGMA) was added and then the cells were incubated and washed. FITC labeled anti-mouse IgG antibody (BECTON DICKINSON) was added thereto and the cells were incubated and washed again. Then the fluorescence intensity was measured using the FACScan apparatus (BECTON DICKINSON).

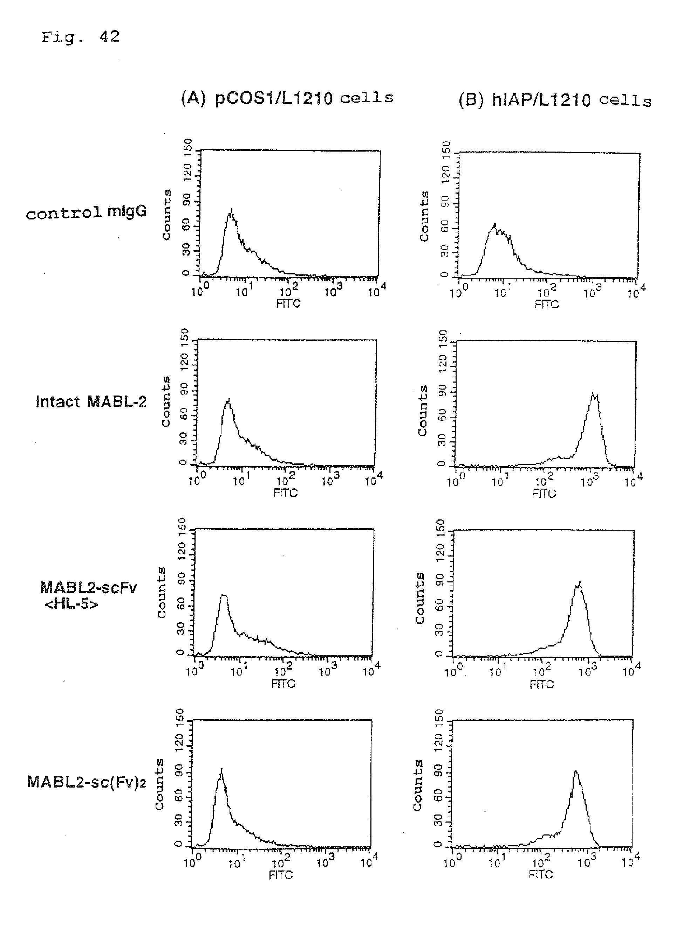

Since the purified dimer of MABL2-scFv <HL-5> and the purified sc(Fv)₂ were specifically bound to hIAP/L1210 cells, it is confirmed that the dimer of scFv <HL-5> and the sc(Fv)₂ have high affinity to human IAP (see Fig. 42).

### 6.11 Apoptosis-inducing activity in vitro of purified dimer of scFv <HL-5> and sc(Fv)₂

An apoptosis-inducing action of the purified dimer of MABL2-scFv <HL-5> and the purified sc(Fv)₂ were examined by Annexin-V staining (Boehringer Mannheim) using the L1210 cells (hIAP/L1210) in which human IAP gene had been introduced and cells of human leukemic cell line CCRF-CEM.

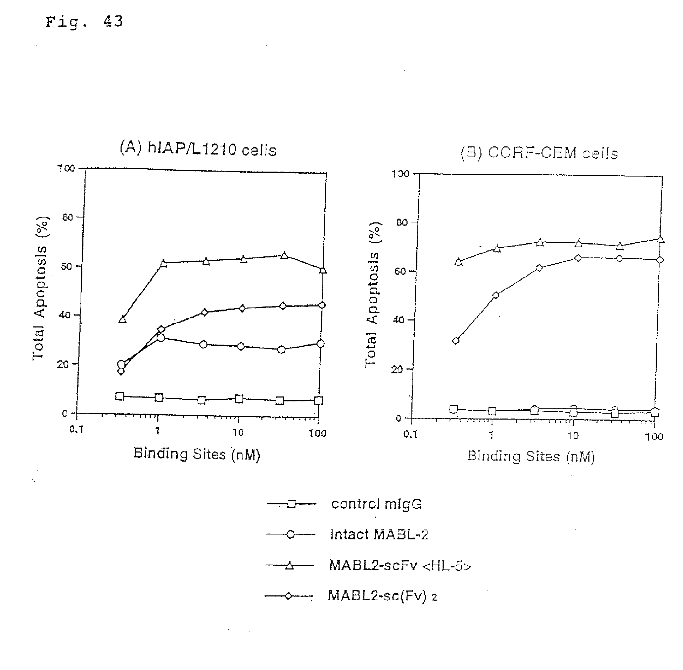

Different concentrations of the purified dimer of MABL2-scFv , the purified MABL2-sc(Fv)_{2,} the antibody MABL-2 as a positive control or a mouse IgG as a negative control were added to 5 × 10⁴ cells of hIAP/L1210 cell line or × 10⁵ cells of CCRF-CEM cell line. After culturing for 24 hours, the Annexin-V staining was carried out and the fluorescence intensity thereof was measured using the FACScan apparatus (BECTON DICKINSON). As a result the dimer of MABL2-scFv <HL-5> and the MABL2-sc(Fv)₂ remarkably induced cell death of hHIAP/L1210 and CCRF-CEM in concentration-dependent manner (see Fig. 43). As a result it was shown that the dimer of MABL2-scFv <HL-5> and MABL2-sc(Fv)₂, had improved efficacy of inducing apoptosis compared with original antibody MABL-2.

### 6.12 Hemagglutination Test of the purified dimer of scFv <HL-5> and the sc(Fv)₂

Hemagglutination test was carried out using different concentrations of the purified dimer of scFv <HL-5> and the purified sc(Fv)₂ in accordance with Example 5.15.

The hemagglutination was observed with the antibody MABL-2 as a positive control, whereas no hemagglutination was observed with both the single chain antibody MABL2-sc(Fv)₂ and the MABL2-scFv . Further, there was no substantial difference in the hemagglutination between two buffers employed with the antibody MABL-2. These results are shown in Table 3.

**Hemagglutination Test TABLE 3**

| | | **Diluent:PBS** | | | | | | | | | | | | | | |
|---|---|---|---|---|---|---|---|---|---|---|---|---|---|---|---|---|
| | | (µg/ml) | | | | | | | | | | | | | | |
| | cont | 28.9 | 14.45 | 7.225 | 3.6125 | 1.8063 | 0.9031 | 0.4516 | 0.2258 | 0.1129 | 0.0564 | 0.0282 | 0.0141 | 0.0071 | 0.0035 | 0.0018 |
| **MABL2-sc(Fv)₂** | - | - | - | - | - | - | - | - | - | - | - | - | - | - | - | - |
| | cont | 28.0 | 14.0 | 7.0 | 3.5 | 1.75 | 0.875 | 0.4375 | 0.2188 | 0.1094 | 0.0547 | 0.0273 | 0.0137 | 0.0068 | 0.0034 | 0.0017 |
| **MABL2-sc(Fv)** | - | - | - | - | - | - | - | - | - | - | - | - | - | - | - | - |
| | cont | 80 | 40 | 20 | 10 | 5 | 2.5 | 1.25 | 0.625 | 0.3125 | 0.1563 | 0.0781 | 0.0391 | 0.0195 | 0.0098 | 0.0049 |
| **MABL2 (intact)** | - | + | + | + | + | + | + | + | + | + | ± | - | - | - | - | - |
| **mlgG** | - | - | - | - | - | - | - | - | - | - | - | - | - | | - | - |

| **Diluent: Acetate Buffer** | | | | | | | **(µg/ml)** | | | | | | | | | |
|---|---|---|---|---|---|---|---|---|---|---|---|---|---|---|---|---|
| | cont | 80 | 40 | 20 | 10 | 5 | 2.5 | 1.25 | 0.625 | 0.3125 | 0.1563 | 0.0781 | 0.0391 | 0.0195 | 0.0098 | 0.0049 |
| **MABL2 (intact)** | - | + | + | + | + | + | + | + | + | + | + | + | - | - | - | - |

### 6.13 Antitumor effect of the purified dimer of scFv and the sc (Fv)₂ for a model mouse of human myeloma

The antitumor effects were tested for the dimer of scFv <HL-5> and the sc(Fv)₂ prepared and purified in Examples 6.8 and 6.9. The test was performed by using the mouse model for human myeloma produced in Example 5.1 and determining the amount of M protein produced by human myeloma cells in the mouse serum using ELISA and examining survival time of the mice. Then, the antitumor effects of the dimer of scFv <HL-5> and the sc(Fv)₂ were evaluated in terms of the change of the amount of M protein in the mouse serum and the survival time of the mice.

In the test, the HL-5 and the sc(Fv)₂ were employed as a solution at 0.01, 0.1 or 1 mg/mL in vehicle consisting of 150 mM NaCl, 0.02% Tween and 20 mM acetate buffer, pH 6.0 and administered to the mice at 0.1, 1 or 10 mg/kg of dosage. Control group of mice were administered only with the vehicle.

The mouse serum was gathered 26 days after the transplantation of the human myeloma cells and the amount of M protein in the serum was measured using ELISA according to Example 5.14. As a result, the amount of M protein in the serum of both mice groups administered with HL-5, the dimer and the sc(Fv)₂ decreased in dose-dependent manner (see Fig. 44). Furthermore, a significant elongation of the survival time was observed in both groups administered with the HL-5 (Fig. 45) and with the sc(Fv)₂ (Fig. 46) in comparison with the control group administered with the vehicle. These results show that the HL-5 and the sc(Fv)₂ of the invention have excellent antitumor effect in vivo.

### Example 7

### Single chain Fv comprising H chain V region and L chain V region of human antibody 12B5 against human MPL

A DNA encoding V regions of human monoclonal antibody 12B5 against human MPL was constructed as follows:

### 7.1 Construction of a gene encoding H chain V region of 12B5

The gene encoding H chain V region of human antibody 12B5 binding to human MPL was designed by connecting the nucleotide sequence of the gene thereof (SEQ ID NO: 55) at the 5'-end to the leader sequence (SEQ ID NO: 56) originated from human antibody gene (Eur. J. Immunol. 1996; 26: 63-69). The designed nucleotide sequence was divided into four oligonucleotides having overlapping sequences of 15 bp each (12B5VH-1, 12B5VH-2, 12B5VH-3, 12B5VH-4). 12B5VH-1 (SEQ ID NO: 57) and 12B5VH-3 (SEQ ID NO: 59) were synthesized in the sense direction, and 12B5VH-2 (SEQ ID NO: 58) and 12B5VH-4 (SEQ ID NO: 60) in the antisense direction, respectively. After assembling each synthesized oligonucleotide by respective complementarity, the outside primers (12B5VH-S and 12B5VH-A) were added to amplify the full length of the gene. 12B5VH-S (SEQ ID NO: 61) was designed to hybridize to 5'-end of the leader sequence by the forward primer and to have Hind III restriction enzyme recognition site and Kozak sequence, and 12B5VH-A (SEQ ID NO: 62) was designed to hybridize to the nucleotide sequence encoding C-terminal of H chain V region by the reverse primer and to have a splice donor sequence and BamHI restriction enzyme recognition site, respectively.

100µl of the PCR solution containing 10µl of 10 × PCR Gold Buffer II, 1.5mM MgCl₂, 0.08mM dNTPs (dATP, dGTP, dCTP, dTTP), 5 units of DNA-polymerase AmpliTaq Gold (all by PERKIN ELMER) and each 2.5 p mole of each synthesized oligonucleotide (12BSVH-1 to -4) was heated at 94°C of the initial temperature for 9 minutes, at 94°C for 2 minutes, at 55°C for 2 minutes and 72°C for 2 minutes. After repeating the cycle two times each 100 pmole of external primer 12B5VH-S and 12B5VH-A was added. The mixture was subjected to the cycle consisting of at 94°C for 30 seconds, at 55°C for 30 seconds and 72°C for 1 minute 35 times and heated at 72°C for further 5 minutes.

The PCR product was purified by 1.5% low-melting-temperature agarose gel (Sigma), digested by restriction enzymes BamHI and Hind III, and cloned into expression vector HEF-gγl for human H chain. After determining the DNA sequence the plasmid containing the correct DNA sequence was named HEF-12B5H-gγl.

The HEF-12B5H-gγl was digested by restriction enzymes EcoRI and BamHI to produce the gene encoding 12B5VH which was then cloned into an expression vector pCOS-Fd for human Fab H chain to produce pFd-12B5H. The expression vector for human Fab H chain was constructed by amplifying the DNA (SEQ ID NO: 63) containing the intron region existing between the genes encoding human antibody H chain V region and the constant region, and the gene encoding a part of the constant region of human, H chain by PCR, and inserting the PCR product into animal cell expression vector pCOS1. The human H chain constant region was amplified for the gene under the same conditions mentioned above using as the template HEF-gγl, as the forward primer G1CH1-S (SEQ ID NO: 64) which was designed to hybridize to 5'-end sequence of intron 1 and to have restriction enzyme recognition sites EcoRI and BamHI and as the reverse primer G1CH1-A (SEQ ID NO: 65) which was designed to hybridize to 3'-end DNA of human H chain constant region CH1 domain and to have a sequence encoding a part of hinge region, two stop codons and restriction enzyme recognition site Bg1 II.

The nucleotide sequence and amino acid sequence of the reconstructed 12B5H chain variable region which were included in plasmids HEF-12B5H-gγl and pFd-12B5H are shown in SEQ ID NO: 66.

### 7.2 Construction of the gene encoding 1285 L chain V region

The gene encoding L chain V region of human antibody 12B5 binding to human MPL was designed by connecting the nucleotide sequence of gene (SEQ ID NO: 67) at the 5'-end to the leader sequence (SEQ ID NO: 68) originated from human antibody gene 3D6 (Nuc. Acid Res. 1990: 18; 4927). In the same way as mentioned above the designed nucleotide sequence was divided into four oligonucleotides having overlapping sequences of 15 bp each (12B5VL-1, 12B5VL-2, 12B5VL-3, 12B5VL-4) and synthesized respectively. 12B5VL-1 (SEQ ID NO: 69) and 12B5VL-3 (SEQ ID NO: 71) had sense sequences, and 12BSVL-2 (SEQ ID NO: 70) and 12B5VL-4 (SEQ ID NO: 72) had antisense sequences, respectively. Each of the synthesized oligonucleotides was assembled by respective complementarity and mixed with the external primer (12B5VL-S and 12B5VL-A) to amplify the full length of the gene. 12B5VL-S (SEQ ID NO: 73) was designed to hybridize to 5'-end of the leader sequence by the forward primer and to have Hind III restriction enzyme recognition site and Kozak sequence. 12B5VL-A (SEQ ID NO: 74) was designed to hybridize to the nucleotide sequence encoding C-terminal of L chain V region by the reverse primer and to have a splice donor sequence and BamHI restriction enzyme recognition site.

Performing the PCR as mentioned above, the PCR product was purified by 1.5% low-melting-temperature agarose gel (Sigma), digested by restriction enzymes BamHI and Hind III, and cloned into an expression vector HEF-gκ for human L chain. After determining the DNA sequence the plasmid containing the correct DNA sequence was named HEF-12B5L-gκ. The nucleotide sequence and amino acid sequence of the reconstructed 12B5 L chain V region which were included in plasmid HEF-12B5L-gκ are shown in SEQ ID NO:75.

### 7.3 Production of reconstructed 12B5 single chain Fv (scFv)

The reconstructed 12B5 antibody single chain Fv was designed to be in the order of 12B5VH-linker-12B5VL and to have FLAG sequence (SEQ ID NO: 76) at C-terminal to facilitate the detection and purification. The reconstructed 12B5 single chain Fv (sc12B5) was constructed using a linker sequence consisting of 15 amino acids represented by (Gly₄Ser)₃.

### (1) Production of the reconstructed 12B5 single chain Fv using the linker sequence consisting of 15 amino acids

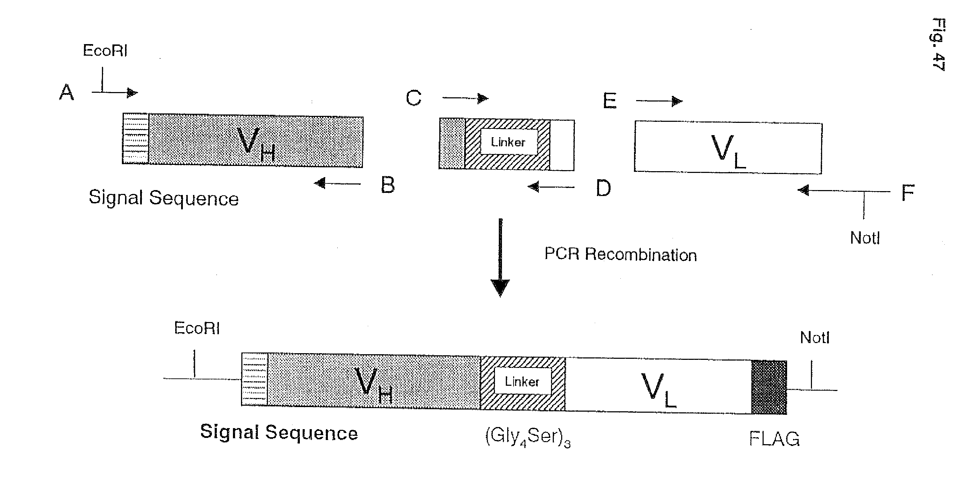

The gene encoding the reconstructed 12B5 antibody single chain Fv, which contained the linker sequence consisting of 15 amino acids, was constructed by connecting 12B5 H chain V region, linker region and 12B5 L chain V region which was amplified by PCR respectively. This method is schematically shown in Fig. 47. Six PCR primers (A-F) were used for production of the reconstructed 12B5 single chain Fv. Primers A, C, and E had sense sequences, and primers B, D, and F had antisense sequences.

The forward primer 12B5-S (Primer A, SEQ ID NO: 77) for H chain V region was designed to hybridize to 5'-end of H chain leader sequence and to have EcoRI restriction enzyme recognition site. The reverse primer HuVHJ3 (Primer B, SEQ ID NO: 78) for H chain V region was designed to hybridize to DNA encoding C-terminal of H chain V region.

The forward primer RHuJH3 (Primer C, SEQ ID NO: 79) for the linker was designed to hybridize to DNA encoding the N-terminal of the linker and to overlap DNA encoding the C-terminal of H chain V region. The reverse primer RHuVK1 (Primer D, SEQ ID NO: 80) for the linker was designed to hybridize to DNA encoding the C-terminal of the linker and overlap DNA encoding the N-terminal of L chain V region.

The forward primer HuVK1.2 (Primer E, SEQ ID NO: 81) for L chain V region was designed to hybridize to DNA encoding the N-terminal of L chain V region. The reverse primer 12B5F-A for L chain V region (Primer F, SEQ ID NO: 82) was designed to hybridize to DNA encoding C-terminal of L chain V region and to have the sequence encoding FLAG peptide (Hopp, T. P. et al., Bio/Technology, 6, 1204-1210, 1988), two transcription stop codons and NotI restriction enzyme recognition site.

In the first PCR step, three reactions A-B, C-D, and E-F were performed, and the three PCR products obtained from the first step PCR were assembled by respective complementarity. After adding primers A and F the full length DNA encoding the reconstructed 12B5 single chain Fv having the linker consisting of 15 amino acids was amplified (the second PCR). In the first step PCR, the plasmid HEF-12B5H-gγl (see Example 7. 1) encoding the reconstructed 12B5 H chain V region, pSCFVT7-hM21 (humanized ONS-M21 antibody) (Ohtomo et al., Anticancer Res. 18 (1998), 4311-4316) containing DNA (SEQ ID NO: 83) encoding the linker region consisting of Gly Gly Gly Gly Ser Gly Gly Gly Gly Ser Gly Gly Gly Gly Ser (Huston et al., Proc. Natl. Acad. Sci. USA, 85, 5879-5883, 1988) and the plasmid HEF-12B5L-gκ (see Example 7. 2) encoding the reconstructed 12B5 L chain V region were used as templates, respectively.

50µl of PCR solution for the first step contained 5µl of 10 x PCR Gold Buffer II, 1.5mM MgCl₂, 0.08mM dNTPs, 5 units of DNA polymerase AmpliTaq Gold (all by PERKIN ELMER), each 100 pmole of each primer and 100ng of each template DNA. The PCR solution was heated at 94°C of the initial temperature for 9 minutes, at 94 for 30 seconds, 55°C for 30 seconds and 72°C for 1 minute. After repeating the cycle 35 times the reaction mixture was further heated 72°C for 5 minutes.

The PCR products A-B, C-D, and E-F were assembled by the second PCR. PCR mixture solution for the second step of 98µl containing as the template 1µl of the first PCR product A-B, 0.5µl of PCR product C-D and 1µl of PCR product E-F, 10µl of 10 x PCR Gold Buffer II, 1.5mM MgCl₂, 0.08mM dNTPs, 5 units of DNA polymerase AmpliTaq Gold (all by PERKIN ELMER) was heated at 94°C of the initial temperature for 9 minutes, at 94°C for 2 minutes, at 65°C for 2 minutes and 72°C for 2 minutes. After repeating the cycle two times, each 100 pmole of each of primers A and F were added. After repeating the cycle consisting of at 94°C for 30 seconds, 55°C for 30 seconds and 72°C for 1 minute 35 times, the reaction mixture was heated at 72°C for 5 minutes.

The DNA fragments produced by the second PCR were purified using 1.5% low-melting-temperature agarose gel, digested by EcoRI and NotI, and cloned into pCHO1 vector and pCOS1 vector (Japanese Patent Application No. 8-255196).
The expression vector pCHO1 was a vector constructed by deleting the antibody gene from DHFR-ΔE-rvH-PM1-f (see WO92/19759) by EcoRI and SmaI digestion, and connecting to EcoRI-NotI-BamHI Adaptor (TAKARA SHUZO). After determining the DNA sequence the plasmids containing the DNA fragment encoding the correct amino acid sequence of reconstructed 12B5 single chain Fv were named pCHO-sc12B5 and pCOS-sc12B5. The nucleotide sequence and amino acid sequence of the reconstructed 12B5 single chain Fv included in the plasmids pCHO-sc12B5 and pCOS-sc12B5 are shown in SEQ ID NO: 84.

### 7.4 Expression of antibody 12B5 (IgG, Fab) and single chain Fv polypeptide by animal cell

Antibody 12B5 (IgG, Fab) and single chain Fv derived from antibody 12B5 were expressed by using COS-7 cells or CHO cells.

The transient expression using COS-7 cells was performed as follows. The transfection was performed by electroporation method using Gene Pulser equipment (BioRad). For the expression of antibody 12B5 (IgG) each 10µg of the above-mentioned expression vector HEF-12B5H-gγl and HEF-12 B5L-gκ were added, for the expression of 12B5Fab fragment each 10µg of pFd-12B5H and HEF-12B5L-gκ were added and for the expression of single chain Fv 10µg of pCOS-sc12B5 was added to COS-7 cells (1×10⁷ cells/ml) suspended in 0.8ml of PBS. The mixture kept in a cuvette was treated by pulse at the capacity of 1.5kV, 25µFD. After recovering for 10 minutes in a room temperature the electroporated cells were added to DMEM culture medium (CIBCO BRL) containing 10% bovine fetal serum cultivated. After cultivating overnight the cells were washed once by PBS, added to serum-free medium CHO-S-SFM II and cultivated for 2 days. The culture medium was centrifuged to remove cell debris and filtered with 0.22µm filter to prepare the culture supernatant.

To establish a stable expression CHO cell line for the single chain Fv (polypeptide) derived from antibody 12B5, the expression vector pCHO-sc12B5 was introduced into CHO cells as follows.

The expression vector was introduced into CHO cells by electroporation method using Gene Pulser equipment (BioRad). Linearized DNA (100µg) obtained by digestion with restriction enzyme PvuI and CHO cells, (1x10⁷ cells /ml) suspended in 0.8 ml of PBS were mixed in a cuvette, left stationary on ice for 10 minutes and treated with pulse at the capacity of 1.5kV, 25µFD. After recovering for 10 minutes at a room temperature the electroporated cells were added to CHO-S-SFM II (GIBCO BRL) containing 10% bovine fetal serum and cultivated. After cultivating for 2 days the cultivation was continued in CHO-S-SFM II (GIBCO BRL) containing 5nM methotrexate (SIGMA) and 10% bovine fetal serum. From thus obtained clones a clone with high expression rate was selected as the production cell line for 12B5 single chain Fv. After cultivating in serum-free medium CHO-S-SFM II (GIBCO BRL) containing 5nM methotrexate (SIGMA), the culture supernatant was obtained by centrifugal separation of cell debris.

### 7.5 Purification of single chain Fv derived from 12B5 produced by CHO cells

The culture supernatant of CHO cell line expressing 12B5 single chain Fv obtained in 7.4 was purified by anti-FLAG antibody column and gel filtration column.

### (1) Anti-FLAG antibody column

The culture supernatant was added to anti-FLAG M2 affinity gel (SIGMA) equilibrated by PBS. After washing the column by the same buffer the proteins adsorbed to the column were eluted by 0.1M glycine-HCl buffer (pH 3.5). The eluted fractions were immediately neutralized by adding 1M Tris-HCl buffer (pH 8.0). The eluted fractions were analyzed by SDS-PAGE and the fraction which was confirmed to contain the single chain Fv was concentrated using Centricon-10 (MILLIPORE) .

### (2) Gel filtration

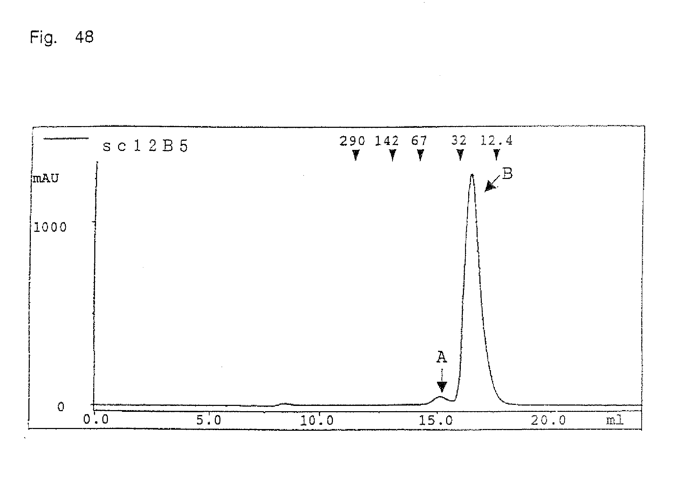

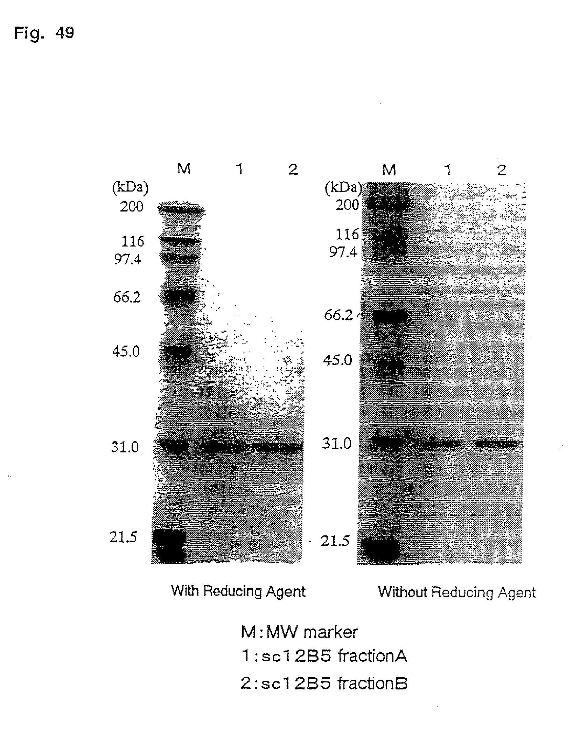

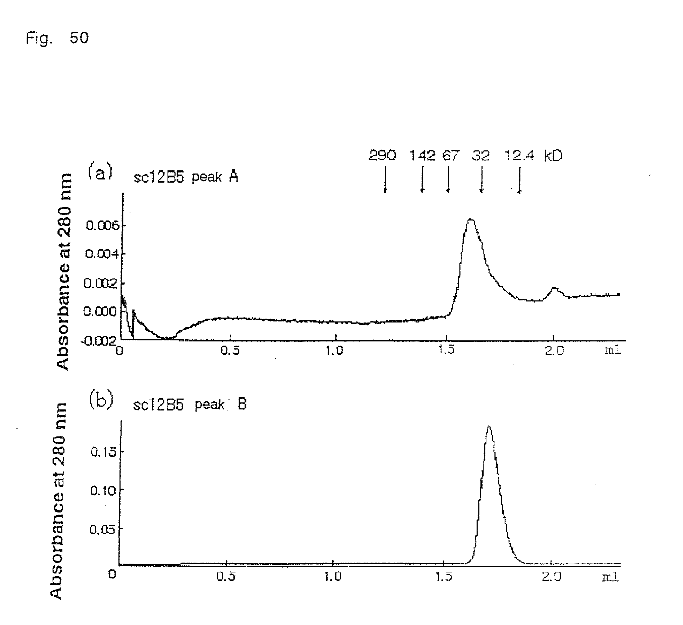

The concentrated solution obtained in (1) was added to Superdex200 column (10x300mm, AMERSHAM PHARMACIA) equilibrated by PBS containing 0.01% Tween20.
The product sc12B5 was eluted in two peaks (A, B) (see Fig. 48). The fractions A and B were analyzed using the 14%-SDS-polyacrylamide gel. The sample was processed by electrophoresis in the presence and absence of a reducing agent according to Laemmli method, and stained by Coomassie Brilliant Blue after the electrophoresis. As shown in Fig. 49 the fractions A and B, regardless of the presence of the reducing agent or its absence, produced a single band having an apparent molecular weight of about 31 kD. When the fractions A and B were analyzed by gel filtration using Superdex200 PC 3.2/30 (3.2x300mm, AMERSHAM PHARMACIA), the fraction A produced an eluted product at an apparent molecular weight of about 44 kD and the fraction B produced at 22kD (see Fig. 50a and b). The results show that the fraction A is the non-covalent bond dimer of sc12B5 single chain Fv, and B is the monomer.

### 7.6 Measurement of TPO-like agonist activity of various single chain Fvs

The TPO-like activity of anti-MPL single chain antibody was evaluated by measuring the proliferation activity to Ba/F3 cells (BaF/mpl) expressing human TPO receptor (MPL). After washing BaF/Mpl cells two times by RPMI1640 culture medium (GIBCO) containing 10% bovine fetal serum (GIBCO), the cells were suspended in the culture medium at cell density of 5x10⁵ cells/ml. The anti-MPL single chain antibody and human TPO (R&D Systems) was diluted with the culture medium, respectively. 50µl of the cell suspension and 50µl of the diluted antibody or human TPO were added in 96-well microplate (flat bottom) (Falcon), and cultivated in CO₂ incubator (CO₂ concentration: 5%) for 24 hours. After the incubation 10µl of WST-8 reagent (reagent for measuring the number of raw cells SF: Nacalai Tesque) was added and the absorbance was immediately measured at measurement wavelength of 450nm and at refference wavelength of 620nm using fluorescence absorbency photometer SPECTRA Fluor (TECAN). After incubating in CO₂ incubator (CO₂ concentration: 5%) for 2 hours, the absorbance at 450nm of measurement wavelength and 620nm of refference wavelength was again measured using SPECTRA Fluor. Since WST-8 reagent developed the color reaction depending upon the number of live cells at wavelength of 450nm, the proliferation activity of BaF/Mpl based on the change of absorbance in 2 hours was evaluated by ED 50 calculated as follows. In the proliferation reaction curve wherein the absorbance was plotted on the ordinate against the antibody concentration on the abscissa, the absorbance at the plateau was set 100% reaction rate. Obtaining an approximation formula by straight line approximation method based on the plotted values close to 50% reaction rate, the antibody concentration of 50% reaction rate was calculated and adopted as ED 50.

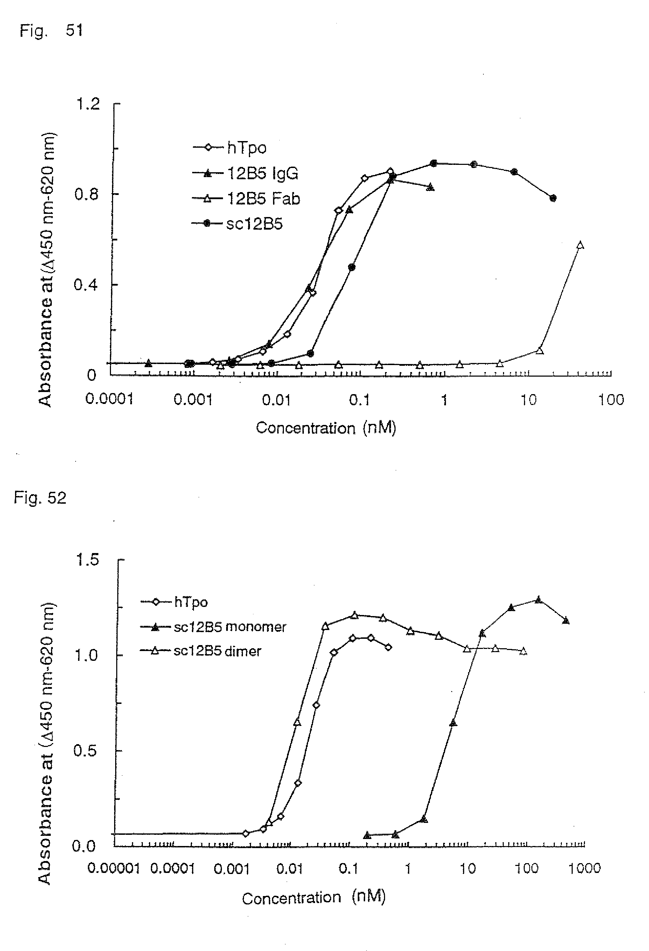

The results of the agonist activity to MPL measured by using culture supernatants of COS-7 cells expressing various 12B5 antibody molecules showed as illustrated in Fig. 51 that 12B5IgG having bivalent antigen-binding site increased the absorbance in concentration-dependent manner and had TPO-like agonist activity (ED50; 29nM), while the agonist activity of 12B5Fab having monovalent antigen-biding site was very weak (ED50; 34,724nM). On the contrary the single chain Fv (sc12B5) having monovalent antigen-binding site like Fab showed strong agonist activity at a level that ED50 was 75nM. However it has been known that variable regions of H chain and L chain of the single chain Fv are associated through non-covalent bond and, therefore, each variable region is dissociated in a solution and can be associated with variable region of other molecule to form multimers like dimers. When the molecular weight of sc12B5 purified by gel filtration was measured, it was confirmed that that there were molecules recognized to be monomer and dimer (see Fig. 48). Then monomer sc12B5 and dimer sc12B5 were isolated (see Fig. 50) and measured for the agonist activity to MPL. As shown in Figs. 51 and 52, ED50 of sc12B5 monomer was 4438.7nM, which confirmed that the agonist activity was reduced compared with the result using culture supernatant of COS-7 cells. On the contrary single chain Fv (sc12B5 dimer) having bivalent antigen-binding site showed about 400-fold stronger agonist activity (ED50; 10.1nM) compared with monovalent sc12B5. Furthermore, the bivalent single chain Fv showed the agonist activity equivalent to or higher than the agonist activity of human TPO and 12B5IgG.

### Example 8

### Construction of a gene encoding the variable region of human antibody 12E10 against human MPL

A DNA encoding variable region of human monoclonal antibody 12E10 against human MPL was constructed as follows:

### 8.1 Construction of a gene encoding 12E10 H chain V region

The nucleotide sequence SEQ ID NO:86 was designed as a gene encoding H chain V region of human antibody 12E10 binding to human MPL on the basis of the amino acid sequence described in WO99/10494 SEQ ID NO:85). The full length of nucleotide sequence was designed by connecting to its 5'-end the leader sequence (SEQ ID NO:87) derived from human antibody gene (GenBank accession No. AF062252). The designed nucleotide sequence was divided into four oligonucleotides having overlapping sequences of 15 bp each (12E10VH1, 12E10VH2, 12E10VH3, 12E10VH4). 12E10VH1 (SEQ ID NO: 88) and 12E10VH3 (SEQ ID NO: 90) were synthesized in the sense direction, and 12E10VH2 (SEQ ID NO: 89) and 12E10VH4 (SEQ ID NO: 91) in the antisense direction, respectively. After assembling each synthesized oligonucleotide by respective complementarity, the external primers (12E10VHS and 12E10VHA) were added to amplify the full length of the gene. 12E10VHS (SEQ ID NO: 92) was designed to hybridize to 5'-end of the leader sequence by the forward primer and to have Hind III restriction enzyme recognition site and Kozak sequence, and 12E10VHA (SEQ ID NO: 93) was designed to hybridize to the nucleotide sequence encoding C-terminal of H chain V region by the reverse primer and to have a splice donor sequence and BamHI restriction enzyme recognition site, respectively.

100µl of the PCR solution containing 10µl of 10 x PCR Gold Buffer II, 1.5mM MgCl₂, 0.08mM dNTPs (dATP, dGTP, dCTP, dTTP) , 5 units of DNA-polymerase AmpliTaq Gold (all by PERKIN ELMER) and each 2.5pmole of each synthesized oligonucleotide (12B5VH-1 to -4) was heated at 94°C of the initial temperature for 9 minutes, at 94°C for 2 minutes, at 55°C for 2 minutes and 72°C for 2 minutes. After repeating the cycle two times each 100 pmole of external primer 12E10VHS and 12E10VHA were added. The mixture was subjected to the cycle consisting of at 94°C for 30 seconds, at 55°C for 30 seconds and 72°C for 1 minute 35 times and heated at 72°C for further 5 minutes.

The PCR. product was purified by 1.5% low-melting-temperature agarose gel (Sigma), digested by restriction enzymes BamHI and Hind III, and cloned into a human H chain expression vector HEF-gγ1. After determining the DNA sequence the plasmid containing the correct DNA sequence was named HEF-12E10H-gγ1.

The HEF-12E10H-gγ1 was digested by restriction enzymes EcoRI and BamHI to produce the gene encoding 12E10VH and then cloned into a human Fab H chain expression vector pCOS-Fd to produce pFd-12E10H. The human Fab H chain expression vector was constructed by amplifying the DNA (SEQ ID NO: 63) containing the intron region existing between the genes encoding human antibody H chain V region and the constant region, and the gene encoding a part of the human H chain constant region by PCR, and inserting the PCR product into animal cell expression vector pCOS1. The human H chain constant region was amplified for the gene under the same conditions mentioned above using as the template HEF-gγ1, as the forward primer G1CH1-S (SEQ ID NO: 64) which was designed to hybridize to 5'-end sequence of intron 1 and to have restriction enzyme recognition sites EcoRI and BamHI and as the reverse primer G1CH1-A (SEQ ID NO: 65) which was designed to hybridize to 3'-end DNA of human H chain constant region CH1 domain and to have a sequence encoding a part of hinge region, two stop codons and restriction enzyme recognition site Bg1 II.

The nucleotide sequence and amino acid sequence of the reconstructed 12E10 H chain variable region which were included in plasmids HEF-12E10H-gγ1 and pFd-12E10H are shown in SEQ ID NO: 94.

### 8.2 Construction of a gene encoding 12E10 L chain V region

The nucleotide sequence SEQ ID NO:96 was designed as a gene encoding L chain V region of human antibody 12E10 binding to human MPL on the basis of the amino acid sequence described in WO99/10494 (SEQ ID NO:95). It was further designed by connecting to its 5'-end the leader sequence (SEQ ID NO: 97) derived from human antibody gene (Mol. Immunol. 1992; 29: 1515-1518). In the same way as mentioned above the designed nucleotide sequence was divided into four oligonucleotides having overlapping sequences of 15 bp each (12E10VL1, 12E10VL2, 12E10VL3, 12E10VL4) and synthesized respectively. 12E10VL1 (SEQ ID NO: 98) and 12E10VL3 (SEQ ID NO: 100) had sense sequences, and 12E10VL2 (SEQ ID NO: 99) and 12E10VL4 (SEQ ID NO: 101) had antisense sequences, respectively. Each of the synthesized oligonucleotides was assembled by respective complementarity and mixed with the external primers (12E10VLS and 12E10VLA) to amplify the full length of the gene. 12E18VLS (SEQ ID NO: 102) was designed to hybridize to 5'-end of the leader sequence by the forward primer and to have EcoRI restriction enzyme recognition site and Kozak sequence. 12E10VLA (SEQ ID NO: 103) was designed to hybridize to the nucleotide sequence encoding C-terminal of L chain V region by the reverse primer and to have a BInI restriction enzyme recognition site.

Performing the PCR as mentioned above, the PCR product was purified by 1.5% low-melting-temperature agarose gel (Sigma), digested by restriction enzymes EcoRI and BlnI, and cloned into pUC19 containing a gene for human lambda chain constant region. After determining the DNA sequence the plasmid containing the correct DNA sequence was digested by EcoRI to produce a gene encoding 12E10 L chain V region and human lambda chain constant region and then inserted in expression vector pCOS1. The plasmid having 12E10 L chain gene (SEQ ID NO: 104) was named pCOS-12E10L

### 8.3 Production of reconstructed 12E10 single chain Fv

The reconstructed 12E10 antibody single chain Fv was designed to be in the order of 12E10VH-linker-12E10VL and to have FLAG sequence (SEQ ID NO: 105) at C-terminal to facilitate the detection and purification. The reconstructed 12E10 chain Fvs (sc12E10 and db12E10) were constructed using a linker sequence consisting of 15 amino acids represented by (Gly₄Ser) or 5 amino acids represented by (Gly₄Ser)₁.

### (1) Production of the reconstructed 12E10 single chain Fv using the linker sequence consisting of 5 amino acids

The gene encoding the reconstructed 12E10 single chain Fv, which contained the linker sequence consisting of 5 amino acids, was constructed by introducing the nucleotide sequence for the linker (Gly₄Ser)₁ to 3'-end of the gene encoding 12E10 H chain V region and to 5'-end of the gene encoding 12E10 L chain V region, amplifying thus obtained respective gene by PCR and connecting the amplified genes. Four PCR primers (A-D) were used to produce the reconstructed 12E10 single chain Fv. Primers A and C had sense sequences, and primers B and D had antisense sequences.

The forward primer for H chain V region was 12E10S (Primer A, SEQ ID NO: 106). The reverse primer DB2 (Primer B, SEQ ID NO: 107) for H chain V region was designed to hybridize to DNA encoding C-terminal of H chain V region and to have the nucleotide sequence encoding the linker (Gly₄Ser)₁ and the nucleotide sequence encoding N-terminal of L chain V region.

The forward primer DB1 (Primer C, SEQ ID NO: 108) for L chain V region was designed to hybridize to DNA encoding the N-terminal of L chain V region and to have the nucleotide sequence encoding the linker (Gly₄Ser)₁ and the nucleotide sequence encoding C-terminal of H chain V region. The reverse primer 12E10FA (Primer D, SEQ ID NO: 109) for L chain V region was designed to hybridize to DNA encoding the C-terminal of L chain V region and to have the nucleotide sequence encoding FLAG and NotI restriction enzyme recognition site.

In the first PCR step, two reactions A-B and C-D were performed, and the two PCR products obtained from the first step PCR were assembled by respective complementarity. After adding primers A and D the full length DNA encoding the reconstructed 12E10 single chain Fv having the linker consisting of 5 amino acids was amplified (the second PCR). In the first step PCR, the plasmid HEF-12E10H-gγ1 (see Example 8. 1) encoding the reconstructed 12E100 H chain V region and pCOS-12E10L (see Example 8.1) encoding the reconstructed 12E10 L chain V region were used as templates, respectively.

50µl of the first step PCR solution contained 5 µl of 10 x PCR Gold Buffer II, 1. 5m.M MgCl₂, 0. 08mM dNTPs, 5 units of DNA polymerase AmpliTaq Gold (by PERKIN ELMER), each 100 pmole of each primer and 100ng of each template DNA. The PCR solution was heated at 94°C of the initial temperature for 9 minutes, at 94 for 30 seconds, 55°C for 30 seconds and 72°C for 1 minute. After repeating the cycle 35 times the reaction mixture was further heated at 72°C for 5 minutes.

The PCR products A-B (429bp) and C-D (395bp) were assembled by the second PCR. The second step PCR mixture solution (98µl) containing 1µl each of the first PCR product A-B and C-D as templates, 100 pmole each of each primer, 10µl of 10 x PCR Gold Buffer II, 1.5mM MgCl₂, 0.08mM dNTPs and 5 units of DNA polymerase AmpliTaq Gold (by PERKIN ELMER) was reacted under the same conditions as mentioned above.

The DNA fragment of 795bp produced by the second PCR was purified using 1.5% low-melting-temperature agarose gel, digested by EcoRI and NotI, and cloned into pCHO1 vector or pCOS1 vector. The expression vector pCHO1 was a vector constructed by deleting the antibody gene from DHFR-ΔE-RVH-PM1-f (see WO92/19759) by EcoRI and SmaI digestion, and connecting to EcoRI-NotI-BamHI Adaptor (TAKARA SHUZO).

After determining the DNA sequence the plasmids containing the DNA fragment encoding the correct amino acid sequence of reconstructed 12B5 single chain Fv were named pCHO-db12E10 and pCOS-db12E10. The nucleotide sequence and amino acid sequence of the reconstructed 12E10 single chain Fv included in the plasmids pCHO-db12E10 and pCOS-db12E10 are shown in SEQ ID NO: 110.

### (2) Production of the reconstructed 12E10 single chain Fv using the linker sequence consisting of 15 amino acids

The gene encoding the reconstructed 12E10 antibody single chain Fv, which contained the linker sequence consisting of 15 amino acids, was constructed by introducing the nucleotide sequence for the linker (Gly₄Ser)₃ to 3'-end of the gene encoding 12E10 H chain V region and to 5'-end of the gene encoding 12E10 L chain V region, amplifying thus obtained respective gene by PCR and connecting the amplified genes. Four PCR primers (A-D) were used for production of the reconstructed 12E10 single chain Fv. Primers A and C had sense sequences, and primers B and D had antisense sequences.

The forward primer for H chain V region was 12E10S (Primer A, SEQ ID NO: 106). The reverse primer sc4.3 (Primer B, SEQ ID NO: 111) for H chain V region was designed to hybridize to DNA encoding C-terminal of H chain V region and to have the nucleotide sequence encoding the linker (Gly=Ser)₃ and the nucleotide sequence encoding N-terminal of L chain V region.

The forward primer sc1.3 (Primer C, SEQ ID NO: 112) for L chain V region was designed to hybridize to DNA encoding the N-terminal of L chain V region and to have the nucleotide sequence encoding the linker (Gly₄Ser)₃ and the nucleotide sequence encoding C-terminal of H chain V region. The reverse primer 12E10FA (Primer D, SEQ ID NO: 109) for L chain V region was designed to hybridize to DNA encoding the C-terminal of L chain V region and to have the nucleotide sequence encoding FLAG and NotI restriction enzyme recognition site.

In the first PCR step, two reactions A-B and C-D were performed, and the two PCR products obtained from the first step PCR were assembled by respective complementarity. After adding primers A and D the full length DNA encoding the reconstructed 12E10 single chain Fv having the linker consisting of 15 amino acids was amplified (the second PCR). In the first step PCR, the plasmid pCOS-db12E10 (see Example 8. 1(1)) encoding the reconstructed 12E10 single chain Fv was used as template.

50µl of the first step PCR solution contained 5µl of 10 x ExTaq Buffer, 0.4mM dNTPs, 2.5 units of DNA polymerase TaKaRa ExTaq (by TAKARA), each 100 pmole of each primer and long of each template DNA. The PCR solution was heated at 94°C of the initial temperature for 30 seconds, at 94 for 15 seconds and 72°C for 2 minute, and the cycle was repeated 5 times. After repeating 28 times the cycle of at 94°C for 15 seconds and at 70°C for 2 minutes, the reaction mixture was further heated at 72°C for 5 minutes.

The PCR products A-B (477bp) and C-D (447bp) were assembled by the second PCR. The second step PCR mixture solution (98µl) containing 1µl each of the first PCR products A-B and C-D as templates, 100 pmole each of each primer A and D, 5µl of 10 x ExTaq Buffer, 0.4mM dNTPs, 2.5 units of DNA polymerase TaKaRa ExTaq (by TAKARA) was reacted under the same conditions as mentioned above.

The DNA fragment of 825bp produced by the second PCR was purified using 1.0% low-melting-temperature agarose gel, digested by EcoRI and NotI. Thus obtained DNA fragment was cloned into pCHO1 vector or pCOS1 vector. After determining the DNA sequence the plasmids containing the DNA fragment encoding the correct amino acid sequence of reconstructed 12E10 single chain Fv were named pCHO-sc12E10 and pCOS-sc12E10. The nucleotide sequence and amino acid sequence of the reconstructed 12E10 single chain Fv included in the plasmids pCHO-sc12E10 and pCOS-sc12E10 are shown in SEQ ID NO: 113.

### 8.4 Expression of antibody 12E10 (IgG, Fab) and single chain Fv polypeptide by animal cell

Antibody 12E10 (IgG, Fab) and single chain Fv derived from antibody 12E10 (linker sequence 5 amino acids, 15 amino acids) were expressed by using COS-7 cells or CHO cells.

The transient expression using COS-7 cells was performed as follows. The transfection was performed by electroporation method using Gene Pulser II equipment (BioRad). For the expression of antibody 12E10 (IgG) each 10µg of the above-mentioned expression vector HEF-12E10H-gγ1 and pCOS-12E10L were added, for the expression of 12E10Fab fragment each 10µg of pFd-12E10H and pCOS-12E10L were added and for the expression of single chain Fv of pCOS-sc12E10 (10µg) or pCOS-db12E10 (10µg) was added to COS-7 cells (1×10⁷ cells/ml) suspended in 0.8ml of PBS. The mixture kept in a cuvette was treated by pulse at the capacity of 1.5kV, 25 µFD. After recovering for 10 minutes in a room temperature the electroporated cells were added to DMEM medium (GIBCO BRL) containing 10% bovine fetal serum and cultivated. After cultivating overnight the cells were washed once by PBS, added to serum-free medium CHO-S-SFM II (GIBCO BRL) and cultivated for 3 days. The culture supernatant was centrifuged to remove cell debris and filtered with 0.22 µm filter.

To establish a stable expression CHO cell line for the single chain Fv (polypeptide) derived from antibody 12E10, the expression vector pCHO-sc12E10 or pCHO-ds12E10 was introduced into CHO cells respectively.

Each expression vector was introduced into CHO cells by electroporation method using Gene Pulser II equipment (BioRad). Linearized DNA (100µg) obtained by digestion with restriction enzyme PvuI and CHO cells (1x10⁷ cells /ml) suspended in 0.8 ml of PBS were mixed in a cuvette, left stationary on ice for 10 minutes and treated with pulse at the capacity of 1.5kV, 25µFD. After recovering for 10 minutes at a room temperature the electroporated cells were added to CHO-S-SFM II medium (GIBCO BRL) containing 10% dialyzed bovine fetal serum and nucleic acid and cultivated. After cultivating for 2 days the cultivation was continued in nucleic acid-free CHO-S-SFM II medium (GIBCO BRL) containing 10% dialyzed bovine fetal serum. From thus obtained clones a clone with high expression rate was selected as the production cell line for 12E10 single chain Fv. After cultivating in serum-free CHO-S-SFM II medium (GIBCO BRL), the culture supernatant was centrifuged to remove cell debris and filtered with 0.22µm filter.

### 8.5 Purification of single chain Fv derived from 12E10 produced by CHO cells

The culture supernatants produced by CHO cell lines expressing 12E10 single chain Fvs (sc12E10, db12E10) obtained in Example 8.4 were purified by anti-FLAG antibody column and gel filtration column respectively to produce purified single chain Fvs.

### (1) Purification with anti-FLAG antibody column

Each culture supernatant (sc12E10, db12E10) was added to anti-FLAG M2 affinity gel column (SIGMA) equilibrated by 50mM Tris-HCl buffer (pH7.4) containing 150mM NaCl. After washing the column by the same buffer the proteins adsorbed to the column were eluted by 100mM glycine buffer (pH 3.5). The eluted fractions were immediately neutralized by adding 1M Tris-HCl buffer (pH 8.0) and analyzed by SDS-PAGE. The fraction which was confirmed to contain the single chain Fv was pooled and concentrated about 20-fold using Centricon-10 (AMICON).

### (2) Gel filtration

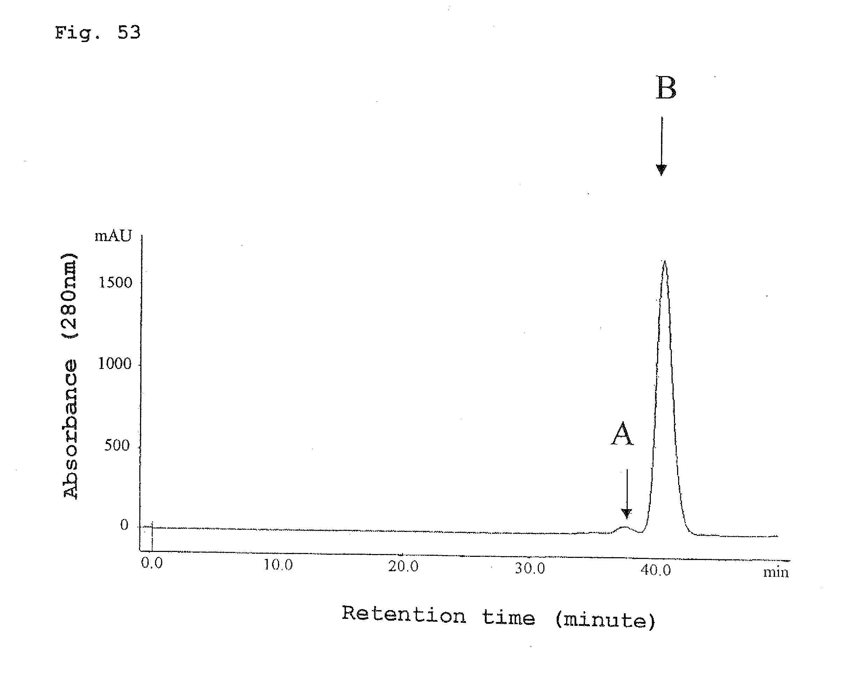

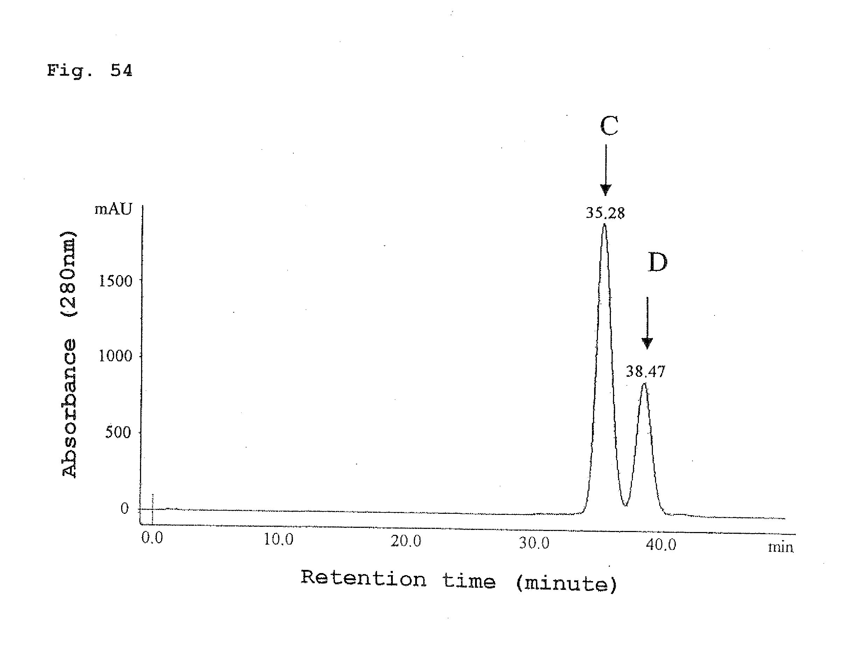

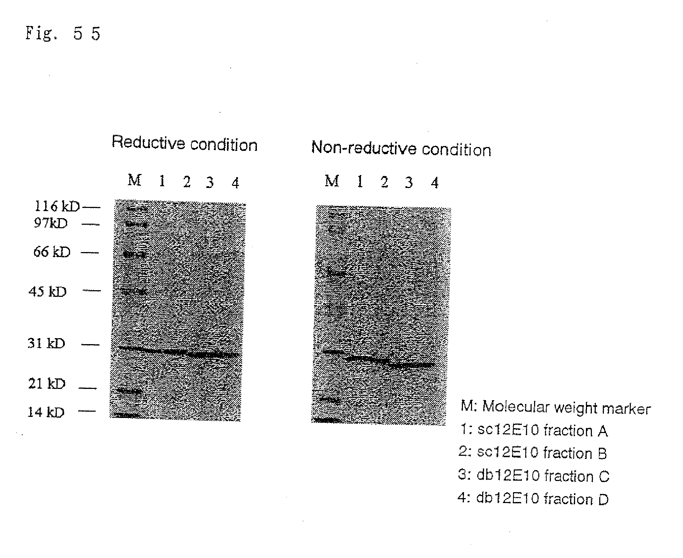

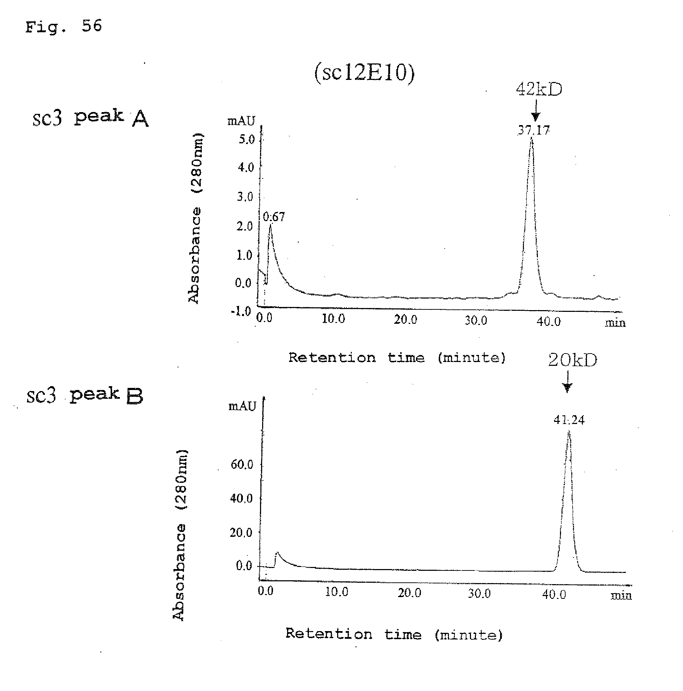

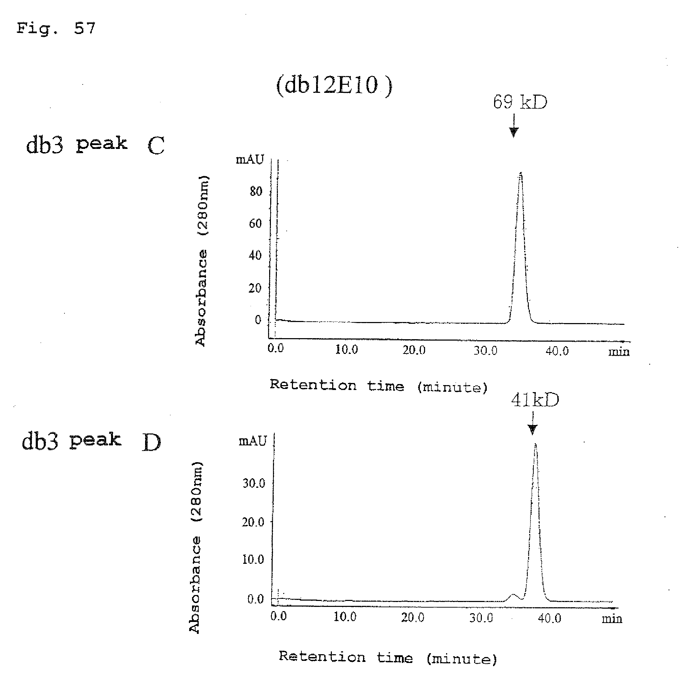

The concentrated solution obtained in (1) was added to Superdex200 column HR (10x300mm, AMERSHAM PHARMACIA) equilibrated by PBS containing 0.01% Tween20. Chlomatograms were shown in Fig. 53 and 54. The product sc12E10 was eluted in two peaks (A, B) (see Fig. 53). The product db12E10 was eluted in two peaks (C, D) (see Fig. 54). Each peak fraction was collected, treated in the presence and absence of a reducing agent, processed by electrophoresis according to Laemmli method and stained by Coomassie Brilliant Blue after the electrophoresis. As shown in Fig. 55 the all of fractions A, B, C and D, regardless of the presence or absence of the reducing agent, produced a single band having an apparent molecular weight of about 31 kD. When these fractions were analyzed by gel filtration using Superdex200 HR, the fraction A produced a product eluted at an apparent molecular weight of about 20 kD, the fraction B at 42kD (see Fig. 56), fraction C at 69kD and fraction D at 41kD (see Fig. 57) . The results suggest that sc12E10-derived fraction A is the non-covalent bond dimer of single chain Fv and the fraction B is the monomer of single chain Fv, and the db12E10-derived fraction C is the non-covalent bond trimer of single chain Fv and D is non-covalent bond dimer of single chain Fv.

### 8. Measurement of TPO-like agonist activity of various single chain Fvs

The TPO-like activity of anti-mpl single chain antibody was evaluated by measuring the proliferation activity to Ba/F3 cells (BaF/mpl) expressing human TPO receptor (MPL).

After washing BaF/mpl cells two times by RPMI1640 medium (GIBCO) containing 1% bovine fetal serum (GIBCO), the cells were suspended in the medium at cell density of 5x10⁵ cells/mL. The anti-MPL single chain antibody or human TPO (R&D Systems) was diluted with the medium, respectively. 50µl of the cell suspension and 50µl of the diluted antibody or human TPO were added in 96-well microplate (flat bottom) (Corning), and cultivated in CO₂ incubator (CO₂ concentration: 5%) for 24 hours. After the incubation 10µl of WST-8 reagent (reagent for measuring the number of raw cells SF: Nacalai Tesque) was added and the absorbance was immediately measured at measurement wavelength of 450nm and at reference wavelength of 655nm using absorbency photometer Benchmark Plus (BioRad). After incubating in CO₂ incubator (CO₂ concentration: 5%) for 2 hours, the absorbance at 450nm of measurement wavelength and 655nm of reference wavelength was again measured using Benchmark Plus. Since WST-8 reagent developed the color reaction depending upon the number of live cells at wavelength of 450nm, the proliferation activity of BaF/mpl was evaluated based on the change of absorbance in 2 hours.

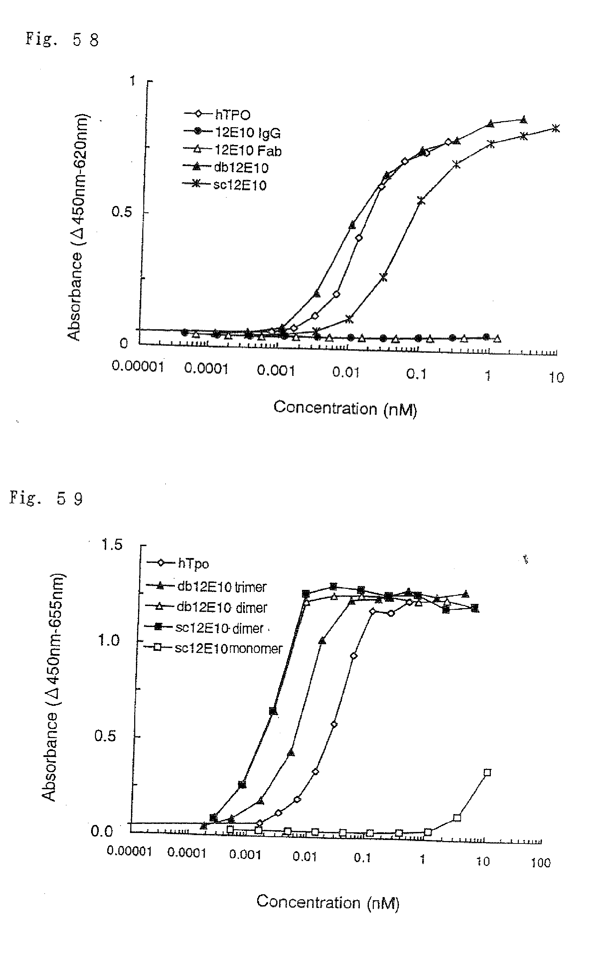

The agonist activity to MPL measured by using culture supernatants of COS-7 cells expressing various 12E10 antibody molecules are shown in Fig. 58. Single chain Fvs having the 5-amino-acid-linker (ds12E10) and the 15-amino-acid-linker (sc12E10) increased the absorbance in concentration-dependent manner, showing TPO-like agonist activity (ED50; 9pM and 51pM respectively), while 12E10IgG and 12E10Fab had no activity.

It has been known that H chain and L chain of the single chain Fv are associated not only within a molecule but also between molecules to form multimers such as dimer. When the culture supernatants of CHO cells expressing single chain Fvs of 12E10 were gel filtrated and tested for agonist activity on MPL. The results were shown in Fig. 59. The dimer, which was contained in sc12E10 in a small amount, showed about 5000-fold stronger TPO-like agonist activity (sc12E10 dimer, ED50; 1.9pM) compared with the monomer (sc12E10 monomer, ED50; 10nM). The activity was higher than that of TPO (ED50; 27pM). The dimer of db12E10 (db12E10 dimer, ED50; 2.0pM) showed strong activity comparable to that of sc12E10 dimer. db12E10 trimer (ED50; 7.4pM), which was presumed to be a trimer from molecular weight obtained by gel filtration, showed a high activity which is lower than that of db12E10 dimer. Those results suggest that it is important for the activity of agonist antibody 12E10 that the antigen-binding site is bivalent (dimer). Considering the fact that 12E10 IgG had no activity, other factors than being bivalent are presumed to be important such as the location of antigen-binding site, the distance or the angle.

### EXPLANATION OF DRAWINGS

Fig. 1 shows the result of flow cytometry, illustrating that human IgG antibody does not bind to L1210 cells expressing human IAP (hIAP/L1210).
Fig. 2 shows the result of flow cytometry, illustrating that the chimera MABL-1 antibody specifically binds to L1210 cells expressing human IAP (hIAP/L1210).

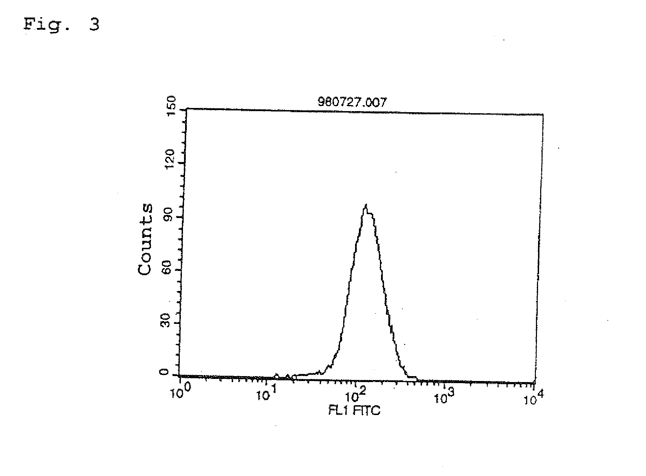

Fig. 3 shows the result of flow cytometry, illustrating that the chimera MABL-2 antibody specifically binds to L1210 cells expressing human IAP (hIAP/L1210).
Fig. 4 schematically illustrates the process for producing the single chain Fv according to the present invention.
Fig. 5 illustrates a structure of an expression plasmid which can be used to express a DNA encoding the single chain Fv of the invention in E. coli.
Fig. 6 illustrates a structure of an expression plasmid which is used to express a DNA encoding the single chain Fv of the invention in mammalian cells.
Fig. 7 shows the result of western blotting in Example 5.4. From the left, a molecular weight marker (which indicates 97.4, 66, 45, 31, 21.5 and 14.5 kDa from the top), the culture supernatant of pCHO1-introduced COS7 cells and the culture supernatant of pCHOM2-introduced COS7 cells. It illustrates that the reconstructed single chain Fv of the antibody MABL-2 (arrow) is contained in the culture supernatant of the pCHOM2-introduced cells.
Fig. 8 shows the result of flow cytometry, illustrating that an antibody in the culture supernatant of nCHO1/COS7 cell as a control does not bind to pCOS1/L1210 cell as a control.
Fig. 9 shows the result of flow cytometry, illustrating that an antibody in the culture supernatant of MABL2-scFv/COS7 cells does not bind to pCOS1/L1210 cells as a control.

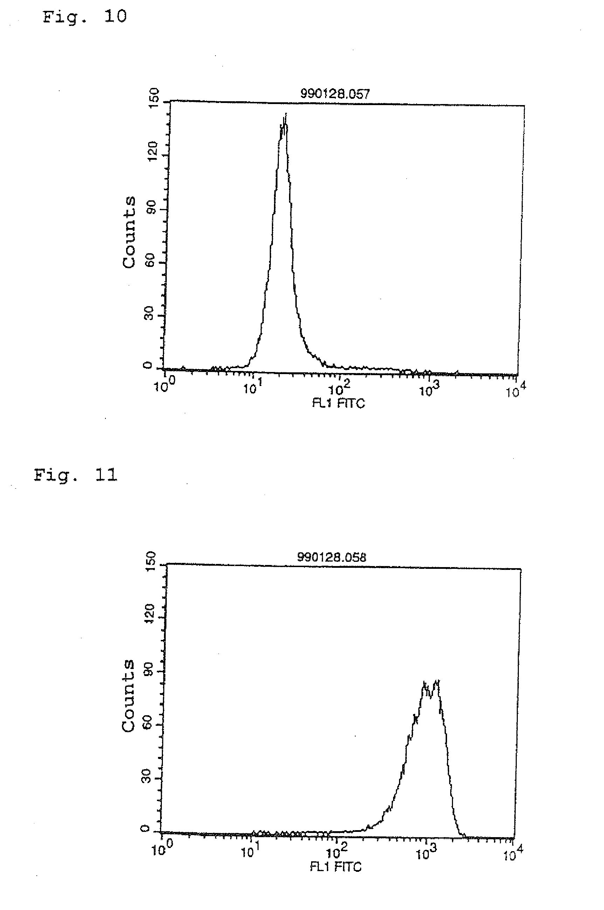

Fig. 10 shows the result of flow cytometry, illustrating that an antibody in the culture supernatant of pCCS1/COS7 cells as a control does not bind to hIAP/L1210 cells.
Fig. 11 shows the result of flow cytometry, illustrating that an antibody in the culture supernatant of MABL2-scFv/COS7 cells specifically binds to hIAP/L1210 cells.
Fig. 12 shows the result of the competitive ELISA in Example 5.6, wherein the binding activity of the single chain Fv of the invention (MABL2-scFv) to the antigen is demonstrated in terms of the inhibition of binding of the mouse monoclonal antibody MABL-2 to the antigen as an index, in comparison with the culture supernatant of pCHO1/COS cells as a control.
Fig. 13 shows the results of the apoptosis-inducing effect in Example 5.7, illustrating that the antibody in the culture supernatant of pCHO1/COS7 cells as a control does not induce the apoptosis of pCOS1/L1210 cells as a control.

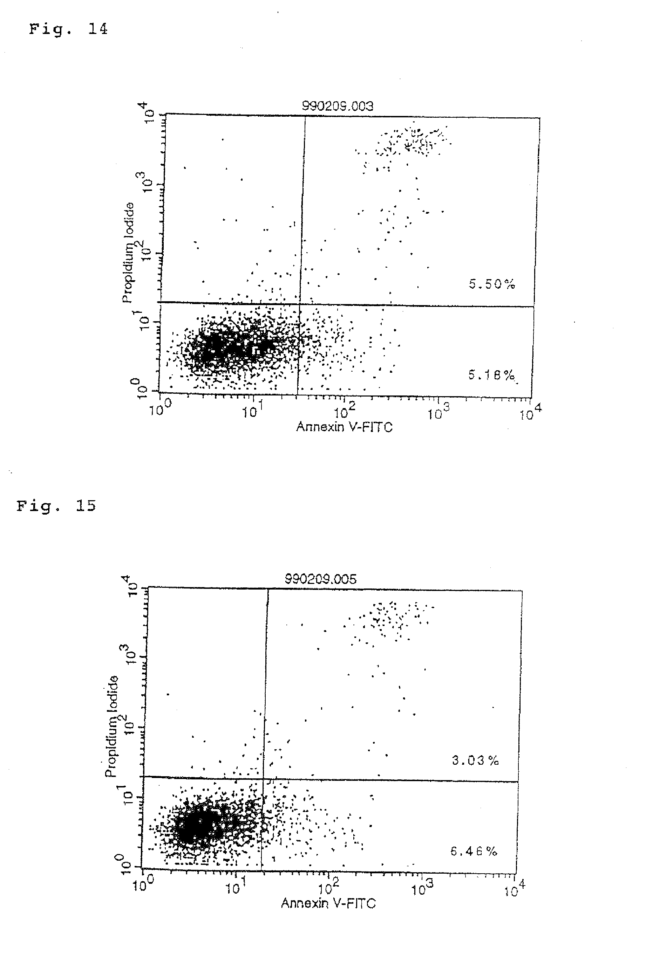

Fig. 14 shows the results of the apoptosis-inducing effect in Example 5.7, illustrating that the antibody in the culture supernatant of MABL2-scFv/COS7 cells does not induce apoptosis of pCO51/L1210 cells as a control.
Fig. 15 shows the results of the apoptosis-inducing effect in Example 5.7, illustrating that the antibody in the culture supernatant of pCH01/COS7 cells as a control does not induce apoptosis of hIAP/L1210 cells.
Fig. 16 shows the results of the apoptosis-inducing effect in Example 5.7, illustrating that the antibody in the culture supernatant of MABL2-scFv/COS7 cells specifically induces apoptosis of hIAP/L1210 cells.
Fig. 17 shows the results of the apoptosis-inducing effect in Example 5.7, illustrating that the antibody in the culture supernatant of pCH01/COS7 cells as a control does not induce apoptosis of CCRF-CEM cells (at 50% of the final concentration).

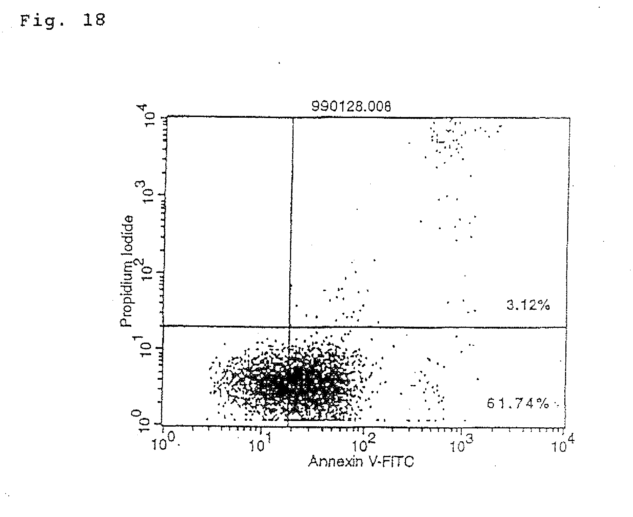

Fig. 18 shows the results of the apoptosis-inducing effect in Example 5.7, illustrating that the antibody in the culture supernatant of MABL2-scFv/COS7 cells specifically induces apoptosis of CCRF-CEM cells (at 50% of the final concentration).
Fig. 19 shows the chromatogram obtained in the purification of the single chain Fv derived form the antibody MABL-2 produced by the CHO cells in Example 5.9, illustrating that fraction A and fraction B were obtained as the major peaks when the fraction from Blue-sepharose column was purified with hydroxyapatite column.
Fig. 20 shows the results of purification by gel filtration of fraction A and fraction B obtained in Example 5.9-(2), illustrating that the major peaks (AI and BI, respectively) were eluted from fraction A at approximately 36 kD of the apparent molecular weight and from fraction B at approximately 76 kD.
Fig. 21 is the analysis on SDS-PAGE of the fractions obtained in the purification of the single chain Fv derived from the antibody MABL-2 produced by the CHO cells in Example 5.9, illustrating that a single band of approximately 35 kD of molecular weight was observed in both fractions.
Fig. 22 shows the results of analysis of fractions AI and BI obtained by gel filtration in the purification of the single chain Fv derived from the antibody MABL-2 produced by the CHO cells, wherein fraction AI comprises monomer and fraction BI comprises dimer.
Fig. 23 illustrates a structure of an expression plasmid which can be used to express a DNA encoding the single chain Fv of the invention in E. coli.
Fig. 24 shows the results of purification on the gel filtration column of crude products of the single chain Fv polypeptide derived from the antibody MABL-2 produced by E. coli obtained in Example 5.12, wherein each peak indicates monomer or dimer, respectively, of the single chain Fv produced by E. coli.
Fig. 25 shows the results of the apoptosis-inducing effect in Example 5.13, illustrating that mouse IgG antibody as a control does not induce apoptosis of hIAP/L1210 cells (the final concentration of 3 µg/ml).
Fig. 26 shows the results of the apoptosis-inducing effect in Example 5.13, illustrating that the dimer of MABL2-scfv produced by the CHO cells remarkably induces apoptosis of hIAP/L1210 cells (the final concentration of 3 µg/ml) .
Fig. 27 shows the results of the apoptosis-inducing effect in Example 5.13, illustrating that the dimer of MABL2-scFv produced by E. coli remarkably induces apoptosis of hIAP/L1210 cells (the final concentration of 3 µg/ml) .
Fig. 28 shows the results of the apoptosis-inducing effect in Example 5.13, illustrating that apoptosis induction to hIAP/L1210 cells by the MABL2-scFv monomer produced by the CHO cells is the same level as that of the control (the final concentration of 3 µg/ml).
Fig. 29 shows the results of the apoptosis-inducing effect in Example 5.13, illustrating that apoptosis induction to hIAP/L1210 cells of the MABL2-scFv monomer produced by E. coli is the same level as that of control (the final concentration of 3 µg/ml).
Fig. 30 shows the results of the apoptosis-inducing effect in Example 5.13, illustrating that mouse IgG antibody used as a control does not induce apoptosis of hIAP/L1210 cells even when anti-FLAG antibody is added (the final concentration of 3 µg/ml).
Fig. 31 shows the results of the apoptosis-inducing effect in Example 5.13, illustrating that MABL2-scFv monomer produced by the CHO cells remarkably induces apoptosis of hIAP/L1210 cells when anti-FLAG antibody is added (the final concentration of 3 µg/ml).
Fig. 32 shows the results of quantitative measurement of human IgG in the serum of a human myeloma cell line KPMM2-transplanted mouse, indicating amounts of human IgG produced by the human myeloma cells in the mouse. It illustrates that the dimer of scFv/CHO remarkably inhibited growth of the KPMM2 cells.
Fig. 33 shows the survival time of the mouse after the transplantation of tumor, illustrating that the scFv/CHO dimer-administered group elongated remarkably the survival time.
Fig. 34 illustrates a structure of an expression plasmid which expresses a modified antibody [sc(Fv)₂] comprising two H chain V regions and two L chain V regions derived from the antibody MABL-2.
Fig. 35 illustrates a structure of a plasmid which expresses a scFv (HL type) wherein the V regions are linked in the manner of [H chain]-[L chain] without a peptide linker.
Fig. 36 illustrates a structure of the HL-type polypeptide and amino acid sequences of peptide linkers.
Fig. 37 illustrates a structure of a plasmid which expresses a scFv (LH type) wherein the V regions are linked in the manner of [L chain]-[H chain] without a peptide linker.
Fig. 38 illustrates a structure of the LH-type polypeptide and amino acid sequences of peptide linkers.
Fig. 39 shows the results of the western blotting in Example 6.4, illustrating that the modified antibody sc(FV)₂ comprising two H chain V regions and two L chain V regions, and the MABL2-scFv having peptide linkers with different length are expressed.
Figs. 40a and 40b show the results of flow cytometry using the culture supernatant of COS7 cells prepared in Example 6.3 (1), illustrating that the MABL2-scFv and sc(Fv)₂ having peptide linkers with different length have high affinities against human IAP.
Fig. 41 shows the results of the apoptosis-inducing effect in Example 6.6, illustrating that the scFv <HL3, 4, 6, 7, LH3, 4, 6 and 7> and the sc(Fv)₂ remarkably induce cell death of hIAP/L1210 cells.
Fig. 42 shows the results of the evaluation of antigen binding capacity in Example 6.10, illustrating that the dimer of scFv <HL5> and sc(Fv)₂ have high affinities against human IAP.
Fig. 43 shows the results of the in vitro apoptosis-inducing effect in Example 6.11, illustrating that the dimer of scFv <HL5> and the sc(Fv)₂ induce apoptosis of hIAP/L1210 cells and CCRF-CEM cells in concentration-dependent manner.

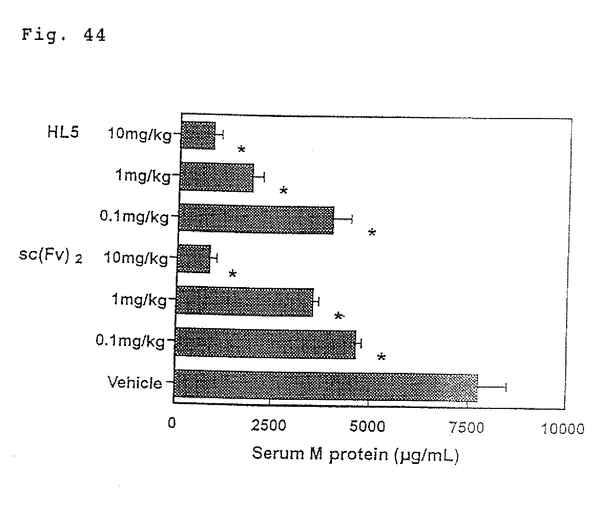

Fig. 44 shows the results of the quantitative measurement of M protein produced by a human myeloma cell line KPMM2 in the serum of the human myeloma cell-transplanted mouse. It illustrates that the dimer of scFv <HL5> and the sc(Fv)₂ remarkably inhibited growth of the KPMM2 cells.

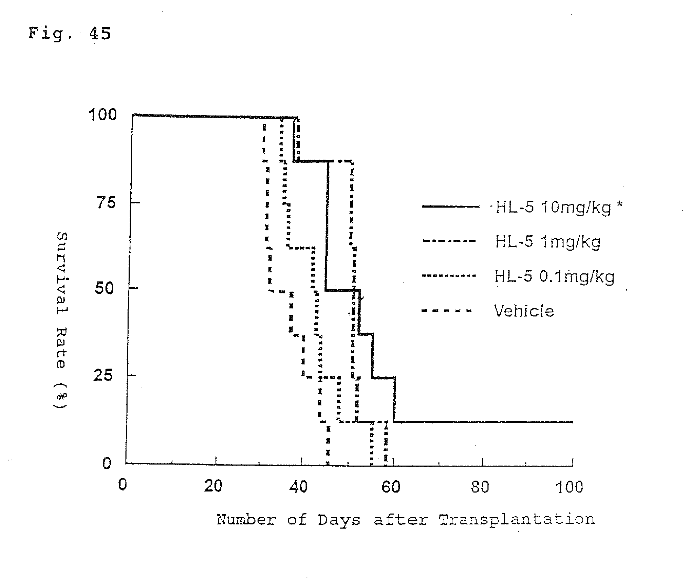

Fig. 45 shows the survival time (days) of mice after the transplantation of tumor, illustrating that the survival time of the scFv <HL5> administrated-group was remarkably prolonged.

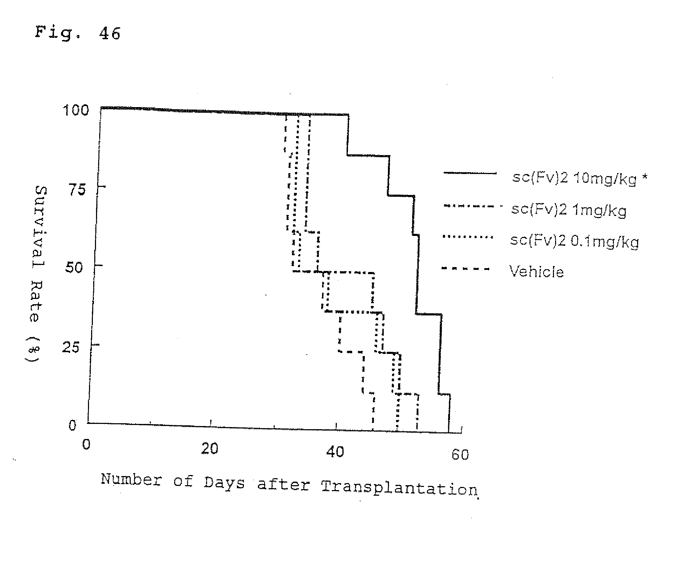

Fig. 46 shows the survival time (days) of mice after the transplantation of tumor, illustrating that the survival time of the sc(Fv)₂ administrated-group was remarkably prolonged.
Fig. 47 is a scheme showing the method for constructing DNA fragment encoding the reconstructed 12B5 single chain Fv containing the linker sequence consisting of 15 amino acids and the structure thereof.
Fig. 48 shows the purification result of each 12B5 single chain Fv by gel filtration obtained in Example 7. 5 (1), illustrating that sc12B5 was divided into two peaks (fractions A and B).
Fig. 49 shows the analytical result of each fraction A and B by SDS-PAGE performed in Example 7. 5 (2).
Fig. 50 shows the analytical result of each fraction A and B by Superdex200 column performed in Example 7. 5 (2), illustrating that the major peak of fraction A was eluted at an apparent molecular weight of about 44 kD shown in (a) and that the major peak of fraction B was eluted at an apparent molecular weight of about 22kD shown in (b).
Fig. 51 shows the measurement result of the TPO-like agonist activity of sc12B5 and antibody 12B5 (IgG, Fab), illustrating that 12B5IgG and monovalent single chain Fv (sc12B5) showed TPO-like agonist activity in concentration-dependent manner.
Fig. 52 shows the measurement result of TOP-like agonist activity of sc12B5 monomer and dimer, illustrating that single chain Fv (sc12B5 dimer) having bivalent antigen-binding site had agonist activity about 400-fold higher than monovalent sc12B5 and that the efficacy is equivalent to or higher than human TPO.
Fig. 53 shows the purification result of obtained sc12E10 single chain antibody by gel filtration chromatography using Superdex200HR column, illustrating that 12E10sc3 was divided into two peaks (fractions A and B).
Fig. 54 shows the purification result of obtained db12E10 single chain antibody by gel filtration chromatography using Superdex200HR column, illustrating that 12E10sc3 was divided into two peaks (fractions C and D).
Fig. 55 shows SDS-PAGE analysis of fractions A and B (sc12E10) and fractions C and D (dh12E10) under the reductive or non-reductive condition.
Fig. 56 shows the analytical result of fractions A and B by gel filtration chromatography using Superdex200HR column, illustrating (1) the major peak of fraction A was eluted at an apparent molecular weight of about 42 kD and (2) the major peak of fraction B was eluted at an apparent molecular weight of about 20kD.
Fig. 57 shows the analytical result of fractions C and D by gel filtration chromatography using Superdex200HR column, illustrating (1) the major peak of fraction C was eluted at an apparent molecular weight of about 69 kD and (2) the major peak of fraction B was eluted at an apparent molecular weight of about 41kD.
Fig. 58 is a graph showing the agonist activity of various 12E10 antibody molecules on MPL, illustrating that single chain Fvs (sc12E10, db12E10) showed TPO-like agonist activity while 12E10 IgG and 12E10 Fab did not.
Fig. 59 is a graph showing the agonist activity of monomer and dimer of sc12E10 and dimer and trimer of db12E10 on MPL, illustrating that dimer of sc12E10 and dimer and trimer of db12E10 showed TPO-like agonist activity higher than TPO.

### INDUSTRIAL APPLICABILITY

The modified antibodies of the invention have an agonist action capable of transducing a signal into cells by crosslinking a cell surface molecule(s) and are advantageous in that the permeability to tissues and tumors is high due to the lowered molecular size compared with the parent antibody molecule (whole IgG). The present invention provides the modified antibodies which have remarkably high agonist activity compared with natural ligands such as TPO and the parent antibody (whole IgG). Even if the parent antibody has no agonist activity, modified antibodies with a higher agonist activity compared with natural ligands can be provided. This is attributable to that the modified antibodies are in a shape closer to a ligand as compared with original antibodies. Therefore the modified antibodies can be used as signal-transducing agonist to achieve apoptosis induction, cell proliferation induction, cell differentiation induction, cell division induction or cell cycle regulation action. The modification of antibody molecule to the modified antibody according to the invention results in the reduction of side effects caused by intercellular crosslinking and provides novel medicines inducing only required action by crosslinking a cell surface molecule(s). Medical preparations containing as active ingredient the modified antibody of the invention are useful as preventives and/or remedies for cancers, inflammation, hormone disorders, autoimmune diseases and blood diseases, for example, leukemia, malignant lymphoma, aplastic anemia, myelodysplasia syndrome and polycythemia vera.

### Preferred embodiments

1. A modified antibody comprising two or more H chain V regions and two or more L chain V regions of the same or different monoclonal antibody and showing an agonist action by crosslinking a cell surface molecule(s) or intracellular molecule(s)*.*
2. The modified antibody comprising two or more H chain V regions and two or more L chain V regions of monoclonal antibody and showing an agonist action by crosslinking a cell surface molecule(s).
3. The modified antibody of item 1 or 2, wherein the H chain V region and the L chain V region are connected through a linker.
4. The modified antibody of item 3, wherein the linker is a peptide linker comprising at least one amino acid.
5. The modified antibody of any one of items 1 to 4, wherein the modified monoclonal antibody is a multimer of single chain Fv comprising an H chain V region and an L chain V region.
6. The modified antibody of item 5, wherein the modified antibody is composed of tetramer, trimer or dimer of single chain Fv.
7. The modified antibody of item 6, wherein the modified antibody is composed of dimer of single chain Fv.
8. The modified antibody of any one of items 5 to 7,
   wherein the H chain V region and the L chain V region existing in the same chain are not associated to form an antigen-binding site.
9. The modified antibody of any one of items 1 to 4, wherein the modified antibody is a single chain polypeptide comprising two or more H chain V regions and two or more L chain V regions.
10. The modified antibody of item 9, wherein the modified antibody is a single chain polypeptide comprising two H chain V regions and two L chain V regions.
11. The modified antibody of any one of items 1 to 10, wherein the modified antibody further comprises an amino acid sequence(s) for peptide purification.
12. The modified antibody of any one of items 1 to 11, wherein the modified antibody has been purified.
13. The modified antibody of any one of items 1 to 12, wherein H chain V region and/or L chain V region is H chain V region and/or L chain V region derived from a human antibody.
14. The modified antibody of any one of items 1 to 13, wherein H chain V region and/or L chain V region is humanized H chain V region and/or L chain V region.
15. The modified antibody of any one of items 1 to 14, wherein the cell surface molecule or intracellular molecule is a hormone receptor, a cytokine receptor, tyrosine kinase receptor or intranuclear receptor.
16. The modified antibody of any one of items 1 to 15, wherein the cell surface molecule or intracellular molecule is erythropoietin (EPO) receptor, thrombopoietin (TPO) receptor, granulocyte colony stimulating factor (G-CSF) receptor, macrophage colony stimulating factor (M-CSF) receptor, granular macrophage colony stimulating factor (GMCSF) receptor, tumor necrosis factor (TNF) receptor, interleukin-1 (IL-1) receptor, interleukin-2 (IL-2) receptor, interleukin-3 (IL-3) receptor, interleukin-4 (IL-4) receptor, interleukin-5 (IL-5) receptor, interleukin-6 (IL-6) receptor, interleukin-7 (IL-7) receptor, interleukin-9 (IL-9) receptor, interleukin-10 (IL-10) receptor, interleukin-11 (IL-11) receptor, interleukin-12 (IL-12) receptor, interleukin-13 (IL-13) receptor, interleukin-15 (IL-15) receptor, interferon-alpha (IFN-alpha) receptor, interferon-beta (IFN-beta) receptor, interferon-gamma (IFN-gamma) receptor, growth hormone (GH) receptor, insulin receptor, blood stem cell proliferation factor (SCF) receptor, vascular endothelial growth factor (VEGF) receptor, epidermal cell growth factor (EGF) receptor, nerve growth factor (NGF) receptor, fibroblast growth factor (EGF) receptor, platelet-derived growth factor (PDGF) receptor, transforming growth factor-beta (TGF-beta) receptor, leukocyte migration inhibitory factor (LIF) receptor, ciliary neurotrophic factor (CNTF) receptor, oncostatin M (OSM) receptor, Notch family receptor, E2F, E2F/DP1 or TAK1/TAB1.
17. The modified antibody of any one of items 1 to 16, wherein the agonist action is apoptosis induction, cell proliferation induction, cell differentiation induction, cell division induction or cell cycle regulation action.
18. The modified antibody of any one of items 1 to 17, wherein the modified antibody is mono-specific modified antibody.
19. The modified antibody of any one of items 1 to 17, wherein the modified antibody is multi-specific modified antibody.
20. The modified antibody of item 19, wherein the modified antibody is bi-specific modified antibody.
21. The monoclonal antibody of item 20, wherein the L chain V region and the H chain V region are from the same monoclonal antibody.
22. The monoclonal antibody of any one of items 1 to 21 which shows an equivalent or better agonist action (EDSO) compared with the parent monoclonal antibody.
23. The monoclonal antibody of item 22 which shows at least 2-fold agonist action (ED50) compared with the parent monoclonal antibody.
24. The monoclonal antibody of item 23 which shows at least 10-fold agonist action (ED50) compared with the parent monoclonal antibody.
25. The monoclonal antibody of any one of items 1 to 21 which is derived from a parent antibody having substantially no agonist action.
26. A compound comprising two or more H chain V regions and two or more L chain V regions of monoclonal antibody and showing an equivalent or better agonist action (ED50) compared with a natural ligand that binds to a cell surface molecule(s) or intracellular molecule (s)
27. The compound of item 26 which shows at least 2-fold agonist action (ED50) compared with a natural ligand that binds to a cell surface molecules (s) or intracellular molecule(s).
28. The compound of item 27 which shows at least 10-fold agonist action (ED50) compared with a natural ligand that binds to a cell surface molecule(s) or intracellular molecule(s).
29. The modified antibody or compound of any one of items 1 to 28 which has substantially no intercellular adhesion action.
30. The modified antibody or compound of any one of items 1 to 28 which has intercellular adhesion action (ED50) not more than 1/10 compared with the parent antibody.
31. A DNA which encodes the modified antibody or compound of any one of items 1 to 28.
32. An animal cell which produces the modified antibody or compound of any one of items 1 to 28.
33. A microorganism which produces the modified antibody or compound of any one of items 7. to 28.
34. Use of the modified antibody or compound of any one of items 1 to 28 as an agonist.
35. A method of inducing an agonist action to cells which comprises administering the first ligand and the second ligand that bind to a cell surface molecule (s) or intracellular molecule (s) and administering a substance that binds to the first and the second ligands and crosslinks the first and the second ligands.
36. The method of item 35 wherein the first and the second ligands are the same or different single chain Fv monomers.
37. The method of item 35 or 36 wherein the substance that crosslinks the ligands is an antibody, an antibody fragment or a bivalent modified antibody.
38. A method of causing agonist action to cells by crosslinking a cell surface molecule(s) or intracellular molecule(s) using the modified antibody or compound of any one of items 1 to 28.
39. The method of item 38 wherein the agonist action is apoptosis induction, cell proliferation induction, cell differentiation induction, cell division induction or cell cycle regulation action.
40. A medicine comprising as active ingredient the modified antibody or compound of any one of items 1 to 29.
41. Use of the modified antibody or compound of any one of items 1 to 29 as medicine.
42. A method of screening a modified antibody comprising two or more H chain V regions and two or more L chain V regions or antibody and showing an agonist action by crosslinking a cell surface molecule(s) or intracellular molecule(s) which comprises the steps
   (1) to produce a modified antibody comprising two or more H chain V regions and two or more L chain V regions of antibody and binding specifically to a cell surface molecule(s) or intracellular molecule(s),
   (2) to subject cells expressing said cell surface molecule(s) or intracellular molecule(s) to react with the modified antibody and
   (3) to measure the agonist action in the cells caused by crosslinking said cell surface molecule(s) or intracellular molecule(s).
43. A method of measuring an agonist action of a modified antibody comprising two or more H chain V regions and two or more L chain V regions of antibody and showing an agonist action by crosslinking a cell surface molecule(s) or intracellular molecule(s) which comprises the steps
   (1) to produce a modified antibody comprising two or more H chain V regions and two or more L chain V regions of antibody and binding specifically to a cell surface molecule(s) or intracellular molecule(s),
   (2) to subject cells expressing said cell surface molecule(s) or intracellular molecule(s) to react with the modified antibody and
   (3) to measure the agonist action in the cells caused by crosslinking said cell surface molecule(s) or intracellular molecule (s) .
44. A method of producing a modified antibody comprising two or more H chain V regions and two or more L chain V regions of monoclonal antibody and showing an agonist action by crosslinking a cell surface molecule(s) or intracellular molecule(s) which comprises the steps
   (1) to culture animal cells of item 32 or microorganisms of claim 33 to produce the modified antibody and
   (2) to purify said monoclonal antibody.

## Claims

1. A modified antibody comprising two or more H chain V regions and two or more L chain V regions of a monoclonal antibody and showing an agonist action by crosslinking a cell surface molecule(s) or intracellular molecule(s).

2. The modified antibody of claim 1, wherein the H chain V region and the L chain V region are connected through a linker.

3. The modified antibody of claim 2, wherein the linker is a peptide linker comprising at least one amino acid.

4. The modified antibody of any one of claims 1 to 3, wherein the modified monoclonal antibody is a multimer of single chain Fv comprising an H chain V region and an L chain V region.

5. The modified antibody of claim 4, wherein, the modified antibody is composed of tetramer, trimer or dimer of single chain Fv.

6. The modified antibody of any one of claims 4 to 5, wherein the H chain V region and the L chain V region existing in the same chain are not associated to form an antigen-binding site.

7. The modified antibody of any one of claims 1 to 3, wherein the modified antibody is a single chain polypeptide comprising two or more H chain V regions and two or more L chain V regions.

8. The modified antibody of claim 7, wherein the modified antibody is a single chain polypeptide comprising two H chain V regions and two L chain V regions.

9. The modified antibody of any one of claims 1 to 8, wherein H chain V region and/or L chain V region is H chain V region and/or L chain V region derived from a human antibody or is humanized H chain V region and/or L chain V region.

10. The modified antibody of any one of claims 1 to 9, wherein the cell surface molecule or intracellular molecule is a hormone receptor, a cytokine receptor, tyrosine kinase receptor or intranuclear receptor.

11. The modified antibody of any one of claims 1 to 10, wherein the cell surface molecule or intracellular molecule is erythropoietin (EPO) receptor, thrombopoietin (TPO) receptor, granulocyte colony stimulating factor (G-CSF) receptor, macrophage colony stimulating factor (M-CSF) receptor, granular macrophage colony stimulating factor (GMCSF) receptor, tumor necrosis factor (TNF) receptor, interleukin-1 (IL-1) receptor, interleukin-2 (IL-2) receptor, interleukin-3 (IL-3) receptor, interleukin-4 (IL-4) receptor, interleukin-5 (IL-5) receptor, interleukin-6 (IL-6) receptor, interleukin-7 (IL-7) receptor, interleukin-9 (IL-9) receptor, interleukin-10 (IL-10) receptor, interleukin-11 (IL-11) receptor, interleukin-12 (IL-12) receptor, interleukin-13 (IL-13) receptor, interleukin-15 (IL-15) receptor, interferon-alpha (IFN-alpha) receptor, interferon-beta (IFN-beta) receptor, interferon-gamma (IFN-gamma) receptor, growth hormone (GH) receptor, insulin receptor, blood stem cell proliferation factor (SCF) receptor, vascular endothelial growth factor (VEGF) receptor, epidermal cell growth factor (EGF) receptor, nerve growth factor (NGF) receptor, fibroblast growth factor (FGF) receptor, platelet-derived growth factor (PDGF) receptor, transforming growth factor-beta (TGF-beta) receptor, leukocyte migration inhibitory factor (LIF) receptor, receptor, ciliary neurotrophic factor (CNTF) receptor, oncostatin M (OSM) receptor, Notch family receptor, E2F, E2F/DP1 or TAK1/TAB1.

12. The modified antibody of any one of claims 1 to 11, wherein the agonist action is apoptosis induction, cell proliferation induction, cell differentiation induction, cell division induction or cell cycle regulation action.

13. The modified antibody of any one of claims 1 to 12, wherein the modified antibody is mono-specific modified antibody.

14. The modified antibody of any one of claims 1 to 12, wherein the modified antibody is multi-specific modified antibody.

15. The modified antibody of claim 14, wherein the modified antibody is bi-specific modified antibody.

16. The monoclonal antibody of claim 15, wherein the L chain V region and the H chain V region are from the same monoclonal antibody.

17. A DNA which encodes the modified antibody or compound of any one of claims 1 to 16.

18. An animal cell which produces the modified antibody or compound of any one of claims 1 to 16.

19. A microorganism which produces the modified antibody or compound of any one of claims 1 to 16.

20. A medicine comprising as active ingredient the modified antibody or compound of any one of claims 1 to 16.

21. A method of screening a modified antibody comprising two or more H chain V regions and two or more L chain V regions of antibody and showing an agonist action by crosslinking a cell surface molecule(s) or intracellular molecule(s) which comprises the steps
(1) to produce a modified antibody comprising two or more H chain V regions and two or more L chain V regions of antibody and binding specifically to a cell surface molecule(s) or intracellular molecule(s),
(2) to subject cells expressing said cell surface molecule(s) or intracellular molecule(s) to react with the modified antibody and,
(3) to measure the agonist action in the cells caused by crosslinking said cell surface molecule(s) or intracellular molecule(s).

22. A method of measuring an agonist action of a modified antibody comprising two or more H chain V regions and two or more L chain V regions of antibody and showing an agonist action by crosslinking a cell surface molecule(s) or intracellular molecule(s) which comprises the steps
(1) to produce a modified antibody comprising two or more H chain V regions and two or more L chain V regions of antibody and binding specifically to a cell surface molecule(s) or intracellular molecule(s),
(2) to subject cells expressing said cell surface molecule(s) or intracellular molecule(s) to react with the modified antibody and
(3) to measure the agonist action in the cells caused by crosslinking said cell surface molecule(s) or intracellular molecule (s) .

23. A method of producing a modified antibody comprising two or more H chain V regions and two or more L chain V regions of monoclonal antibody and showing an agonist action by crosslinking a cell surface molecule(s) or intracellular molecule(s) which comprises the steps
(1) to culture animal cells of claim 18 or microorganisms of claim 19 to produce the modified antibody and
(2) to purify said monoclonal antibody.
